# DEMANDE DE BREVET EUROPEEN

(11) **EP 2 322 206 A2**
(43) Date de publication de la demande: **18.05.2011**
(21) Numéro de dépôt: 10185304.2
(22) Date de dépôt: 22.11.2005
(51) Int. Cl.: A61K 38/18, C07K 14/475

(54) **Nétrine 1 mutée, ses fragments et leur utilisation comme médicaments**

(30) Priorité: 22.11.2004 FR 0412362; 22.11.2004 FR 0412364
(62) Demande divisionnaire de: 05820955.2
(71) Demandeur: Centre National de la Recherche Scientifique, 75016 Paris Cedex 16 (FR); IVS Institut des Vaisseaux et du Sang, 75475 Paris Cedex 10 (FR)
(72) Inventeur: Plouët, Jean, décédé (FR); Alemany, Monica, 75020, PARIS (FR)
(74) Mandataire: Grosset-Fournier, Chantal Catherine

(57) **Abrégé**

La présente invention concerne l'utilisation d'une nétrine choisie parmi la nétrine 4, la nétrine 4 mutée, la nétrine 1, la nétrine G1 ou la nétrine 3, ou l'un de leurs fragments, ou d'une séquence nucléotidique codant pour l'une de ces nétrines ou l'un de ces fragments, ou d'un anticorps anti-idiotypique de l'une de ces nétrines ou de l'un de ces fragments, ou d'un fragment Fab desdits anticorps anti-idiotypiques, pour la préparation d'un médicament destiné à la prévention ou au traitement des pathologies tumorales ou non tumorales.

## Description

La présente invention a pour objet la nétrine 4 mutée, ses fragments et leur utilisation comme médicament.

La présente invention a également pour objet de nouvelles utilisations thérapeutiques de la nétrine 4 non mutée et de ses fragments.

La présente invention a également pour objet de nouvelles utilisations de ligands de la néogénine, tels que les nétrines, à savoir la nétrine 1, la nétrine 3, la nétrine G1 ou la nétrine 4, et les RGM ("repulsive guidance molecule"), en association avec un agent de chimiothérapie, notamment dans le cadre de la préparation d'un médicament destiné au traitement des cancers.

La présente invention a également pour objet de nouvelles utilisations des nétrines, notamment de la nétrine 1, de la nétrine G1, de la nétrine 3 et de la nétrine 4, notamment dans le cadre du traitement de pathologies non tumorales liées à une raréfaction des péricytes.

La nétrine 4 appartient à la famille des nétrines, qui sont des molécules de guidage des axones. A ce jour, 4 membres de cette famille sont connus (nétrines 1, G, 3, 4). La nétrine 4 est une protéine constituée d'un domaine basique C-terminal interagissant avec l'héparine, de 3 domaines de type EGF et d'un domaine de type laminine (Yurchenko et al., 2004).

La nétrine 1 stimule l'attraction quand elle est liée au récepteur dcc (ou à la néogénine) et la répulsion quand elle est liée aux récepteurs dcc et un des récepteurs de la famille UNC5H (Livesey, 1999 ; Mehlen et al., 2003 ; Cooper et al., 1999). De plus, la nétrine 1 peut se lier au récepteur A2 et modifier le taux d'AMPc cellulaire, l'attraction ou la répulsion étant aussi contrôlées par le taux AMPc/GMPc (Corset et al., 2000).

La nétrine G représente une famille de gènes différant des autres nétrines par la présence d'un domaine C-terminal hydrophobe se liant au glycosyl phosphoinositolphosphate (Nakashiba et al., 2000). Leur fonction est encore inconnue. En revanche, il a été rapporté que la nétrine G ne se lie ni à dcc ni à UNC5H1, H2, H3 (Nakashiba et al., 2002).

La nétrine 3 stimule l'attraction des neurones quand elle est liée à un récepteur dcc ou néogénine et induit leur répulsion quand elle est liée à un récepteur de la famille des récepteurs UNC5H (Livesey et al., 1999).

Les molécules RGM ("repulsive guidance molecule") sont des molécules impliquées dans le guidage des axones des neurones. Il en existe 3 gènes dénommés RGM-A, RGM-B, RGM-C. On sait depuis peu que le RGM-A est un ligand de la néogénine (Matsunaga et al., 2004) et qu'un variant du gène RGM-C est le gène de transport du fer dénommé HFE (Papanicolaou et al., 2004).

Le document US 2003/0207347A1, publié le 6 novembre 2003, décrit la nétrine 4 native et ses utilisations. Plus particulièrement, cette demande décrit un polypeptide dérivé de la nétrine 4 présentant des propriétés de modulation de l'angiogenèse, ainsi que l'utilisation de la nétrine 4 dans un procédé de modulation du développement vasculaire, notamment de l'angiogenèse, et plus particulièrement d'inhibition de l'angiogenèse, notamment dans le cadre de tumeurs.

A ce jour, rien n'est connu sur les récepteurs de la nétrine 4 ni sur ses éventuelles fonctions neuronales.

Il a été récemment publié que la nétrine 1 et la nétrine 4 inhibent l'activité des cellules endothéliales par l'intermédiaire du récepteur UNC5H2 (Lu et al., 2004) et inhibent la vascularisation de la rétine.

La présente invention a également pour but de fournir de nouveaux agents anti-angiogéniques.

La présente invention a pour but de fournir un traitement de combinaison permettant d'augmenter l'efficacité des traitements impliquant l'angiogenèse et notamment de traitements anti-tumoraux conventionnels, ou de traitements anti-angiogéniques utilisés dans des pathologies autres que les tumeurs.

A ce jour, il n'existe pas d'agent thérapeutique susceptible d'entrer en synergie avec les médicaments classiquement utilisés dans le cadre du traitement de la dégénérescence maculaire liée à l'âge, ou d'autres maladies oculaires impliquant une néovascularisation.

La présente invention concerne une protéine caractérisée en ce qu'elle comprend ou est constituée par :
* la séquence SEQ ID NO : 522 ou SEQ ID NO : 524, ou
* un fragment de la dite séquence SEQ ID NO : 522 ou SEQ ID NO : 524, sous réserve que ce fragment présente une activité anti-angiogénique et / ou une activité d'activation des péricytes, ledit fragment comprenant notamment environ 40 à environ 450 acides aminés, et de préférence d'environ 40 à environ 260 acides aminés, et de préférence d'environ 40 à environ 230 acides aminés, et étant notamment représenté par l'une des séquences SEQ ID NO : 2q, q variant de 187 à 248, ou par la séquence SEQ ID NO : 526 ou par la séquence SEQ ID NO : 528,
* toute séquence dérivée de l'une des séquences susmentionnées, notamment par substitution, suppression ou addition d'un ou plusieurs acides aminés, sous réserve que cette séquence dérivée présente une activité anti-angiogénique et / ou une activité d'activation des péricytes, ou
* toute séquence homologue de l'une des séquences susmentionnées, ayant de préférence une identité d'au moins environ 50% avec la région comprise entre les acides aminés en position 261 et 515 de SEQ ID NO : 522, sous réserve que cette séquence homologue présente une activité anti-angiogénique et / ou une activité d'activation des péricytes.

La protéine SEQ ID NO : 522 susmentionnée est une nouvelle protéine correspondant à la protéine nétrine 4 mutée et la protéine SEQ ID NO : 524 susmentionnée correspond à la protéine SEQ ID NO : 522 sans peptide signal.

La séquence SEQ ID NO : 522 comprend 628 acides aminés et la séquence SEQ ID NO : 524 comprend 609 acides aminés et correspond à un fragment de la séquence SEQ ID NO : 522 allant du résidu 20 au résidu 628.

La nétrine 4 mutée, représentée par la séquence SEQ ID NO : 522, correspond à la protéine nétrine 4 représentée par SEQ ID NO : 498 présentant les 9 mutations ponctuelles suivantes :
- remplacement de la cystéine en position 13 par une arginine,
- remplacement de la lysine en position 68 par une thréonine,
- remplacement de la sérine en position 183 par une proline,
- remplacement de l'histidine en position 205 par une tyrosine,
- remplacement de la cystéine en position 234 par une tyrosine,
- remplacement de l'alanine en position 331 par une asparagine,
- remplacement de la cystéine en position 332 par une arginine,
- remplacement de l'asparagine en position 353 par une sérine,
- remplacement de l'asparagine en position 515 par une lysine.

Les fragments susmentionnés, correspondant aux séquences protéiques SEQ ID NO : 374 à SEQ ID NO : 496, sont de nouveaux fragments correspondant à des fragments de la nétrine 4 mutée susmentionnée.

Les séquences SEQ ID NO : 2q susmentionnées correspondent aux séquences protéiques SEQ ID NO : 374 à 496, c'est-à-dire aux séquence protéiques suivantes : SEQ ID NO : 374, SEQ ID NO : 376, SEQ ID NO : 378, SEQ ID NO : 380, SEQ ID NO : 382, SEQ ID NO : 384, SEQ ID NO : 386, SEQ ID NO : 388, SEQ ID NO : 390, SEQ ID NO : 392, SEQ ID NO : 394, SEQ ID NO : 396, SEQ ID NO : 398, SEQ ID NO : 400, SEQ ID NO : 402, SEQ ID NO : 404, SEQ ID NO : 406, SEQ ID NO : 408, SEQ ID NO : 410, SEQ ID NO : 412, SEQ ID NO : 414, SEQ ID NO : 416, SEQ ID NO : 418, SEQ ID NO : 420, SEQ ID NO : 422, SEQ ID NO : 424, SEQ ID NO : 426, SEQ ID NO : 428, SEQ ID NO : 430, SEQ ID NO : 432, SEQ ID NO : 434, SEQ ID NO : 436, SEQ ID NO : 438, SEQ ID NO : 440, SEQ ID NO : 442, SEQ ID NO : 444, SEQ ID NO : 446, SEQ ID NO : 448, SEQ ID NO : 450, SEQ ID NO : 452, SEQ ID NO : 454, SEQ ID NO : 456, SEQ ID NO : 458, SEQ ID NO : 460, SEQ ID NO : 462, SEQ ID NO : 464, SEQ ID NO : 466, SEQ ID NO : 468, SEQ ID NO : 470, SEQ ID NO : 472, SEQ ID NO : 474, SEQ ID NO : 476, SEQ ID NO : 478, SEQ ID NO : 480, SEQ ID NO : 482, SEQ ID NO : 484, SEQ ID NO : 486, SEQ ID NO : 488, SEQ ID NO : 490, SEQ ID NO : 492, SEQ ID NO : 494 ou SEQ ID NO : 496.

La séquence SEQ ID NO : 374 correspond à un fragment de type laminine (protéine de la matrice extracellulaire se liant à certaines intégrines) de la protéine humaine nétrine 4 mutée, ledit fragment étant codé par la séquence nucléotidique SEQ ID NO : 373. Ce fragment comprend 260 acides aminés et correspond au fragment de la protéine nétrine 4 mutée allant du résidu 1 au résidu 260 de la séquence SEQ ID NO : 522.

La séquence SEQ ID NO : 376 correspond à un fragment de type EGF (domaine de répétition facteur EGF) de la protéine humaine nétrine 4 mutée, ledit fragment étant codé par la séquence nucléotidique SEQ ID NO : 375. Ce fragment comprend 255 acides aminés et correspond au fragment de la protéine nétrine 4 mutée allant du résidu 261 au résidu 515 de la séquence SEQ ID NO : 522.

La séquence SEQ ID NO : 378 est un fragment correspondant à la protéine humaine nétrine 4 mutée délétée de la séquence SEQ ID NO : 364, ledit fragment étant codé par la séquence nucléotidique SEQ ID NO : 377. Ce fragment comprend 515 acides aminés et correspond au fragment de la protéine nétrine 4 mutée allant du résidu 1 au résidu 515 de la séquence SEQ ID NO : 522.

La séquence SEQ ID NO : 386 est un fragment de type laminine de la protéine humaine nétrine 4 mutée, ledit fragment étant codé par la séquence nucléotidique SEQ ID NO : 385. Ce fragment comprend 229 acides aminés et correspond au fragment de la protéine nétrine 4 mutée allant du résidu 32 au résidu 260 de la séquence SEQ ID NO : 522.

La séquence SEQ ID NO : 384 est un fragment de type EGF de la protéine humaine nétrine 4 mutée, ledit fragment étant codé par la séquence nucléotidique SEQ ID NO : 383. Ce fragment comprend 56 acides aminés et correspond au fragment de la protéine nétrine 4 mutée allant du résidu 332 au résidu 387 de la séquence SEQ ID NO : 522.

Les fragments de la protéine nétrine 4 mutée correspondant aux séquences protéiques SEQ ID NO : 380 à SEQ ID NO : 496, ainsi que les séquences nucléotidiques correspondantes SEQ ID NO : 379 à SEQ ID NO : 495 sont regroupés dans le tableau qui suit :

| **Séquence protéique** | **Séquence nucléotidique** | **Positions du fragment par rapport** **à la séquence SEQ ID NO : 522** |
|---|---|---|
| SEQ ID NO : 380 | SEQ ID NO : 379 | 32-515 |
| SEQ ID NO : 382 | SEQ ID NO : 381 | 32-628 |
| SEQ ID NO : 384 | SEQ ID NO : 383 | 332-387 |
| SEQ ID NO : 386 | SEQ ID NO : 385 | 32-260 |
| SEQ ID NO : 388 | SEQ ID NO : 387 | 1-260 + 516-628 |
| SEQ ID NO : 390 | SEQ ID NO : 389 | 261-628 |
| SEQ ID NO : 392 | SEQ ID NO : 391 | 1-320 |
| SEQ ID NO : 394 | SEQ ID NO : 393 | 1-260 + 332-387 |
| SEQ ID NO : 396 | SEQ ID NO : 395 | 1-260 + 394-445 |
| SEQ ID NO : 398 | SEQ ID NO : 397 | 32-320 |
| SEQ ID NO : 400 | SEQ ID NO : 399 | 32-260 + 332-387 |
| SEQ ID NO : 402 | SEQ ID NO : 401 | 32-260 + 394-445 |
| SEQ ID NO : 404 | SEQ ID NO : 403 | 261-320 + 332-387 |
| SEQ ID NO : 406 | SEQ ID NO : 405 | 332-387 + 394-445 |
| SEQ ID NO : 408 | SEQ ID NO : 407 | 261-320 +332-387 + 394-445 |
| SEQ ID NO : 410 | SEQ ID NO : 409 | 1-320 + 332-387 |
| SEQ ID NO : 412 | SEQ ID NO : 411 | 1-260 + 332-387 + 394-445 |
| SEQ ID NO : 414 | SEQ ID NO : 413 | 1-320 + 394-445 |
| SEQ ID NO : 416 | SEQ ID NO : 415 | 32-320 + 332-387 |
| SEQ ID NO : 418 | SEQ ID NO : 417 | 32-260 + 332-387 + 394-445 |
| SEQ ID NO : 420 | SEQ ID NO : 419 | 32-320 + 394-445 |
| SEQ ID NO : 422 | SEQ ID NO : 421 | 1-320 +332-387 + 394-445 |
| SEQ ID NO : 424 | SEQ ID NO : 423 | 32-320 +332-387 + 394-445 |
| SEQ ID NO : 426 | SEQ ID NO : 425 | 332-387 + 516-628 |
| SEQ ID NO : 428 | SEQ ID NO : 427 | 32-260 + 516-628 |
| SEQ ID NO : 430 | SEQ ID NO : 429 | 32-320 + 516-628 |
| SEQ ID NO : 432 | SEQ ID NO : 431 | 132-260 + 332-387 + 516-628 |
| SEQ ID NO : 434 | SEQ ID NO : 433 | 32-260 + 394-445 + 516-628 |
| SEQ ID NO : 436 | SEQ ID NO : 435 | 1-320 + 516-628 |
| SEQ ID NO : 438 | SEQ ID NO : 437 | 1-260 + 332-387 + 516-628 |
| SEQ ID NO : 440 | SEQ ID NO : 439 | 1-260 + 394-445 + 516-628 |
| SEQ ID NO : 442 | SEQ ID NO : 441 | 261-320 + 332-387 + 516-628 |
| SEQ ID NO : 444 | SEQ ID NO : 443 | 332-387 + 394-445 + 516-628 |
| SEQ ID NO : 446 | SEQ ID NO : 445 | 261-320 + 332-387 + 394-445 + 516-628 |
| SEQ ID NO : 448 | SEQ ID NO : 447 | 1-320 + 332-387 + 516-628 |
| SEQ ID NO : 450 | SEQ ID NO : 449 | 1-260 + 332-387 + 394-445 + 516-628 |
| SEQ ID NO : 452 | SEQ ID NO : 451 | 1-320 + 394-445 + 516-628 |
| SEQ ID NO : 454 | SEQ ID NO : 453 | 32-320 + 332-387 + 516-628 |
| SEQ ID NO : 456 | SEQ ID NO : 455 | 32-260 + 332-387 + 394-445 + 516-628 |
| SEQ ID NO : 458 | SEQ ID NO : 457 | 32-320 + 394-445 + 516-628 |
| SEQ ID NO : 460 | SEQ ID NO : 459 | 1-320 + 332-387 + 394-445 + 516-628 |
| SEQ ID NO : 462 | SEQ ID NO : 461 | 32-320 + 332-387 + 394-445 + 516-628 |
| SEQ ID NO : 464 | SEQ ID NO : 463 | 20-260 |
| SEQ ID NO : 466 | SEQ ID NO : 465 | 20-516 |
| SEQ ID NO : 468 | SEQ ID NO : 467 | 20-260 + 516-628 |
| SEQ ID NO : 470 | SEQ ID NO : 469 | 20-320 |
| SEQ ID NO : 472 | SEQ ID NO : 471 | 20-260 + 332-387 |
| SEQ ID NO : 474 | SEQ ID NO : 473 | 20-260 + 394-445 |
| SEQ ID NO : 476 | SEQ ID NO : 475 | 20-320 + 332-387 |
| SEQ ID NO : 478 | SEQ ID NO : 477 | 20-260 + 332-387 + 394-445 |
| SEQ ID NO : 480 | SEQ ID NO : 479 | 20-320 + 394-445 |
| SEQ ID NO : 482 | SEQ ID NO : 481 | 20-320 +332-387 + 394-445 |
| SEQ ID NO : 484 | SEQ ID NO : 483 | 20-320 + 516-628 |
| SEQ ID NO : 486 | SEQ ID NO : 485 | 20-260 + 332-387 + 516-628 |
| SEQ ID NO : 488 | SEQ ID NO : 487 | 20-260 + 394-445 + 516-628 |
| SEQ ID NO : 490 | SEQ ID NO : 489 | 20-320 + 332-387 + 516-628 |
| SEQ ID NO : 492 | SEQ ID NO : 491 | 20-260 + 332-387 + 394-445 + 516-628 |
| SEQ ID NO : 494 | SEQ ID NO : 493 | 20-320 + 394-445 + 516-628 |
| SEQ ID NO : 496 | SEQ ID NO : 495 | 20-320 + 332-387 + 394-445 + 516-628 |

La séquence SEQ ID NO : 526 correspond au fragment délimité des positions 1 à 288 de la SEQ ID NO : 522.

La séquence SEQ ID NO : 528 correspond au fragment délimité des positions 1 à 288 de la SEQ ID NO : 522, fusionné à une séquence peptidique de 24 acides aminés.

La présente invention concerne une protéine caractérisée en ce qu'elle comprend ou est constituée par :
* la séquence SEQ ID NO : 522 ou SEQ ID NO : 524, ou
* un fragment de la dite séquence SEQ ID NO : 522 ou SEQ ID NO : 524, sous réserve que ce fragment présente une activité anti-angiogénique, ledit fragment comprenant notamment environ 40 à environ 450 acides aminés, et de préférence d'environ 40 à environ 260 acides aminés, et de préférence d'environ 40 à environ 230 acides aminés, et étant notamment représenté par l'une des séquences SEQ ID NO : 2q, q variant de 187 à 248, ou par la séquence SEQ ID NO : 526 ou par la séquence SEQ ID NO : 528,
* toute séquence dérivée de l'une des séquences susmentionnées, notamment par substitution, suppression ou addition d'un ou plusieurs acides aminés, sous réserve que cette séquence dérivée présente une activité anti-angiogénique, ou
* toute séquence homologue de l'une des séquences susmentionnées, ayant de préférence une identité d'au moins environ 50% avec la région comprise entre les acides aminés en position 261 et 515 de SEQ ID NO : 522, sous réserve que cette séquence homologue présente une activité anti-angiogénique.

L'activité d'inhibition de l'angiogenèse est également désignée activité anti-angiogénique. Cette activité peut être par exemple mise en évidence *in vitro* en démontrant l'inhibition à la fois de la multiplication, de la migration et de la différenciation, de cellules endothéliales par la protéine susmentionnée. La mesure de l'inhibition de la multiplication des cellules endothéliales peut être réalisée en cultivant des cellules endothéliales en présence de la protéine ou du fragment dont on souhaite évaluer l'activité. La mesure de l'inhibition de la migration des cellules endothéliales peut être réalisée en effectuant une "blessure" sur un tapis de cellules endothéliales et en incubant ensuite les cellules en présence du fragment à tester. On mesure alors le nombre de cellules ayant migré sur la blessure. La mesure de l'inhibition de la différenciation (tubulogenèse) des cellules endothéliales peut être réalisée en mesurant la longueur de tubules formés par des cellules endothéliales cultivées sur gel en présence du fragment à tester.

Parmi les modèles classiques de mesure d'angiogenèse, on peut citer les modèles par délivrance locale comme :
- l'injection sous-cutanée de Matrigel (Becton Dickinson) imprégné du composé de l'invention (Inoki et al., 2002), ou
- le dépôt sur la membrane chorio-allantoïdienne de poulet d'un implant contenant un composé de l'invention (Celerier et al., 2002).

Alternativement, les fragments de l'invention peuvent être injectés par voie systémique (intraveineuse, intrapéritonéale, sous-cutanée) à des animaux chez qui on a créé une maladie angiogénique expérimentale. Les fragments de l'invention peuvent aussi être injectés directement dans une tumeur. Alternativement les fragments ou les anticorps anti-idiotypiques selon l'invention (décrits ci-après) peuvent être délivrés par une méthode de thérapie génique par voie locale ou systémique par toute méthode permettant l'expression des fragments ou des anticorps anti-idiotypiques selon l'invention. Alternativement les fragments ou les anticorps anti-idiotypiques selon l'invention peuvent être insérés dans un plasmide qui est transfecté dans les cellules cancéreuses. Tous ces procédés de mesure sont notamment décrits dans l'article de Jain et al. (1997).

On désigne par activité anti-tumorale, une activité permettant d'inhiber la croissance tumorale et/ou d'induire la régression, voire la disparition de tumeurs. Cette activité peut être par exemple mise en évidence *in vivo* en mesurant la masse de tumeurs, dont on a induit le développement chez la souris par injection de cellules tumorales, en présence et en absence d'administration de séquences peptidiques de l'invention et/ou d'acides nucléiques exprimant les séquences peptidiques de l'invention.

La présente invention concerne une protéine caractérisée en ce qu'elle comprend ou est constituée par :
* la séquence SEQ ID NO : 522 ou SEQ ID NO : 524, ou
* un fragment de la dite séquence SEQ ID NO : 522 ou SEQ ID NO : 524, sous réserve que ce fragment présente une activité d'activation des péricytes, ledit fragment comprenant notamment environ 40 à environ 450 acides aminés, et de préférence d'environ 40 à environ 260 acides aminés, et de préférence d'environ 40 à environ 230 acides aminés, et étant notamment représenté par l'une des séquences SEQ ID NO : 2q, q variant de 187 à 248, ou par la séquence SEQ ID NO : 526, ou par la séquence SEQ ID NO : 528,

* toute séquence dérivée de l'une des séquences susmentionnées, notamment par substitution, suppression ou addition d'un ou plusieurs acides aminés, sous réserve que cette séquence dérivée présente une activité d'activation des péricytes , ou
* toute séquence homologue de l'une des séquences susmentionnées, ayant de préférence une identité d'au moins environ 50% avec la région comprise entre les acides aminés en position 261 et 515 de SEQ ID NO : 522, sous réserve que cette séquence homologue présente une activité d'activation des péricytes.

L'activité d'activation des péricytes est vérifiée notamment par les tests de prolifération et de migration tels que définis ci-après et dans la partie expérimentale.

La présente invention découle notamment de la mise en évidence par les Inventeurs de la fonction d'activation des péricytes par la liaison des nétrines aux récepteurs UNC5H4 des péricytes et des cellules musculaires lisses.

La présente invention concerne également une séquence nucléotidique codant pour une protéine telle que définie ci-dessus, à savoir une séquence nucléotidique codant pour de la nétrine 4 mutée.

Une séquence nucléotidique préférée selon l'invention est une séquence nucléotidique caractérisée en ce qu'elle comprend ou est constituée par :
- la séquence nucléotidique SEQ ID NO : 521 codant pour SEQ ID NO : 522, ou la séquence nucléotidique SEQ ID NO : 523 codant pour SEQ ID NO : 524,
- un fragment de l'une de ces séquences nucléotidiques, et notamment représenté par l'une des séquences SEQ ID NO : 2q-l, q variant de 187 à 248, ou par la séquence SEQ ID NO : 525 ou par la séquence SEQ ID NO : 527,
- ou toute séquence nucléotidique dérivée, par dégénérescence du code génétique, de l'une des séquences nucléotidiques susmentionnées, et codant pour une protéine représentée par l'une des séquences SEQ ID NO : 2q, q variant de 187 à 248, ou pour la séquence SEQ ID NO : 526 ou pour la séquence SEQ ID NO : 528,
- ou toute séquence nucléotidique dérivée, notamment par substitution, suppression ou addition d'un ou plusieurs nucléotides, de l'une des séquences nucléotidiques susmentionnées codant pour une protéine dérivée de SEQ ID NO : 2q, q variant de 187 à 248, ou pour la séquence SEQ ID NO : 526 ou pour la séquence SEQ ID NO : 528, telle que définie ci-dessus,
- ou toute séquence nucléotidique homologue de l'une des séquences nucléotidiques susmentionnées, ayant de préférence une identité d'au moins environ 50%, avec l'une des séquences SEQ ID NO : 2q-1 codant pour une protéine homologue de SEQ ID NO : 2q, ou avec la séquence SEQ ID NO : 525 codant pour la séquence SEQ ID NO : 526 ou avec la séquence SEQ ID NO : 527 codant pour la séquence SEQ ID NO : 528, telle que définie ci-dessus,
- ou toute séquence nucléotidique complémentaire des séquences susmentionnées,
- ou toute séquence nucléotidique susceptible d'hybrider dans des conditions stringentes avec la séquence complémentaire d'une des séquences susmentionnées.

L'expression "hybrider dans des conditions stringentes" correspond notamment à un tampon d'hybridation tel que 0,5X SSC, à une température d'hybridation de 60°C, ainsi qu'à un milieu de lavage tel que 0,1X SSC additionné de 1% de SDS et à une température de lavage de 50°C.

Les séquences SEQ ID NO : 2q-1 susmentionnées codent pour les fragments de nétrine 4 mutée susmentionnés, représentés par SEQ ID NO : 2q, et correspondent aux séquences nucléotidiques suivantes : SEQ ID NO : 373 codant pour SEQ ID NO : 374, SEQ ID NO : 375 codant pour SEQ ID NO : 376, SEQ ID NO : 377 codant pour SEQ ID NO : 378, SEQ ID NO : 379 codant pour SEQ ID NO : 380, SEQ ID NO : 381 codant pour SEQ ID NO : 382, SEQ ID NO : 383 codant pour SEQ ID NO : 384, SEQ ID NO : 385 codant pour SEQ ID NO : 386, SEQ ID NO : 387 codant pour SEQ ID NO : 388, SEQ ID NO : 389 codant pour SEQ ID NO : 390, SEQ ID NO : 391 codant pour SEQ ID NO : 392, SEQ ID NO : 393 codant pour SEQ ID NO : 394, SEQ ID NO : 395 codant pour SEQ ID NO : 396, SEQ ID NO : 397 codant pour SEQ ID NO : 398, SEQ ID NO : 399 codant pour SEQ ID NO : 400, SEQ ID NO : 401 codant pour SEQ ID NO : 402, SEQ ID NO : 403 codant pour SEQ ID NO : 404, SEQ ID NO : 405 codant pour SEQ ID NO : 406, SEQ ID NO : 407 codant pour SEQ ID NO : 408, SEQ ID NO : 409 codant pour SEQ ID NO : 410, SEQ ID NO : 411 codant pour SEQ ID NO : 412, SEQ ID NO : 413 codant pour SEQ ID NO : 414, SEQ ID NO : 415 codant pour SEQ ID NO : 416, SEQ ID NO : 417 codant pour SEQ ID NO : 418, SEQ ID NO : 419 codant pour SEQ ID NO : 420, SEQ ID NO : 421 codant pour SEQ ID NO : 422, SEQ ID NO : 423 codant pour SEQ ID NO : 424, SEQ ID NO : 425 codant pour SEQ ID NO : 426, SEQ ID NO : 427 codant pour SEQ ID NO : 428, SEQ ID NO : 429 codant pour SEQ ID NO : 430, SEQ ID NO : 431 codant pour SEQ ID NO : 432, SEQ ID NO : 433 codant pour SEQ ID NO : 434, SEQ ID NO : 435 codant pour SEQ ID NO : 436, SEQ ID NO : 437 codant pour SEQ ID NO : 438, SEQ ID NO : 439 codant pour SEQ ID NO : 440, SEQ ID NO : 441 codant pour SEQ ID NO : 442, SEQ ID NO : 443 codant pour SEQ ID NO : 444, SEQ ID NO : 445 codant pour SEQ ID NO : 446, SEQ ID NO : 447 codant pour SEQ ID NO : 448, SEQ ID NO : 449 codant pour SEQ ID NO : 450, SEQ ID NO : 451 codant pour SEQ ID NO : 452, SEQ ID NO : 453 codant pour SEQ ID NO : 454, SEQ ID NO : 455 codant pour SEQ ID NO : 456, SEQ ID NO : 457 codant pour SEQ ID NO : 458, SEQ ID NO : 459 codant pour SEQ ID NO : 460, SEQ ID NO : 461 codant pour SEQ ID NO : 462, SEQ ID NO : 463 codant pour SEQ ID NO : 464, SEQ ID NO : 465 codant pour SEQ ID NO : 466, SEQ ID NO : 467 codant pour SEQ ID NO : 468, SEQ ID NO : 469 codant pour SEQ ID NO : 470, SEQ ID NO : 471 codant pour SEQ ID NO : 472, SEQ ID NO : 473 codant pour SEQ ID NO : 474, SEQ ID NO : 475 codant pour SEQ ID NO : 476, SEQ ID NO : 477 codant pour SEQ ID NO : 478, SEQ ID NO : 479 codant pour SEQ ID NO : 480, SEQ ID NO : 481 codant pour SEQ ID NO : 482, SEQ ID NO : 483 codant pour SEQ ID NO : 484, SEQ ID NO : 485 codant pour SEQ ID NO : 486, SEQ ID NO : 487 codant pour SEQ ID NO : 488, SEQ ID NO : 489 codant pour SEQ ID NO : 490, SEQ ID NO : 491 codant pour SEQ ID NO : 492, SEQ ID NO : 493 codant pour SEQ ID NO : 494 ou SEQ ID NO : 495 codant pour SEQ ID NO : 496.

La séquence SEQ ID NO : 525 code pour la séquence SEQ ID NO : 526.

La séquence SEQ ID NO : 527 code pour la séquence SEQ ID NO : 528.

La présente invention concerne également un vecteur recombinant, notamment plasmide, cosmide, phage ou ADN de virus, contenant une séquence nucléotidique telle que définie ci-dessus, à savoir une séquence nucléotidique codant pour la nétrine 4 mutée ou l'un de ses fragments tels que définis ci-dessus, ledit vecteur recombinant étant notamment caractérisé en ce qu'il contient les éléments nécessaire à l'expression dans une cellule hôte des polypeptides codés par les acides nucléiques susmentionnés, insérés dans ledit vecteur.

La présente invention concerne également une cellule hôte, choisie notamment parmi les bactéries, les virus, les levures, les champignons, les plantes ou les cellules de mammifères, ladite cellule hôte étant transformée, notamment à l'aide d'un vecteur recombinant tel que défini ci-dessus.

La présente invention concerne également un anticorps caractérisé en ce qu'il est dirigé de manière spécifique contre une protéine telle que définie ci-dessus, notamment dirigé contre la nétrine 4 mutée ou l'un de ses fragments tels que définis ci-dessus.

La présente invention concerne également un anticorps anti-idiotypique d'une protéine telle que définie ci-dessus, notamment dirigé contre la nétrine 4 mutée ou l'un de ses fragments tels que définis ci-dessus.

La présente invention concerne également un fragment Fab d'anticorps anti-idiotypiques tels que définis ci-dessus, notamment dirigé contre la nétrine 4 mutée ou l'un de ses fragments tels que définis ci-dessus.

La présente invention concerne également une composition pharmaceutique, comprenant à titre de substance active,
- une protéine telle que définie ci-dessus, ou
- une séquence nucléotidique telle que définie ci-dessus , ou
- un anticorps tel que défini ci-dessus , ou
- un anticorps anti-idiotypique tel que défini ci-dessus, ou
- un fragment Fab d'anticorps anti-idiotypiques tels que définis ci-dessus.

La présente invention concerne également l'utilisation telle que définie ci-dessus de la protéine nétrine 4 mutée, représentée par la séquence SEQ ID NO : 522 ou SEQ ID NO : 524, pour la préparation d'un médicament susceptible d'être administré à raison d'environ 0,1 à environ 2000 µg/kg, notamment par voie intraveineuse, par voie sous-cutanée, par voie systémique, par injection intra-vitréenne, par voie locale au moyen d'infiltrations ou par l'intermédiaire d'un collyre, éventuellement associé à une électroperméation.

La protéine nétrine 4 mutée peut également être délivrée par une injection de plasmide codant pour la protéine d'intérêt nétrine 4 mutée.

Alternativement, l'une quelconque des nétrines de l'invention ou l'un de ses fragments peut être délivré par tout procédé de dispositif intravasculaire (stents) après fixation de la protéine ou du fragment sur ledit dispositif.

La présente invention concerne l'utilisation :
- d'une protéine telle que définie ci-dessus ou,
- d'une séquence nucléotidique telle que définie ci-dessus ou,
- d'un anticorps anti-idiotypique tel que défini ci-dessus,
pour la préparation d'un médicament destiné à la prévention ou au traitement des pathologies nécessitant l'inhibition de la prolifération et/ou de la migration endothéliale, notamment dans le cadre des pathologies suivantes : les cancers et les leucémies, la dégénérescence maculaire liée à l'âge, la néovascularisation choroïdienne compliquant la myopie, la néovascularisation de la cornée en particulier le rejet de greffe, les glaucomes, les rétinopathies diabétiques ou du prématuré, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

L'expression "inhibition de la prolifération endothéliale" désigne toute substance capable de freiner la prolifération de cellules endothéliales selon le test de prolifération décrit ci-après.

La présente invention concerne le produit de combinaison comprenant:
- de la nétrine 4 mutée représentée par SEQ ID NO : 522 ou SEQ ID NO : 524, ou
- d'un fragment de la dite séquence SEQ ID NO : 522 ou SEQ ID NO : 524, notamment représenté par l'une des séquences SEQ ID NO : 2q, q variant de 187 à 248, ou par la séquence SEQ ID NO : 526 ou la séquence SEQ ID NO : 528,
en association avec un agent anti-angiogénique notamment choisi parmi : AVASTIN (bevacizumab) développé par Genentech et Roche, MACUGEN (pegaptanib) développé par Eyetech et Pfizer et LUCENTIS (ranibizumab) développé par Genentech et Novartis, pour une utilisation simultanée, séparée ou étalée dans le temps destiné au traitement des pathologies suivantes : les cancers et les leucémies, la dégénérescence maculaire liée à l'âge, la néovascularisation choroïdienne compliquant la myopie, la néovascularisation de la cornée en particulier le rejet de greffe, les glaucomes, les rétinopathies diabétiques ou du prématuré, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

Dans le cadre de la présente invention, les doses de la nétrine 4 mutée sont comprises d'environ 10 à environ 10 000 ng/injection, préférentiellement d'environ 100 à environ 5 000 ng/injection, toutes les 6 semaines.

Dans le cadre de la présente invention, les doses d'agent anti-angiogénique (AVASTIN, MACUGEN ou LUCENTIS par exemple) sont comprises d'environ 0,3 et 1mg toutes les 6 semaines.

L'invention a pour objet l'utilisation :
- d'un anticorps tel que défini ci-dessus, ou
- un fragment Fab d'anticorps anti-idiotypiques tels que définis ci-dessus,
pour la préparation d'un médicament destiné à la prévention ou au traitement des pathologies nécessitant la stimulation de la prolifération et/ou de la migration endothéliale, notamment dans le cadre des pathologies suivantes : pathologies ischémiques telles que l'artérite des membres inférieurs, l'infarctus du myocarde, les accidents vasculaires cérébraux, la sclérodermie ou la maladie de Raynaud.

La mesure de l'activation de la prolifération des cellules endothéliales peut être réalisée en plaçant les cellules endothéliales dans un milieu de culture approprié et en mesurant ensuite le nombre total de cellules.

La mesure de l'activation de la migration des cellules endothéliales peut être réalisée en effectuant une "blessure" sur un tapis de cellules et en incubant ensuite les cellules en présence de la protéine, ou de la séquence nucléotidique ou de l'anticorps anti-idiotypique à tester. On mesure alors le nombre de cellules ayant migré sur la blessure.

La présente invention concerne l'utilisation :
- d'une protéine telle que définie ci-dessus
- d'une séquence nucléotidique telle que définie ci-dessus, ou
- d'un anticorps tel que défini ci-dessus, ou
- d'un anticorps anti-idiotypique tel que défini ci-dessus, ou
- d'un fragment Fab d'anticorps anti-idiotypiques tel que défini ci-dessus,
pour la préparation d'un médicament destiné à la prévention ou au traitement des pathologies non tumorales liées à, ou causées par, une raréfaction des péricytes ou des cellules musculaires lisses.

La présente invention concerne l'utilisation :
- d'une protéine telle que définie ci-dessus
- d'une séquence nucléotidique telle que définie ci-dessus, ou
- d'un anticorps anti-idiotypique tel que défini ci-dessus, ou
pour la préparation d'un médicament destiné à la prévention ou au traitement des pathologies tumorales liées à, ou causées par, une raréfaction des péricytes ou des cellules musculaires lisses.

L'utilisation selon la présente invention est caractérisée en ce que les pathologies non tumorales liées à, ou causées par, une raréfaction des péricytes ou des cellules musculaires lisses, et nécessitant une activation de la prolifération ou de la migration des péricytes ou des cellules musculaires lisses, sont choisies parmi :
- la dégénérescence maculaire liée à l'âge,
- la néovascularisation choroidienne compliquant la myopie,
- les rétinopathies diabétiques ou du prématuré,
- le glaucome néovasculaire (de la cornée, de la rétine...)
- les néovascularisations cornéennes en particulier le rejet de greffe
- la polyarthrite rhumatoïde,
- le psoriasis, en particulier la polyarthrite psoriasique,
- les angiomes,
- l'athérosclérose,
- l'obésité,
- les malformations intestinales,
- la maladie de Crohn,
- les démences vasculaires, sous-corticales vasculaires dont Cadasil est un exemple,
- la maladie d'Alzheimer,
- les pathologies dégénératives osseuses et les fractures, et
- les anévrysmes et les dissections vasculaires.

Selon un mode de réalisation avantageux, l'utilisation selon la présente invention est caractérisée en ce que les pathologies non tumorales liées à, ou causées par, une raréfaction des péricytes ou des cellules musculaires lisses, et nécessitant une activation de la prolifération ou de la migration des péricytes ou des cellules musculaires lisses, sont choisies parmi :
- les rétinopathies diabétiques,
- les malformations intestinales,
- la maladie de Crohn,
- l'athérosclérose,
- l'obésité,
- les démences vasculaires, telles que la maladie d'Alzheimer,
- les pathologies dégénératives osseuses et les fractures, et
- les anévrysmes et les dissections vasculaires.

La présente invention concerne également l'utilisation telle que définie ci-dessus, caractérisée en ce que l'activité d'activation de la prolifération ou de la migration des péricytes ou des cellules musculaires lisses est mesurée selon le test de prolifération ou de migration, et en ce que cette activité d'activation correspond à au moins 120% des cellules obtenues en l'absence de la protéine, ou de la séquence nucléotidique ou de l'anticorps anti-idiotypique tels que définis ci-dessus.

Cette propriété peut être utilisée pour expandre des cellules musculaires lisses ou des péricytes à partir de tout prélèvement de cellules progénitrices ou de cellules souches afin de pratiquer des thérapies cellulaires.

La mesure de l'activation de la migration des péricytes ou des cellules musculaires lisses peut être réalisée en effectuant une "blessure" sur un tapis de cellules et en incubant ensuite les cellules en présence de la protéine, ou de la séquence nucléotidique ou de l'anticorps anti-idiotypique à tester. On mesure alors le nombre de cellules ayant migré sur la blessure.

La mesure de l'activation de la prolifération des péricytes ou des cellules musculaires lisses peut être réalisée en plaçant les péricytes ou cellules musculaires lisses dans un milieu de culture approprié, notamment dans du milieu DMEM ne contenant pas de sérum, et en mesurant ensuite le nombre total de cellules.

La présente invention concerne l'utilisation:
- de la nétrine 4 mutée représentée par SEQ ID NO : 522 ou SEQ ID NO : 524, ou
- d'un fragment de la dite séquence SEQ ID NO : 522 ou SEQ ID NO : 524, notamment représenté par l'une des séquences SEQ ID NO : 2q, q variant de 187 à 248, ou par la séquence SEQ ID NO : 526 ou par la séquence SEQ ID NO : 528,
en association avec un agent de chimiothérapie, pour la préparation d'un médicament destiné au traitement des cancers.

La présente invention concerne le produit de combinaison :
- de la nétrine 4 mutée représentée par SEQ ID NO : 522 ou SEQ ID NO : 524, ou
- d'un fragment de la dite séquence SEQ ID NO : 522 ou SEQ ID NO : 524, notamment représenté par l'une des séquences SEQ ID NO : 2q, q variant de 187 à 248, ou par la séquence SEQ ID NO : 526 ou par la séquence SEQ ID NO : 528,
en association avec un agent de chimiothérapie, pour une utilisation simultanée, séparée ou étalée dans le temps destiné au traitement des pathologies tumorales.

La présente invention concerne le produit de combinaison comprenant:
- de la nétrine 4 mutée représentée par SEQ ID NO : 522 ou SEQ ID NO : 524, ou
- d'un fragment de la dite séquence SEQ ID NO : 522 ou SEQ ID NO : 524, notamment représenté par l'une des séquences SEQ ID NO : 2q, q variant de 187 à 248, ou par la séquence SEQ ID NO : 526 ou par la séquence SEQ ID NO : 528,
en association avec un agent anti-angiogénique notamment choisi parmi : AVASTIN (bevacizumab) développé par Genentech et Roche, MACUGEN (pegaptanib) développé par Eyetech et Pfizer et LUCENTIS (ranibizumab) développé par Genentech et Novartis, ou tout autre agent anti-VEGF pour une utilisation simultanée, séparée ou étalée dans le temps destiné au traitement ou à la prévention des pathologies tumorales ou non tumorales telles que définies ci-dessus.

La présente invention concerne l'utilisation :
- de la nétrine 4 mutée représentée par SEQ ID NO : 522 ou SEQ ID NO : 524, ou
- d'un fragment de la dite séquence SEQ ID NO : 522 ou SEQ ID NO : 524, notamment représenté par l'une des séquences SEQ ID NO : 2q, q variant de 187 à 248, ou par la séquence SEQ ID NO : 526 ou par la séquence SEQ ID NO : 528,
en association avec un agent anti-angiogénique notamment choisi parmi : *AVASTIN* (bevacizumab) développé par Genentech et Roche, MACUGEN (pegaptanib) développé par Eyetech et Pfizer et LUCENTIS (ranibizumab) développé par Genentech et Novartis, ou tout autre agent anti-VEGF pour la préparation d'un médicament destiné à la prévention ou au traitement des pathologies tumorales ou non tumorales telles que définies ci-dessus.

La présente invention concerne également l'utilisation combinée d'un agent anti-angiogénique et d'un agent de chimiothérapie.

En particulier, la présente invention concerne l'utilisation d'un ligand de la néogénine, ou le cas échéant d'une séquence nucléotidique codant pour un ligand de la néogénine, ou d'un vecteur contenant une séquence nucléotidique codant pour ledit ligand, ou d'une cellule hôte transformée par ledit vecteur, ou d'un anticorps anti-idiotypique dirigé contre ledit ligand, en association avec un agent de chimiothérapie, pour la préparation d'un médicament destiné au traitement des cancers.

La combinaison d'un agent anti-angiogénique, choisi parmi ledit ligand de la néogénine ou ladite séquence nucléotidique ou ledit vecteur ou ladite cellule hôte ou ledit anticorps anti-idiotypique, avec un agent de chimiothérapie permet d'obtenir un effet synergique et d'induire une moindre résistance aux traitements anti-tumoraux conventionnels.

Parmi les agents de chimiothérapie préférés selon l'invention, on peut notamment citer la doxorubicine, le méthotrexate, la vinblastine, la vincristine, la cladribine, le fluorouracile, la cytarabine, les anthracyclines, le cisplatine, le cyclophosphamide, la fludarabine, la gemcitabine, les inhibiteurs de l'aromatase, l'irinotecan, la navelbine, l'oxaliplatine, le taxofène, le taxol et le taxotère.

Selon un mode de réalisation avantageux, l'utilisation selon l'invention est caractérisée en ce que le ligand ou la séquence nucléotidique ou l'anticorps anti-idiotypique est choisi parmi :
- l'une des protéines suivantes : la nétrine 1 représentée par SEQ ID NO : 502 ou SEQ ID NO : 504, la nétrine G1 représentée par SEQ ID NO : 506 ou SEQ ID NO : 508, la nétrine 3 représentée par SEQ ID NO : 510, la nétrine 4 représentée par SEQ ID NO : 498 ou SEQ ID NO : 500, l'une des molécules RGM représentées par SEQ ID NO : 512, SEQ ID NO : 514, SEQ ID NO : 516, SEQ ID NO : 518 ou SEQ ID NO : 520, ou la nétrine 4 mutée représentée par SEQ ID NO : 522 ou SEQ ID NO : 524,
- ou un fragment de l'une des ces protéines, notamment représenté par l'une des séquences SEQ ID NO : 2n, n variant de 1 à 248,
- ou une séquence nucléotidique codant pour l'une des protéines susmentionnées, notamment représentée par l'une des séquences nucléotidiques suivantes : SEQ ID NO : 501, SEQ ID NO : 503, SEQ ID NO : 505, SEQ ID NO : 507, SEQ ID NO : 509, SEQ ID NO : 497, SEQ ID NO : 499, SEQ ID NO : 511, SEQ ID NO : 513, SEQ ID NO : 515, SEQ ID NO : 517, SEQ ID NO : 519, SEQ ID NO : 521, SEQ ID NO : 523, ou l'une des séquences SEQ ID NO : 2n-1, n variant de 1 à 248,
- ou un anticorps anti-idiotypique de l'une des protéines telles que définies ci-dessus.

Les séquences SEQ ID NO : 2n susmentionnées correspondent aux séquences protéiques SEQ ID NO : 2 à SEQ ID NO : 496.

Les séquences SEQ ID NO : 2n susmentionnées correspondent aux séquences protéiques suivantes : SEQ ID NO : 2, SEQ ID NO : 4, SEQ ID NO : 6, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22, SEQ ID NO : 24, SEQ ID NO : 26, SEQ ID NO : 28, SEQ ID NO : 30, SEQ ID NO : 32, SEQ ID NO : 34, SEQ ID NO : 36, SEQ ID NO : 38, SEQ ID NO :40, SEQ ID NO : 42, SEQ ID NO : 44, SEQ ID NO : 46, SEQ ID NO : 48, SEQ ID NO : 50, SEQ ID NO : 52, SEQ ID NO : 54, SEQ ID NO : 56, SEQ ID NO : 58, SEQ ID NO : 60, SEQ ID NO : 62, SEQ ID NO : 64, SEQ ID NO : 66, SEQ ID NO : 68, SEQ ID NO : 70, SEQ ID NO : 72, SEQ ID NO : 74, SEQ ID NO : 76, SEQ ID NO : 78, SEQ ID NO : 80, SEQ ID NO : 82, SEQ ID NO : 84, SEQ ID NO : 86, SEQ ID NO : 88, SEQ ID NO : 90, SEQ ID NO : 92, SEQ ID NO : 94, SEQ ID NO : 96, SEQ ID NO : 98, SEQ ID NO : 100, SEQ ID NO : 102, SEQ ID NO : 104, SEQ ID NO : 106, SEQ ID NO : 108, SEQ ID NO : 110, SEQ ID NO : 112, SEQ ID NO : 114, SEQ ID NO : 116, SEQ ID NO : 118, SEQ ID NO : 120, SEQ ID NO : 122, SEQ ID NO : 124, SEQ ID NO : 126, SEQ ID NO : 128, SEQ ID NO : 130, SEQ ID NO : 132, SEQ ID NO : 134, SEQ ID NO : 136, SEQ ID NO : 138, SEQ ID NO : 140, SEQ ID NO : 142, SEQ ID NO : 144, SEQ ID NO : 146, SEQ ID NO : 148, SEQ ID NO : 150, SEQ ID NO : 152, SEQ ID NO : 154, SEQ ID NO : 156, SEQ ID NO : 158, SEQ ID NO : 160, SEQ ID NO : 162, SEQ ID NO : 164, SEQ ID NO : 166, SEQ ID NO : 168, SEQ ID NO : 170, SEQ ID NO : 172, SEQ ID NO : 174, SEQ ID NO : 176, SEQ ID NO : 178, SEQ ID NO : 180, SEQ ID NO : 182, SEQ ID NO : 184, SEQ ID NO : 186, SEQ ID NO : 188, SEQ ID NO : 190, SEQ ID NO : 192, SEQ ID NO : 194, SEQ ID NO : 196, SEQ ID NO : 198, SEQ ID NO : 200, SEQ ID NO : 202, SEQ ID NO : 204, SEQ ID NO : 206, SEQ ID NO : 208, SEQ ID NO : 210, SEQ ID NO : 212, SEQ ID NO : 214, SEQ ID NO : 216, SEQ ID NO : 218, SEQ ID NO : 220, SEQ ID NO : 222, SEQ ID NO : 224, SEQ ID NO : 226, SEQ ID NO : 228, SEQ ID NO : 230, SEQ ID NO : 232, SEQ ID NO : 234, SEQ ID NO : 236, SEQ ID NO : 238, SEQ ID NO : 240, SEQ ID NO : 242, SEQ ID NO : 244, SEQ ID NO : 246, SEQ ID NO : 248, SEQ ID NO : 250, SEQ ID NO : 252, SEQ ID NO : 254, SEQ ID NO : 256, SEQ ID NO : 258, SEQ ID NO : 260, SEQ ID NO : 262, SEQ ID NO : 264, SEQ ID NO : 266, SEQ ID NO : 268, SEQ ID NO : 270, SEQ ID NO : 272, SEQ ID NO : 274, SEQ ID NO : 276, SEQ ID NO : 278, SEQ ID NO : 280, SEQ ID NO : 282, SEQ ID NO : 284, SEQ ID NO : 286, SEQ ID NO : 288, SEQ ID NO : 290, SEQ ID NO : 292, SEQ ID NO : 294, SEQ ID NO : 296, SEQ ID NO : 298, SEQ ID NO : 300, SEQ ID NO : 302, SEQ ID NO : 304, SEQ ID NO : 306, SEQ ID NO : 308, SEQ ID NO : 310, SEQ ID NO : 312, SEQ ID NO : 314, SEQ ID NO : 316, SEQ ID NO : 318, SEQ ID NO : 320, SEQ ID NO : 322, SEQ ID NO : 324, SEQ ID NO : 326, SEQ ID NO : 328, SEQ ID NO : 330, SEQ ID NO : 332, SEQ ID NO : 334, SEQ ID NO : 336, SEQ ID NO : 338, SEQ ID NO : 340, SEQ ID NO : 342, SEQ ID NO : 344, SEQ ID NO : 346, SEQ ID NO : 348, SEQ ID NO : 350, SEQ ID NO : 352, SEQ ID NO : 354, SEQ ID NO : 356, SEQ ID NO : 358, SEQ ID NO : 360, SEQ ID NO : 362, SEQ ID NO : 364, SEQ ID NO : 366, SEQ ID NO : 368, SEQ ID NO : 370, SEQ ID NO : 372, SEQ ID NO : 374, SEQ ID NO : 376, SEQ ID NO : 378, SEQ ID NO : 380, SEQ ID NO : 382, SEQ ID NO : 384, SEQ ID NO : 386, SEQ ID NO : 388, SEQ ID NO : 390, SEQ ID NO : 392, SEQ ID NO : 394, SEQ ID NO : 396, SEQ ID NO : 398, SEQ ID NO : 400, SEQ ID NO : 402, SEQ ID NO : 404, SEQ ID NO : 406, SEQ ID NO : 408, SEQ ID NO : 410, SEQ ID NO : 412, SEQ ID NO : 414, SEQ ID NO : 416, SEQ ID NO : 418, SEQ ID NO : 420, SEQ ID NO : 422, SEQ ID NO : 424, SEQ ID NO : 426, SEQ ID NO : 428, SEQ ID NO : 430, SEQ ID NO : 432, SEQ ID NO : 434, SEQ ID NO : 436, SEQ ID NO : 438, SEQ ID NO : 440, SEQ ID NO : 442, SEQ ID NO : 444, SEQ ID NO : 446, SEQ ID NO : 448, SEQ ID NO : 450, SEQ ID NO : 452, SEQ ID NO : 454, SEQ ID NO : 456, SEQ ID NO : 458, SEQ ID NO : 460, SEQ ID NO : 462, SEQ ID NO : 464, SEQ ID NO : 466, SEQ ID NO : 468, SEQ ID NO : 470, SEQ ID NO : 472, SEQ ID NO : 474, SEQ ID NO : 476, SEQ ID NO : 478, SEQ ID NO : 480, SEQ ID NO : 482, SEQ ID NO : 484, SEQ ID NO : 486, SEQ ID NO : 488, SEQ ID NO : 490, SEQ ID NO : 492, SEQ ID NO : 494 ou SEQ ID NO : 496.

Les séquences SEQ ID NO : 2n-1 susmentionnées correspondent aux séquences nucléotidiques SEQ ID NO : 1 à SEQ ID NO : 495.

Les séquences SEQ ID NO : 2n-1 susmentionnées codent pour les séquences protéiques susmentionnées SEQ ID NO : 2n, et correspondent aux séquences nucléotidiques suivantes : SEQ ID NO : 1 codant pour SEQ ID NO : 2, SEQ ID NO : 3 codant pour SEQ ID NO : 4, SEQ ID NO : 5 codant pour SEQ ID NO : 6, SEQ ID NO : 7 codant pour SEQ ID NO : 8, SEQ ID NO : 9 codant pour SEQ ID NO : 10, SEQ ID NO : 11 codant pour SEQ ID NO : 12, SEQ ID NO : 13 codant pour SEQ ID NO : 14, SEQ ID NO : 15 codant pour SEQ ID NO : 16, SEQ ID NO : 17 codant pour SEQ ID NO : 18, SEQ ID NO : 19 codant pour SEQ ID NO : 20, SEQ ID NO : 21 codant pour SEQ ID NO : 22, SEQ ID NO : 23 codant pour SEQ ID NO : 24, SEQ ID NO : 25 codant pour SEQ ID NO : 26, SEQ ID NO : 27 codant pour SEQ ID NO : 28, SEQ ID NO : 29 codant pour SEQ ID NO : 30, SEQ ID NO : 31 codant pour SEQ ID NO : 32, SEQ ID NO : 33 codant pour SEQ ID NO : 34, SEQ ID NO : 35 codant pour SEQ ID NO : 36, SEQ ID NO : 37 codant pour SEQ ID NO : 38, SEQ ID NO : 39 codant pour SEQ ID NO : 40, SEQ ID NO : 41 codant pour SEQ ID NO : 42, SEQ ID NO : 43 codant pour SEQ ID NO : 44, SEQ ID NO : 45 codant pour SEQ ID NO : 46, SEQ ID NO : 47 codant pour SEQ ID NO : 48, SEQ ID NO : 49 codant pour SEQ ID NO : 50, SEQ ID NO : 51 codant pour SEQ ID NO : 52, SEQ ID NO : 53 codant pour SEQ ID NO : 54, SEQ ID NO : 55 codant pour SEQ ID NO : 56, SEQ ID NO : 57 codant pour SEQ ID NO : 58, SEQ ID NO : 59 codant pour SEQ ID NO : 60, SEQ ID NO : 61 codant pour SEQ ID NO : 62, SEQ ID NO : 63 codant pour SEQ ID NO : 64, SEQ ID NO : 65 codant pour SEQ ID NO : 66, SEQ ID NO : 67 codant pour SEQ ID NO : 68, SEQ ID NO : 69 codant pour SEQ ID NO : 70, SEQ ID NO : 71 codant pour SEQ ID NO : 72, SEQ ID NO : 73 codant pour SEQ ID NO : 74, SEQ ID NO : 75 codant pour SEQ ID NO : 76, SEQ ID NO : 77 codant pour SEQ ID NO : 78, SEQ ID NO : 79 codant pour SEQ ID NO : 80, SEQ ID NO : 81 codant pour SEQ ID NO : 82, SEQ ID NO : 83 codant pour SEQ ID NO : 84, SEQ ID NO : 85 codant pour SEQ ID NO : 86, SEQ ID NO : 87 codant pour SEQ ID NO : 88, SEQ ID NO : 89 codant pour SEQ ID NO : 90, SEQ ID NO : 91 codant pour SEQ ID NO : 92, SEQ ID NO : 93 codant pour SEQ ID NO : 94, SEQ ID NO : 95 codant pour SEQ ID NO : 96, SEQ ID NO : 97 codant pour SEQ ID NO : 98, SEQ ID NO : 99 codant pour SEQ ID NO : 100, SEQ ID NO : 101 codant pour SEQ ID NO : 102, SEQ ID NO : 103 codant pour SEQ ID NO : 104, SEQ ID NO : 105 codant pour SEQ ID NO : 106, SEQ ID NO : 107 codant pour SEQ ID NO : 108, SEQ ID NO : 109 codant pour SEQ ID NO : 110, SEQ ID NO : 111 codant pour SEQ ID NO : 112, SEQ ID NO : 113 codant pour SEQ ID NO : 114, SEQ ID NO : 115 codant pour SEQ ID NO : 116, SEQ ID NO : 117 codant pour SEQ ID NO : 118, SEQ ID NO : 119 codant pour SEQ ID NO : 120, SEQ ID NO : 121 codant pour SEQ ID NO : 122, SEQ ID NO : 123 codant pour SEQ ID NO : 124, SEQ ID NO : 125 codant pour SEQ ID NO : 126, SEQ ID NO : 127 codant pour SEQ ID NO : 128, SEQ ID NO : 129 codant pour SEQ ID NO : 130, SEQ ID NO : 131 codant pour SEQ ID NO : 132, SEQ ID NO : 133 codant pour SEQ ID NO : 134, SEQ ID NO : 135 codant pour SEQ ID NO : 136, SEQ ID NO : 137 codant pour SEQ ID NO : 138, SEQ ID NO : 139 codant pour SEQ ID NO : 140, SEQ ID NO : 141 codant pour SEQ ID NO : 142, SEQ ID NO : 143 codant pour SEQ ID NO : 144, SEQ ID NO : 145 codant pour SEQ ID NO : 146, SEQ ID NO : 147 codant pour SEQ ID NO : 148, SEQ ID NO : 149 codant pour SEQ ID NO : 150, SEQ ID NO : 151 codant pour SEQ ID NO : 152, SEQ ID NO : 153 codant pour SEQ ID NO : 154, SEQ ID NO : 155 codant pour SEQ ID NO : 156, SEQ ID NO : 157 codant pour SEQ ID NO : 158, SEQ ID NO : 159 codant pour SEQ ID NO : 160, SEQ ID NO : 161 codant pour SEQ ID NO : 162, SEQ ID NO : 163 codant pour SEQ ID NO : 164, SEQ ID NO : 165 codant pour SEQ ID NO : 166, SEQ ID NO : 167 codant pour SEQ ID NO : 168, SEQ ID NO : 169 codant pour SEQ ID NO : 170, SEQ ID NO : 171 codant pour SEQ ID NO : 172, SEQ ID NO : 173 codant pour SEQ ID NO : 174, SEQ ID NO : 175 codant pour SEQ ID NO : 176, SEQ ID NO : 177 codant pour SEQ ID NO : 178, SEQ ID NO : 179 codant pour SEQ ID NO : 180, SEQ ID NO : 181 codant pour SEQ ID NO : 182, SEQ ID NO : 183 codant pour SEQ ID NO : 184, SEQ ID NO : 185 codant pour SEQ ID NO : 186, SEQ ID NO : 187 codant pour SEQ ID NO : 188, SEQ ID NO : 189 codant pour SEQ ID NO : 190, SEQ ID NO : 191 codant pour SEQ ID NO : 192, SEQ ID NO : 193 codant pour SEQ ID NO : 194, SEQ ID NO : 195 codant pour SEQ ID NO : 196, SEQ ID NO : 197 codant pour SEQ ID NO : 198, SEQ ID NO : 199 codant pour SEQ ID NO : 200, SEQ ID NO : 201 codant pour SEQ ID NO : 202, SEQ ID NO : 203 codant pour SEQ ID NO : 204, SEQ ID NO : 205 codant pour SEQ ID NO : 206, SEQ ID NO : 207 codant pour SEQ ID NO : 208, SEQ ID NO : 209 codant pour SEQ ID NO : 210, SEQ ID NO : 211 codant pour SEQ ID NO : 212, SEQ ID NO : 213 codant pour SEQ ID NO : 214, SEQ ID NO : 215 codant pour SEQ ID NO : 216, SEQ ID NO : 217 codant pour SEQ ID NO : 218, SEQ ID NO : 219 codant pour SEQ ID NO : 220, SEQ ID NO : 221 codant pour SEQ ID NO : 222, SEQ ID NO : 223 codant pour SEQ ID NO : 224, SEQ ID NO : 225 codant pour SEQ ID NO : 226, SEQ ID NO : 227 codant pour SEQ ID NO : 228, SEQ ID NO : 229 codant pour SEQ ID NO : 230, SEQ ID NO : 231 codant pour SEQ ID NO : 232, SEQ ID NO : 233 codant pour SEQ ID NO : 234, SEQ ID NO : 235 codant pour SEQ ID NO : 236, SEQ ID NO : 237 codant pour SEQ ID NO : 238, SEQ ID NO : 239 codant pour SEQ ID NO : 240, SEQ ID NO : 241 codant pour SEQ ID NO : 242, SEQ ID NO : 243 codant pour SEQ ID NO : 244, SEQ ID NO : 245 codant pour SEQ ID NO : 246, SEQ ID NO : 247 codant pour SEQ ID NO : 248, SEQ ID NO : 249 codant pour SEQ ID NO : 250, SEQ ID NO : 251 codant pour SEQ ID NO : 252, SEQ ID NO : 253 codant pour SEQ ID NO : 254, SEQ ID NO : 255 codant pour SEQ ID NO : 256, SEQ ID NO : 257 codant pour SEQ ID NO : 258, SEQ ID NO : 259 codant pour SEQ ID NO : 260, SEQ ID NO : 261 codant pour SEQ ID NO : 262, SEQ ID NO : 263 codant pour SEQ ID NO : 264, SEQ ID NO : 265 codant pour SEQ ID NO : 266, SEQ ID NO : 267 codant pour SEQ ID NO : 268, SEQ ID NO : 269 codant pour SEQ ID NO : 270, SEQ ID NO : 271 codant pour SEQ ID NO : 272, SEQ ID NO : 273 codant pour SEQ ID NO : 274, SEQ ID NO : 275 codant pour SEQ ID NO : 276, SEQ ID NO : 277 codant pour SEQ ID NO : 278, SEQ ID NO : 279 codant pour SEQ ID NO : 280, SEQ ID NO : 281 codant pour SEQ ID NO : 282, SEQ ID NO : 283 codant pour SEQ ID NO : 284, SEQ ID NO : 285 codant pour SEQ ID NO : 286, SEQ ID NO : 287 codant pour SEQ ID NO : 288, SEQ ID NO : 289 codant pour SEQ ID NO : 290, SEQ ID NO : 291 codant pour SEQ ID NO : 292, SEQ ID NO : 293 codant pour SEQ ID NO : 294, SEQ ID NO : 295 codant pour SEQ ID NO : 296, SEQ ID NO : 297 codant pour SEQ ID NO : 298, SEQ ID NO : 299 codant pour SEQ ID NO : 300, SEQ ID NO : 301 codant pour SEQ ID NO : 302, SEQ ID NO : 303 codant pour SEQ ID NO : 304, SEQ ID NO : 305 codant pour SEQ ID NO : 306, SEQ ID NO : 307 codant pour SEQ ID NO : 308, SEQ ID NO : 309 codant pour SEQ ID NO : 310, SEQ ID NO : 311 codant pour SEQ ID NO : 312, SEQ ID NO : 313 codant pour SEQ ID NO : 314, SEQ ID NO : 315 codant pour SEQ ID NO : 316, SEQ ID NO : 317 codant pour SEQ ID NO : 318, SEQ ID NO : 319 codant pour SEQ ID NO : 320, SEQ ID NO : 321 codant pour SEQ ID NO : 322, SEQ ID NO : 323 codant pour SEQ ID NO : 324, SEQ ID NO : 325 codant pour SEQ ID NO : 326, SEQ ID NO : 327 codant pour SEQ ID NO : 328, SEQ ID NO : 329 codant pour SEQ ID NO : 330, SEQ ID NO : 331 codant pour SEQ ID NO : 332, SEQ ID NO : 333 codant pour SEQ ID NO : 334, SEQ ID NO : 335 codant pour SEQ ID NO : 336, SEQ ID NO : 337 codant pour SEQ ID NO : 338, SEQ ID NO : 339 codant pour SEQ ID NO : 340, SEQ ID NO : 341 codant pour SEQ ID NO : 342, SEQ ID NO : 343 codant pour SEQ ID NO : 344, SEQ ID NO : 345 codant pour SEQ ID NO : 346, SEQ ID NO : 347 codant pour SEQ ID NO : 348, SEQ ID NO : 349 codant pour SEQ ID NO : 350, SEQ ID NO : 351 codant pour SEQ ID NO : 352, SEQ ID NO : 353 codant pour SEQ ID NO : 354, SEQ ID NO : 355 codant pour SEQ ID NO : 356, SEQ ID NO : 357 codant pour SEQ ID NO : 358, SEQ ID NO : 359 codant pour SEQ ID NO : 360, SEQ ID NO : 361 codant pour SEQ ID NO : 362, SEQ ID NO : 363 codant pour SEQ ID NO : 364, SEQ ID NO : 365 codant pour SEQ ID NO : 366, SEQ ID NO : 367 codant pour SEQ ID NO : 368, SEQ ID NO : 369 codant pour SEQ ID NO : 370, SEQ ID NO : 371 codant pour SEQ ID NO : 372, SEQ ID NO : 373 codant pour SEQ ID NO : 374, SEQ ID NO : 375 codant pour SEQ ID NO : 376, SEQ ID NO : 377 codant pour SEQ ID NO : 378, SEQ ID NO : 379 codant pour SEQ ID NO : 380, SEQ ID NO : 381 codant pour SEQ ID NO : 382, SEQ ID NO : 383 codant pour SEQ ID NO : 384, SEQ ID NO : 385 codant pour SEQ ID NO : 386, SEQ ID NO : 387 codant pour SEQ ID NO : 388, SEQ ID NO : 389 codant pour SEQ ID NO : 390, SEQ ID NO : 391 codant pour SEQ ID NO : 392, SEQ ID NO : 393 codant pour SEQ ID NO : 394, SEQ ID NO : 395 codant pour SEQ ID NO : 396, SEQ ID NO : 397 codant pour SEQ ID NO : 398, SEQ ID NO : 399 codant pour SEQ ID NO : 400, SEQ ID NO : 401 codant pour SEQ ID NO : 402, SEQ ID NO : 403 codant pour SEQ ID NO : 404, SEQ ID NO : 405 codant pour SEQ ID NO : 406, SEQ ID NO : 407 codant pour SEQ ID NO : 408, SEQ ID NO : 409 codant pour SEQ ID NO : 410, SEQ ID NO : 411 codant pour SEQ ID NO : 412, SEQ ID NO : 413 codant pour SEQ ID NO : 414, SEQ ID NO : 415 codant pour SEQ ID NO : 416, SEQ ID NO : 417 codant pour SEQ ID NO : 418, SEQ ID NO : 419 codant pour SEQ ID NO : 420, SEQ ID NO : 421 codant pour SEQ ID NO : 422, SEQ ID NO : 423 codant pour SEQ ID NO : 424, SEQ ID NO : 425 codant pour SEQ ID NO : 426, SEQ ID NO : 427 codant pour SEQ ID NO : 428, SEQ ID NO : 429 codant pour SEQ ID NO : 430, SEQ ID NO : 431 codant pour SEQ ID NO : 432, SEQ ID NO : 433 codant pour SEQ ID NO : 434, SEQ ID NO : 435 codant pour SEQ ID NO : 436, SEQ ID NO : 437 codant pour SEQ ID NO : 438, SEQ ID NO : 439 codant pour SEQ ID NO : 440, SEQ ID NO : 441 codant pour SEQ ID NO : 442, SEQ ID NO : 443 codant pour SEQ ID NO : 444, SEQ ID NO : 445 codant pour SEQ ID NO : 446, SEQ ID NO : 447 codant pour SEQ ID NO : 448, SEQ ID NO : 449 codant pour SEQ ID NO : 450, SEQ ID NO : 451 codant pour SEQ ID NO : 452, SEQ ID NO : 453 codant pour SEQ ID NO : 454, SEQ ID NO : 455 codant pour SEQ ID NO : 456, SEQ ID NO : 457 codant pour SEQ ID NO : 458, SEQ ID NO : 459 codant pour SEQ ID NO : 460, SEQ ID NO : 461 codant pour SEQ ID NO : 462, SEQ ID NO : 463 codant pour SEQ ID NO : 464, SEQ ID NO : 465 codant pour SEQ ID NO : 466, SEQ ID NO : 467 codant pour SEQ ID NO : 468, SEQ ID NO : 469 codant pour SEQ ID NO : 470, SEQ ID NO : 471 codant pour SEQ ID NO : 472, SEQ ID NO : 473 codant pour SEQ ID NO : 474, SEQ ID NO : 475 codant pour SEQ ID NO : 476, SEQ ID NO : 477 codant pour SEQ ID NO : 478, SEQ ID NO : 479 codant pour SEQ ID NO : 480, SEQ ID NO : 481 codant pour SEQ ID NO : 482, SEQ ID NO : 483 codant pour SEQ ID NO : 484, SEQ ID NO : 485 codant pour SEQ ID NO : 486, SEQ ID NO : 487 codant pour SEQ ID NO : 488, SEQ ID NO : 489 codant pour SEQ ID NO : 490, SEQ ID NO : 491 codant pour SEQ ID NO : 492, SEQ ID NO : 493 codant pour SEQ ID NO : 494 ou SEQ ID NO : 495 codant pour SEQ ID NO : 496.

La séquence SEQ ID NO : 498 correspond à la protéine humaine nétrine 4 codée par la séquence nucléotidique SEQ ID NO : 497, et la séquence SEQ ID NO : 500 correspond à la protéine humaine nétrine 4 susmentionnée sans le peptide signal, codée par la séquence nucléotidique SEQ ID NO : 499. La séquence SEQ ID NO : 498 comprend 628 acides aminés et la séquence SEQ ID NO : 500 comprend 609 acides aminés et correspond à un fragment de la séquence SEQ ID NO : 498 allant du résidu 20 au résidu 628.

La séquence SEQ ID NO : 502 correspond à la protéine humaine nétrine 1 codée par la séquence nucléotidique SEQ ID NO : 501, et la séquence SEQ ID NO : 504 correspond à la protéine humaine nétrine 1 susmentionnée sans le peptide signal, codée par la séquence nucléotidique SEQ ID NO : 503. La séquence SEQ ID NO : 502 comprend 604 acides aminés et la séquence SEQ ID NO : 504 comprend 580 acides aminés et correspond à un fragment de la séquence SEQ ID NO : 502 allant du résidu 25 au résidu 604.

La séquence SEQ ID NO : 506 correspond à la protéine humaine nétrine G1 codée par la séquence nucléotidique SEQ ID NO : 505, et la séquence SEQ ID NO : 508 correspond à la protéine humaine nétrine G1 susmentionnée sans le peptide signal, codée par la séquence nucléotidique SEQ ID NO : 507. La séquence SEQ ID NO : 506 comprend 438 acides aminés et la séquence SEQ ID NO : 508 comprend 410 acides aminés et correspond à un fragment de la séquence SEQ ID NO : 506 allant du résidu 29 au résidu 438.

La séquence SEQ ID NO : 510 correspond à la protéine humaine nétrine 3 codée par la séquence nucléotidique SEQ ID NO : 509. La séquence SEQ ID NO : 510 comprend 580 acides aminés.

La séquence SEQ ID NO : 512 correspond à la molécule RGM-A codée par la séquence nucléotidique SEQ ID NO : 511. La séquence SEQ ID NO : 512 comprend 450 acides aminés.

La séquence SEQ ID NO : 514 correspond à la molécule RGM-B codée par la séquence nucléotidique SEQ ID NO : 513, et la séquence SEQ ID NO : 518 correspond à la molécule RGM-B susmentionnée sans le peptide signal, codée par la séquence nucléotidique SEQ ID NO : 517. La séquence SEQ ID NO : 514 comprend 437 acides aminés et la séquence SEQ ID NO : 518 comprend 392 acides aminés et correspond à un fragment de la séquence SEQ ID NO : 514 allant du résidu 46 au résidu 437.

La séquence SEQ ID NO : 516 correspond à la molécule RGM-C codée par la séquence nucléotidique SEQ ID NO : 515, et la séquence SEQ ID NO : 520 correspond à la molécule RGM-C susmentionnée sans le peptide signal, codée par la séquence nucléotidique SEQ ID NO : 519. La séquence SEQ ID NO : 516 comprend 426 acides aminés et la séquence SEQ ID NO : 520 comprend 391 acides aminés et correspond à un fragment de la séquence SEQ ID NO : 516 allant du résidu 36 au résidu 426.

La séquence SEQ ID NO : 360 correspond à un fragment de type laminine (protéine de la matrice extracellulaire se liant à certaines intégrines) de la protéine humaine nétrine 4, ledit fragment étant codé par la séquence nucléotidique SEQ ID NO : 359. Ce fragment comprend 260 acides aminés et correspond au fragment de la protéine nétrine 4 allant du résidu 1 au résidu 260 de la séquence SEQ ID NO : 498.

La séquence SEQ ID NO : 362 correspond à un fragment de type EGF (domaine de répétition facteur EGF) de la protéine humaine nétrine 4, ledit fragment étant codé par la séquence nucléotidique SEQ ID NO : 361. Ce fragment comprend 255 acides aminés et correspond au fragment de la protéine nétrine 4 allant du résidu 261 au résidu 515 de la séquence SEQ ID NO : 498.

La séquence SEQ ID NO : 364 correspond au fragment de liaison à l'héparine de la protéine humaine nétrine 4, ledit fragment étant codé par la séquence nucléotidique SEQ ID NO : 363. Ce fragment comprend 113 acides aminés et correspond au fragment de la protéine nétrine 4 allant du résidu 516 au résidu 628 de la séquence SEQ ID NO : 498.

La séquence SEQ ID NO : 366 est un fragment correspondant à la protéine humaine nétrine 4 délétée de la séquence SEQ ID NO : 364, ledit fragment étant codé par la séquence nucléotidique SEQ ID NO : 365. Ce fragment comprend 515 acides aminés et correspond au fragment de la protéine nétrine 4 allant du résidu 1 au résidu 515 de la séquence SEQ ID NO : 498.

La séquence SEQ ID NO : 8 est un fragment de type laminine de la protéine humaine nétrine 4, ledit fragment étant codé par la séquence nucléotidique SEQ ID NO : 7. Ce fragment comprend 229 acides aminés et correspond au fragment de la protéine nétrine 4 allant du résidu 32 au résidu 260 de la séquence SEQ ID NO : 498.

La séquence SEQ ID NO : 2 est un fragment de type EGF de la protéine humaine nétrine 4, ledit fragment étant codé par la séquence nucléotidique SEQ ID NO : 1. Ce fragment comprend 60 acides aminés et correspond au fragment de la protéine nétrine 4 allant du résidu 261 au résidu 320 de la séquence SEQ ID NO : 498.

La séquence SEQ ID NO : 4 est un fragment de type EGF de la protéine humaine nétrine 4, ledit fragment étant codé par la séquence nucléotidique SEQ ID NO : 3. Ce fragment comprend 56 acides aminés et correspond au fragment de la protéine nétrine 4 allant du résidu 332 au résidu 387 de la séquence SEQ ID NO : 498.

La séquence SEQ ID NO : 6 est un fragment de type EGF de la protéine humaine nétrine 4, ledit fragment étant codé par la séquence nucléotidique SEQ ID NO : 5. Ce fragment comprend 52 acides aminés et correspond au fragment de la protéine nétrine 4 allant du résidu 394 au résidu 445 de la séquence SEQ ID NO : 498.

Les fragments de la protéine nétrine 4 correspondant aux séquences protéiques SEQ ID NO : 10 à SEQ ID NO : 126 et aux séquences protéiques SEQ ID NO : 368 et SEQ ID NO : 370, ainsi que les séquences nucléotidiques correspondantes SEQ ID NO : 9 à SEQ ID NO : 125 et les séquences nucléotidiques SEQ ID NO : 367 et SEQ ID NO : 369 sont regroupés dans le tableau qui suit :

| **Séquence protéique** | **Séquence nucléotidique** | **Positions du fragment par rapport** **à la séquence SEQ ID NO : 498** |
|---|---|---|
| SEQ ID NO : 10 | SEQ ID NO : 9 | 1-260 + 516-628 |
| SEQ ID NO : 12 | SEQ ID NO : 11 | 261-628 |
| SEQ ID NO : 14 | SEQ ID NO : 13 | 1-320 |
| SEQ ID NO : 16 | SEQ ID NO : 15 | 1-260 + 332-387 |
| SEQ ID NO : 18 | SEQ ID NO : 17 | 1-260 + 394-445 |
| SEQ ID NO : 20 | SEQ ID NO : 19 | 32-320 |
| SEQ ID NO : 22 | SEQ ID NO : 21 | 32-260 + 332-387 |
| SEQ ID NO : 24 | SEQ ID NO : 23 | 32-260 + 394-445 |
| SEQ ID NO : 26 | SEQ ID NO : 25 | 261-320 + 332-387 |
| SEQ ID NO : 28 | SEQ ID NO : 27 | 332-387 + 394-445 |
| SEQ ID NO : 30 | SEQ ID NO : 29 | 261-320 + 394-445 |
| SEQ ID NO : 32 | SEQ ID NO : 31 | 261-320 +332-387 + 394-445 |
| SEQ ID NO : 34 | SEQ ID NO : 33 | 1-320 + 332-387 |
| SEQ ID NO : 36 | SEQ ID NO : 35 | 1-260 + 332-387 + 394-445 |
| SEQ ID NO : 38 | SEQ ID NO : 37 | 1-320 + 394-445 |
| SEQ ID NO : 40 | SEQ ID NO : 39 | 32-320 + 332-387 |
| SEQ ID NO : 42 | SEQ ID NO : 41 | 32-260 + 332-387 + 394-445 |
| SEQ ID NO : 44 | SEQ ID NO : 43 | 32-320 + 394-445 |
| SEQ ID NO : 46 | SEQ ID NO : 45 | 1-320 +332-387 + 394-445 |
| SEQ ID NO : 48 | SEQ ID NO : 47 | 32-320 +332-387 + 394-445 |
| SEQ ID NO : 50 | SEQ ID NO : 49 | 261-320 + 516-628 |
| SEQ ID NO : 52 | SEQ ID NO : 51 | 332-387 + 516-628 |
| SEQ ID NO : 54 | SEQ ID NO : 53 | 394-445 + 516-628 |
| SEQ ID NO : 56 | SEQ ID NO : 55 | 32-260 + 516-628 |
| SEQ ID NO : 58 | SEQ ID NO : 57 | 32-320 + 516-628 |
| SEQ ID NO : 60 | SEQ ID NO : 59 | 32-260 + 332-387 + 516-628 |
| SEQ ID NO : 62 | SEQ ID NO : 61 | 32-260 + 394-445 + 516-628 |
| SEQ ID NO : 64 | SEQ ID NO : 63 | 1-320 + 516-628 |
| SEQ ID NO : 66 | SEQ ID NO : 65 | 1-260 + 332-387 + 516-628 |
| SEQ ID NO : 68 | SEQ ID NO : 67 | 1-260 + 394-445 + 516-628 |
| SEQ ID NO : 70 | SEQ ID NO : 69 | 261-320 + 332-387 + 516-628 |
| SEQ ID NO : 72 | SEQ ID NO : 71 | 261-320 + 394-445 + 516-628 |
| SEQ ID NO : 74 | SEQ ID NO : 73 | 332-387 + 394-445 + 516-628 |
| SEQ ID NO : 76 | SEQ ID NO : 75 | 261-320 + 332-387 + 394-445 + 516-628 |
| SEQ ID NO : 78 | SEQ ID NO : 77 | 1-320 + 332-387 + 516-628 |
| SEQ ID NO : 80 | SEQ ID NO : 79 | 1-260 + 332-387 + 394-445 + 516-628 |
| SEQ ID NO : 82 | SEQ ID NO : 81 | 1-320 + 394-445 + 516-628 |
| SEQ ID NO : 84 | SEQ ID NO : 83 | 32-320 + 332-387 + 516-628 |
| SEQ ID NO : 86 | SEQ ID NO : 85 | 32-260 + 332-387 + 394-445 + 516-628 |
| SEQ ID NO : 88 | SEQ ID NO : 87 | 32-320 + 394-445 + 516-628 |
| SEQ ID NO : 90 | SEQ ID NO : 89 | 1-320 + 332-387 + 394-445 + 516-628 |
| SEQ ID NO : 92 | SEQ ID NO : 91 | 32-320 + 332-387 + 394-445 + 516-628 |
| SEQ ID NO : 94 | SEQ ID NO : 93 | 20-260 |
| SEQ ID NO : 96 | SEQ ID NO : 95 | 20-516 |
| SEQ ID NO : 98 | SEQ ID NO : 97 | 20-260 + 516-628 |
| SEQ ID NO : 100 | SEQ ID NO : 99 | 20-320 |
| SEQ ID NO : 102 | SEQ ID NO : 101 | 20-260 + 332-387 |
| SEQ ID NO : 104 | SEQ ID NO : 103 | 20-260 + 394-445 |
| SEQ ID NO : 106 | SEQ ID NO : 105 | 20-320 + 332-387 |
| SEQ ID NO : 108 | SEQ ID NO : 107 | 20-260 + 332-387 + 394-445 |
| SEQ ID NO : 110 | SEQ ID NO : 109 | 20-320 + 394-445 |
| SEQ ID NO : 112 | SEQ ID NO : 111 | 20-320 +332-387 + 394-445 |
| SEQ ID NO : 114 | SEQ ID NO : 113 | 20-320 + 516-628 |
| SEQ ID NO : 116 | SEQ ID NO : 115 | 20-260 + 332-387 + 516-628 |
| SEQ ID NO : 118 | SEQ ID NO : 117 | 20-260 + 394-445 + 516-628 |
| SEQ ID NO : 120 | SEQ ID NO : 119 | 20-320 + 332-387 + 516-628 |
| SEQ ID NO : 122 | SEQ ID NO : 121 | 20-260 + 332-387 + 394-445 + 516-628 |
| SEQ ID NO : 124 | SEQ ID NO : 123 | 20-320 + 394-445 + 516-628 |
| SEQ ID NO : 126 | SEQ ID NO : 125 | 20-320 + 332-387 + 394-445 + 516-628 |
| SEQ ID NO : 368 | SEQ ID NO : 367 | 32-515 |
| SEQ ID NO : 370 | SEQ ID NO : 369 | 32-628 |

La séquence SEQ ID NO : 128 correspond à un fragment de type laminine (protéine de la matrice extracellulaire se liant à certaines intégrines) de la protéine humaine nétrine 1, ledit fragment étant codé par la séquence nucléotidique SEQ ID NO : 127. Ce fragment comprend 283 acides aminés et correspond au fragment de la protéine nétrine 1 allant du résidu 1 au résidu 283 de la séquence SEQ ID NO : 502.

La séquence SEQ ID NO : 130 correspond à un fragment de type EGF (domaine de répétition facteur EGF) de la protéine humaine nétrine 1, ledit fragment étant codé par la séquence nucléotidique SEQ ID NO : 129. Ce fragment comprend 204 acides aminés et correspond au fragment de la protéine nétrine 1 allant du résidu 284 au résidu 487 de la séquence SEQ ID NO : 502.

La séquence SEQ ID NO : 132 correspond au fragment de liaison à l'héparine de la protéine humaine nétrine 1, ledit fragment étant codé par la séquence nucléotidique SEQ ID NO : 131. Ce fragment comprend 117 acides aminés et correspond au fragment de la protéine nétrine 1 allant du résidu 488 au résidu 604 de la séquence SEQ ID NO : 502.

La séquence SEQ ID NO : 372 est un fragment correspondant à la protéine humaine nétrine 1 délétée de la séquence SEQ ID NO : 132, ledit fragment étant codé par la séquence nucléotidique SEQ ID NO : 371. Ce fragment comprend 487 acides aminés et correspond au fragment de la protéine nétrine 1 allant du résidu 1 au résidu 487 de la séquence SEQ ID NO : 502.

La séquence SEQ ID NO : 140 est un fragment de type laminine de la protéine humaine nétrine 1, ledit fragment étant codé par la séquence nucléotidique SEQ ID NO : 139. Ce fragment comprend 235 acides aminés et correspond au fragment de la protéine nétrine 1 allant du résidu 49 au résidu 283 de la séquence SEQ ID NO : 502.

La séquence SEQ ID NO : 134 est un fragment de type EGF de la protéine humaine nétrine 1, ledit fragment étant codé par la séquence nucléotidique SEQ ID NO : 133. Ce fragment comprend 48 acides aminés et correspond au fragment de la protéine nétrine 1 allant du résidu 284 au résidu 331 de la séquence SEQ ID NO : 502.

La séquence SEQ ID NO : 136 est un fragment de type EGF de la protéine humaine nétrine 1, ledit fragment étant codé par la séquence nucléotidique SEQ ID NO : 135. Ce fragment comprend 56 acides aminés et correspond au fragment de la protéine nétrine 1 allant du résidu 341 au résidu 396 de la séquence SEQ ID NO : 502.

La séquence SEQ ID NO : 138 est un fragment de type EGF de la protéine humaine nétrine 1, ledit fragment étant codé par la séquence nucléotidique SEQ ID NO : 137. Ce fragment comprend 48 acides aminés et correspond au fragment de la protéine nétrine 1 allant du résidu 403 au résidu 450 de la séquence SEQ ID NO : 502.

Les fragments de la protéine nétrine 1 correspondant aux séquences protéiques SEQ ID NO: 142 à SEQ ID NO : 262, ainsi que les séquences nucléotidiques correspondantes SEQ ID NO : 141 à SEQ ID NO : 261 sont regroupés dans le tableau qui suit :

| **Séquence protéique** | **Séquence nucléotidique** | **Positions du fragment par rapport** **à la séquence SEQ ID NO : 502** |
|---|---|---|
| SEQ ID NO : 142 | SEQ ID NO : 141 | 1-283 + 488-604 |
| SEQ ID NO : 144 | SEQ ID NO : 143 | 284-604 |
| SEQ ID NO : 146 | SEQ ID NO : 145 | 49-487 |
| SEQ ID NO : 148 | SEQ ID NO : 147 | 49-604 |
| SEQ ID NO : 150 | SEQ ID NO : 149 | 1-331 |
| SEQ ID NO : 152 | SEQ ID NO : 151 | 1-283 + 341-396 |
| SEQ ID NO : 154 | SEQ ID NO : 153 | 1-283 + 403-450 |
| SEQ ID NO : 156 | SEQ ID NO : 155 | 49-331 |
| SEQ ID NO : 158 | SEQ ID NO : 157 | 49-283 + 341-396 |
| SEQ ID NO : 160 | SEQ ID NO : 159 | 49-283 + 403-450 |
| SEQ ID NO : 162 | SEQ ID NO : 161 | 284-331 + 341-396 |
| SEQ ID NO : 164 | SEQ ID NO : 163 | 341-396 + 403-450 |
| SEQ ID NO : 166 | SEQ ID NO : 165 | 284-331 + 403-450 |
| SEQ ID NO : 168 | SEQ ID NO : 167 | 284-331 + 341-396 + 403-450 |
| SEQ ID NO : 170 | SEQ ID NO : 169 | 1-331 + 341-396 |
| SEQ ID NO : 172 | SEQ ID NO : 171 | 1-283 + 341-396 + 403-450 |
| SEQ ID NO : 174 | SEQ ID NO : 173 | 1-331 + 403-450 |
| SEQ ID NO : 176 | SEQ ID NO : 175 | 49-331 + 341-396 |
| SEQ ID NO : 178 | SEQ ID NO : 177 | 49-283 + 341-396 + 403-450 |
| SEQ ID NO : 180 | SEQ ID NO : 179 | 49-331 + 403-450 |
| SEQ ID NO : 182 | SEQ ID NO : 181 | 1-331 + 341-396 + 403-450 |
| SEQ ID NO : 184 | SEQ ID NO : 183 | 49-331 + 341-396 + 403-450 |
| SEQ ID NO : 186 | SEQ ID NO : 185 | 284-331 + 488-604 |
| SEQ ID NO : 188 | SEQ ID NO : 187 | 341-396 + 488-604 |
| SEQ ID NO : 190 | SEQ ID NO : 189 | 403-450 + 488-604 |
| SEQ ID NO : 192 | SEQ ID NO : 191 | 49-283 + 488-604 |
| SEQ ID NO : 194 | SEQ ID NO : 193 | 49-331 + 488-604 |
| SEQ ID NO : 196 | SEQ ID NO : 195 | 49-283 + 341-396 + 488-604 |
| SEQ ID NO : 198 | SEQ ID NO : 197 | 49-283 + 403-450 + 488-604 |
| SEQ ID NO : 200 | SEQ ID NO : 199 | 1-331 + 488-604 |
| SEQ ID NO : 202 | SEQ ID NO : 201 | 1-283 + 341-396 + 488-604 |
| SEQ ID NO : 204 | SEQ ID NO : 203 | 1-283 + 403-450 + 488-604 |
| SEQ ID NO : 206 | SEQ ID NO : 205 | 284-331 + 341-396 + 488-604 |
| SEQ ID NO : 208 | SEQ ID NO : 207 | 284-331 + 403-450 + 488-604 |
| SEQ ID NO : 210 | SEQ ID NO : 209 | 341-396 + 403-450 + 488-604 |
| SEQ ID NO : 212 | SEQ ID NO : 211 | 284-331 + 341-396 + 403-450 + 488-604 |
| SEQ ID NO : 214 | SEQ ID NO : 213 | 1-331 + 341-396 + 488-604 |
| SEQ ID NO : 216 | SEQ ID NO : 215 | 1-283 + 341-396 + 403-450 + 488-604 |
| SEQ ID NO : 218 | SEQ ID NO : 217 | 1-331 + 403-450 + 488-604 |
| SEQ ID NO : 220 | SEQ ID NO : 219 | 49-331 + 341-396 + 488-604 |
| SEQ ID NO : 222 | SEQ ID NO : 221 | 49-283 + 341-396 + 403-450 + 488-604 |
| SEQ ID NO : 224 | SEQ ID NO : 223 | 49-331 + 403-450 + 488-604 |
| SEQ ID NO : 226 | SEQ ID NO : 225 | 1-331 + 341-396 + 403-450 + 488-604 |
| SEQ ID NO : 228 | SEQ ID NO : 227 | 49-331 + 341-396 + 403-450 + 488-604 |
| SEQ ID NO : 230 | SEQ ID NO : 229 | 25-283 |
| SEQ ID NO : 232 | SEQ ID NO : 231 | 25-487 |
| SEQ ID NO : 234 | SEQ ID NO : 233 | 25-283 + 488-604 |
| SEQ ID NO : 236 | SEQ ID NO : 235 | 25-331 |
| SEQ ID NO : 238 | SEQ ID NO : 237 | 25-283 + 341-396 |
| SEQ ID NO : 240 | SEQ ID NO : 239 | 25-283 + 403-450 |
| SEQ ID NO : 242 | SEQ ID NO : 241 | 25-331 + 341-396 |
| SEQ ID NO : 244 | SEQ ID NO : 243 | 25-283 + 341-396 + 403-450 |
| SEQ ID NO : 246 | SEQ ID NO : 245 | 25-331 + 403-450 |
| SEQ ID NO : 248 | SEQ ID NO : 247 | 25-331 + 341-396 + 403-450 |
| SEQ ID NO : 250 | SEQ ID NO : 249 | 25-331 + 488-604 |
| SEQ ID NO : 252 | SEQ ID NO : 251 | 25-283 + 341-396 + 488-604 |
| SEQ ID NO : 254 | SEQ ID NO : 253 | 25-283 + 403-450 + 488-604 |
| SEQ ID NO : 256 | SEQ ID NO : 255 | 25-331 + 341-396 + 488-604 |
| SEQ ID NO : 258 | SEQ ID NO : 257 | 25-283 + 341-396 + 403-450 + 488-604 |
| SEQ ID NO : 260 | SEQ ID NO : 259 | 25-331 + 403-450 + 488-604 |
| SEQ ID NO : 262 | SEQ ID NO : 261 | 25-331 + 341-396 + 403-450 + 488-604 |

La séquence SEQ ID NO : 264 correspond à un fragment de type laminine (protéine de la matrice extracellulaire se liant à certaines intégrines) de la protéine humaine nétrine G1, ledit fragment étant codé par la séquence nucléotidique SEQ ID NO : 263. Ce fragment comprend 295 acides aminés et correspond au fragment de la protéine nétrine G1 allant du résidu 1 au résidu 295 de la séquence SEQ ID NO : 506.

La séquence SEQ ID NO : 266 correspond à un fragment de type EGF (domaine de répétition facteur EGF) de la protéine humaine nétrine G1, ledit fragment étant codé par la séquence nucléotidique SEQ ID NO : 265. Ce fragment comprend 143 acides aminés et correspond au fragment de la protéine nétrine G1 allant du résidu 296 au résidu 438 de la séquence SEQ ID NO : 506.

La séquence SEQ ID NO : 268 est un fragment de type laminine de la protéine humaine nétrine G1, ledit fragment étant codé par la séquence nucléotidique SEQ ID NO : 267. Ce fragment comprend 225 acides aminés et correspond au fragment de la protéine nétrine G1 allant du résidu 71 au résidu 295 de la séquence SEQ ID NO : 506.

La séquence SEQ ID NO : 270 est un fragment de type EGF de la protéine humaine nétrine G1, ledit fragment étant codé par la séquence nucléotidique SEQ ID NO : 269. Ce fragment comprend 47 acides aminés et correspond au fragment de la protéine nétrine G1 allant du résidu 296 au résidu 342 de la séquence SEQ ID NO : 506.

La séquence SEQ ID NO : 272 est un fragment de type EGF de la protéine humaine nétrine G1, ledit fragment étant codé par la séquence nucléotidique SEQ ID NO : 271. Ce fragment comprend 37 acides aminés et correspond au fragment de la protéine nétrine G1 allant du résidu 373 au résidu 409 de la séquence SEQ ID NO : 506.

Les fragments de la protéine nétrine G1 correspondant aux séquences protéiques SEQ ID NO : 274 à SEQ ID NO : 296, ainsi que les séquences nucléotidiques correspondantes SEQ ID NO : 273 à SEQ ID NO : 295, sont regroupés dans le tableau qui suit :

| **Séquence protéique** | **Séquence nucléotidique** | **Positions du fragment par rapport** **à la séquence SEQ ID NO : 506** |
|---|---|---|
| SEQ ID NO : 274 | SEQ ID NO : 273 | 1-342 |
| SEQ ID NO : 276 | SEQ ID NO : 275 | 1-295 + 373-409 |
| SEQ ID NO : 278 | SEQ ID NO : 277 | 1-342 + 373-409 |
| SEQ ID NO : 280 | SEQ ID NO : 279 | 71-438 |
| SEQ ID NO : 282 | SEQ ID NO : 281 | 71-342 |
| SEQ ID NO : 284 | SEQ ID NO : 283 | 71-295 + 373-409 |
| SEQ ID NO : 286 | SEQ ID NO : 285 | 296-342 + 373-409 |
| SEQ ID NO : 288 | SEQ ID NO : 287 | 71-342 + 373-409 |
| SEQ ID NO : 290 | SEQ ID NO : 289 | 29-295 |
| SEQ ID NO : 292 | SEQ ID NO : 291 | 29-342 |
| SEQ ID NO : 294 | SEQ ID NO : 293 | 29-295 + 373-409 |
| SEQ ID NO : 296 | SEQ ID NO : 295 | 29-342 + 373-409 |

La séquence SEQ ID NO : 298 correspond à un fragment de type laminine (protéine de la matrice extracellulaire se liant à certaines intégrines) de la protéine humaine nétrine 3, ledit fragment étant codé par la séquence nucléotidique SEQ ID NO : 297. Ce fragment comprend 253 acides aminés et correspond au fragment de la protéine nétrine 3 allant du résidu 1 au résidu 253 de la séquence SEQ ID NO : 510.

La séquence SEQ ID NO : 300 est un fragment de type laminine de la protéine humaine nétrine 3, ledit fragment étant codé par la séquence nucléotidique SEQ ID NO : 299. Ce fragment comprend 220 acides aminés et correspond au fragment de la protéine nétrine 3 allant du résidu 34 au résidu 253 de la séquence SEQ ID NO : 510.

La séquence SEQ ID NO : 302 correspond à un fragment de type EGF (domaine de répétition facteur EGF) de la protéine humaine nétrine 3, ledit fragment étant codé par la séquence nucléotidique SEQ ID NO : 301. Ce fragment comprend 180 acides aminés et correspond au fragment de la protéine nétrine 3 allant du résidu 254 au résidu 433 de la séquence SEQ ID NO : 510.

La séquence SEQ ID NO : 304 est un fragment de type EGF de la protéine humaine nétrine 3, ledit fragment étant codé par la séquence nucléotidique SEQ ID NO : 303. Ce fragment comprend 46 acides aminés et correspond au fragment de la protéine nétrine 3 allant du résidu 254 au résidu 299 de la séquence SEQ ID NO : 510.

La séquence SEQ ID NO : 306 est un fragment de type EGF de la protéine humaine nétrine 3, ledit fragment étant codé par la séquence nucléotidique SEQ ID NO : 305. Ce fragment comprend 50 acides aminés et correspond au fragment de la protéine nétrine 3 allant du résidu 373 au résidu 422 de la séquence SEQ ID NO : 510.

La séquence SEQ ID NO : 308 correspond au fragment de liaison à l'héparine de la protéine humaine nétrine 3, ledit fragment étant codé par la séquence nucléotidique SEQ ID NO : 307. Ce fragment comprend 148 acides aminés et correspond au fragment de la protéine nétrine 3 allant du résidu 433 au résidu 580 de la séquence SEQ ID NO : 510.

Les fragments de la protéine nétrine 3 correspondant aux séquences protéiques SEQ ID NO : 310 à SEQ ID NO : 352, ainsi que les séquences nucléotidiques correspondantes SEQ ID NO : 309 à SEQ ID NO : 351 sont regroupés dans le tableau qui suit :

| **Séquence protéique** | **Séquence nucléotidique** | **Positions du fragment par rapport** **à la séquence SEQ ID NO : 510** |
|---|---|---|
| SEQ ID NO : 310 | SEQ ID NO : 309 | 1-422 |
| SEQ ID NO : 312 | SEQ ID NO : 311 | 254-433 |
| SEQ ID NO : 314 | SEQ ID NO : 313 | 1-253 + 433-580 |
| SEQ ID NO : 316 | SEQ ID NO : 315 | 1-299 |
| SEQ ID NO : 318 | SEQ ID NO : 317 | 1-253 + 373-422 |
| SEQ ID NO : 320 | SEQ ID NO : 319 | 1-299 + 373-422 |
| SEQ ID NO : 322 | SEQ ID NO : 321 | 254-299 + 433-580 |
| SEQ ID NO : 324 | SEQ ID NO : 323 | 373-422 + 433-580 |
| SEQ ID NO : 326 | SEQ ID NO : 325 | 254-299 + 373-422 |
| SEQ ID NO : 328 | SEQ ID NO : 327 | 254-299 + 373-422 + 433-580 |
| SEQ ID NO : 330 | SEQ ID NO : 329 | 1-299 + 433-580 |
| SEQ ID NO : 332 | SEQ ID NO : 331 | 1-253 + 373-422 + 433-580 |
| SEQ ID NO : 334 | SEQ ID NO : 333 | 1-299 + 373-422 + 433-580 |
| SEQ ID NO : 336 | SEQ ID NO : 335 | 34-433 |
| SEQ ID NO : 338 | SEQ ID NO : 337 | 34-253 + 433-580 |
| SEQ ID NO : 340 | SEQ ID NO : 339 | 34-299 |
| SEQ ID NO : 342 | SEQ ID NO : 341 | 34-253 + 373-422 |
| SEQ ID NO : 344 | SEQ ID NO : 343 | 34-580 |
| SEQ ID NO : 346 | SEQ ID NO : 345 | 34-299 + 373-422 |
| SEQ ID NO : 348 | SEQ ID NO : 347 | 34-299 + 433-580 |
| SEQ ID NO : 350 | SEQ ID NO : 349 | 34-253 + 373-422 + 433-580 |
| SEQ ID NO : 352 | SEQ ID NO : 351 | 34-299 + 373-422 + 433-580 |

La séquence SEQ ID NO : 354 correspond à un fragment de la protéine RGM-A, ledit fragment étant codé par la séquence nucléotidique SEQ ID NO : 353. Ce fragment comprend 111 acides aminés et correspond au fragment de la protéine RGM-A allant du résidu 180 au résidu 290 de la séquence SEQ ID NO : 512.

La séquence SEQ ID NO : 356 correspond à un fragment de la protéine RGM-B, ledit fragment étant codé par la séquence nucléotidique SEQ ID NO : 355. Ce fragment comprend 111 acides aminés et correspond au fragment de la protéine RGM-B allant du résidu 180 au résidu 290 de la séquence SEQ ID NO : 514.

La séquence SEQ ID NO : 358 correspond à un fragment de la protéine RGM-C, ledit fragment étant codé par la séquence nucléotidique SEQ ID NO : 357. Ce fragment comprend 111 acides aminés et correspond au fragment de la protéine RGM-C allant du résidu 180 au résidu 290 de la séquence SEQ ID NO : 516.

La présente invention concerne également un produit de combinaison comprenant au moins un ligand de la néogénine, ou le cas échéant d'une séquence nucléotidique codant pour un ligand de la néogénine, notamment choisi parmi :
- l'une des protéines suivantes : la nétrine 1 représentée par SEQ ID NO : 502 ou SEQ ID NO : 504, la nétrine G1 représentée par SEQ ID NO : 506 ou SEQ ID NO : 508, la nétrine 3 représentée par SEQ ID NO : 510, la nétrine 4 représentée par SEQ ID NO : 498 ou SEQ ID NO : 500, l'une des molécules RGM représentées par SEQ ID NO : 512, SEQ ID NO : 514, SEQ ID NO : 516, SEQ ID NO : 518 ou SEQ ID NO : 520, ou la nétrine 4 mutée représentée par SEQ ID NO : 522 ou SEQ ID NO : 524,
- ou un fragment de l'une des ces protéines, notamment représenté par l'une des séquences SEQ ID NO : 2n, n variant de 1 à 248,
- ou une séquence nucléotidique codant pour l'une des protéines susmentionnées, notamment représentée par l'une des séquences nucléotidiques suivantes : SEQ ID NO : 501, SEQ ID NO : 503, SEQ ID NO : 505, SEQ ID NO : 507, SEQ ID NO : 509, SEQ ID NO : 497, SEQ ID NO : 499, SEQ ID NO : 511, SEQ ID NO : 513, SEQ ID NO : 515, SEQ ID NO : 517, SEQ ID NO : 519, SEQ ID NO : 521, SEQ ID NO : 523, ou l'une des séquences SEQ ID NO : 2n-1, n variant de 1 à 248,
- ou un anticorps anti-idiotypique de l'une des protéines telles que définies ci-dessus,
et au moins un agent de chimiothérapie, notamment choisi parmi : la doxorubicine, le méthotrexate, la vinblastine, la vincristine, la cladribine, le fluorouracile, la cytarabine, les anthracyclines, le cisplatine, le cyclophosphamide, la fludarabine, la gemcitabine, les inhibiteurs de l'aromatase, l'irinotecan, la navelbine, l'oxaliplatine, le taxofène, le taxol ou le taxotère,
pour une utilisation simultanée, séparée ou étalée dans le temps destiné au traitement du cancer.

Dans le cadre de la présente invention, les doses de l'agent anti-angiogénique sont comprises d'environ 10 à environ 20 000 µg/kg de poids corporel. La périodicité des injections est notamment déterminée par analogie à la posologie du traitement anti-angiogénique existant actuellement sur le marché sous le nom d'Avastin.

La présente invention concerne de nouveaux fragments de nétrine 4.

En particulier, la présente invention concerne de nouveaux fragments de la protéine nétrine 4, caractérisés en ce qu'ils comprennent ou sont constitués par l'une des séquences suivantes :
* une séquence SEQ ID NO : 2p, p variant de 1 à 63, ou
* toute séquence dérivée de l'une des séquences susmentionnées, notamment par substitution, suppression ou addition d'un ou plusieurs acides aminés, sous réserve que cette séquence dérivée présente une activité anti-angiogénique, ou
* toute séquence homologue de l'une des séquences susmentionnées, ayant de préférence une identité d'au moins environ 50% avec la région comprise entre les acides aminés en position 261 et 515 de SEQ ID NO : 498, sous réserve que cette séquence homologue présente une activité anti-angiogénique.

Les séquences SEQ ID NO : 2p susmentionnées correspondent aux séquences protéiques SEQ ID NO : 2 à SEQ ID NO : 126, à savoir les séquences protéiques suivantes : SEQ ID NO : 2, SEQ ID NO : 4, SEQ ID NO : 6, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22, SEQ ID NO : 24, SEQ ID NO : 26, SEQ ID NO : 28, SEQ ID NO : 30, SEQ ID NO : 32, SEQ ID NO : 34, SEQ ID NO : 36, SEQ ID NO : 38, SEQ ID NO : 40, SEQ ID NO : 42, SEQ ID NO : 44, SEQ ID NO : 46, SEQ ID NO : 48, SEQ ID NO : 50, SEQ ID NO : 52, SEQ ID NO : 54, SEQ ID NO : 56, SEQ ID NO : 58, SEQ ID NO : 60, SEQ ID NO : 62, SEQ ID NO : 64, SEQ ID NO : 66, SEQ ID NO : 68, SEQ ID NO : 70, SEQ ID NO : 72, SEQ ID NO : 74, SEQ ID NO : 76, SEQ ID NO : 78, SEQ ID NO : 80, SEQ ID NO : 82, SEQ ID NO : 84, SEQ ID NO : 86, SEQ ID NO : 88, SEQ ID NO : 90, SEQ ID NO : 92, SEQ ID NO : 94, SEQ ID NO : 96, SEQ ID NO : 98, SEQ ID NO : 100, SEQ ID NO : 102, SEQ ID NO : 104, SEQ ID NO : 106, SEQ ID NO : 108, SEQ ID NO : 110, SEQ ID NO : 112, SEQ ID NO : 114, SEQ ID NO : 116, SEQ ID NO : 118, SEQ ID NO : 120, SEQ ID NO : 122, SEQ ID NO : 124 ou SEQ ID NO : 126, telles que définies précédemment.

Les fragments SEQ ID NO : 2p sont de nouveaux fragments de la nétrine 4, présentant une activité d'inhibition de l'angiogenèse.

Les séquences SEQ ID NO : 2p-1 susmentionnées codent pour les séquences protéiques susmentionnées SEQ ID N0:2p, et correspondent aux séquences nucléotidiques suivantes : SEQ ID NO : 1 codant pour SEQ ID NO : 2, SEQ ID NO : 3 codant pour SEQ ID NO : 4, SEQ ID NO : 5 codant pour SEQ ID NO : 6, SEQ ID NO : 7 codant pour SEQ ID NO : 8, SEQ ID NO : 9 codant pour SEQ ID NO : 10, SEQ ID NO : 11 codant pour SEQ ID NO : 12, SEQ ID NO : 13 codant pour SEQ ID NO : 14, SEQ ID NO : 15 codant pour SEQ ID NO : 16, SEQ ID NO : 17 codant pour SEQ ID NO : 18, SEQ ID NO : 19 codant pour SEQ ID NO : 20, SEQ ID NO : 21 codant pour SEQ ID NO : 22, SEQ ID NO : 23 codant pour SEQ ID NO : 24, SEQ ID NO : 25 codant pour SEQ ID NO : 26, SEQ ID NO : 27 codant pour SEQ ID NO : 28, SEQ ID NO : 29 codant pour SEQ ID NO : 30, SEQ ID NO : 31 codant pour SEQ ID NO : 32, SEQ ID NO : 33 codant pour SEQ ID NO : 34, SEQ ID NO : 35 codant pour SEQ ID NO : 36, SEQ ID NO : 37 codant pour SEQ ID NO : 38, SEQ ID NO : 39 codant pour SEQ ID NO : 40, SEQ ID NO : 41 codant pour SEQ ID NO : 42, SEQ ID NO : 43 codant pour SEQ ID NO : 44, SEQ ID NO : 45 codant pour SEQ ID NO : 46, SEQ ID NO : 47 codant pour SEQ ID NO : 48, SEQ ID NO : 49 codant pour SEQ ID NO : 50, SEQ ID NO : 51 codant pour SEQ ID NO : 52, SEQ ID NO : 53 codant pour SEQ ID NO : 54, SEQ ID NO : 55 codant pour SEQ ID NO : 56, SEQ ID NO : 57 codant pour SEQ ID NO : 58, SEQ ID NO : 59 codant pour SEQ ID NO : 60, SEQ ID NO : 61 codant pour SEQ ID NO : 62, SEQ ID NO : 63 codant pour SEQ ID NO : 64, SEQ ID NO : 65 codant pour SEQ ID NO : 66, SEQ ID NO : 67 codant pour SEQ ID NO : 68, SEQ ID NO : 69 codant pour SEQ ID NO : 70, SEQ ID NO : 71 codant pour SEQ ID NO : 72, SEQ ID NO : 73 codant pour SEQ ID NO : 74, SEQ ID NO : 75 codant pour SEQ ID NO : 76, SEQ ID NO : 77 codant pour SEQ ID NO : 78, SEQ ID NO : 79 codant pour SEQ ID NO : 80, SEQ ID NO : 81 codant pour SEQ ID NO : 82, SEQ ID NO : 83 codant pour SEQ ID NO : 84, SEQ ID NO : 85 codant pour SEQ ID NO : 86, SEQ ID NO : 87 codant pour SEQ ID NO : 88, SEQ ID NO : 89 codant pour SEQ ID NO : 90, SEQ ID NO : 91 codant pour SEQ ID NO : 92, SEQ ID NO : 93 codant pour SEQ ID NO : 94, SEQ ID NO : 95 codant pour SEQ ID NO : 96, SEQ ID NO : 97 codant pour SEQ ID NO : 98, SEQ ID NO : 99 codant pour SEQ ID NO : 100, SEQ ID NO : 101 codant pour SEQ ID NO : 102, SEQ ID NO : 103 codant pour SEQ ID NO : 104, SEQ ID NO : 105 codant pour SEQ ID NO : 106, SEQ ID NO : 107 codant pour SEQ ID NO : 108, SEQ ID NO : 109 codant pour SEQ ID NO : 110, SEQ ID NO : 111 codant pour SEQ ID NO : 112, SEQ ID NO : 113 codant pour SEQ ID NO : 114, SEQ ID NO : 115 codant pour SEQ ID NO : 116, SEQ ID NO : 117 codant pour SEQ ID NO : 118, SEQ ID NO : 119 codant pour SEQ ID NO : 120, SEQ ID NO : 121 codant pour SEQ ID NO : 122, SEQ ID NO : 123 codant pour SEQ ID NO : 124 ou SEQ ID NO : 125 codant pour SEQ ID NO : 126.

La présente invention concerne également une séquence nucléotidique codant pour une protéine telle que définie ci-dessus.

Selon un mode de réalisation avantageux, la séquence nucléotidique selon l'invention est caractérisée en ce qu'elle comprend ou est constituée par :
- l'une des séquences nucléotidiques SEQ ID NO : 2p-1 codant pour SEQ ID NO : 2p, p variant de 1 à 63,
- ou toute séquence nucléotidique dérivée, par dégénérescence du code génétique, de l'une des séquences nucléotidiques susmentionnées, et codant pour une protéine représentée par SEQ ID NO : 2p, p variant de 1 à 63,
- ou toute séquence nucléotidique dérivée, notamment par substitution, suppression ou addition d'un ou plusieurs nucléotides, de l'une des séquences nucléotidiques susmentionnées codant pour une protéine dérivée de l'une des séquences SEQ ID NO : 2p, p variant de 1 à 63, telle que définie ci-dessus,
- ou toute séquence nucléotidique homologue de l'une des séquences nucléotidiques susmentionnées, ayant de préférence une identité d'au moins environ 50%, avec l'une des séquences SEQ ID NO : 2p-1 codant pour une protéine homologue de SEQ ID NO : 2p, telle que définie ci-dessus, p variant de 1 à 63,
- ou toute séquence nucléotidique complémentaire des séquences susmentionnées,
- ou toute séquence nucléotidique susceptible d'hybrider dans des conditions stringentes avec la séquence complémentaire d'une des séquences susmentionnées.

L'expression "hybrider dans des conditions stringentes" correspond notamment à un tampon d'hybridation tel que 0,5X SSC, à une température d'hybridation de 60°C, ainsi qu'à un milieu de lavage tel que 0,1X SSC additionné de 1% de SDS et à une température de lavage de 50°C.

La présente invention concerne également un vecteur recombinant, notamment plasmide, cosmide, phage ou ADN de virus, contenant une séquence nucléotidique telle que définie ci-dessus, notamment SEQ ID NO : 2p- 1, p variant de 1 à 63.

Un vecteur avantageux selon l'invention contient les éléments nécessaires à l'expression dans une cellule hôte des polypeptides codés par les acides nucléiques susmentionnés, notamment SEQ ID NO : 2p-1, p variant de 1 à 63, insérés dans ledit vecteur.

La présente invention concerne une cellule hôte, choisie notamment parmi les bactéries, les virus, les levures, les champignons, les plantes ou les cellules de mammifères, ladite cellule hôte étant transformée, notamment à l'aide d'un vecteur recombinant telle que défini ci-dessus.

La présente invention concerne également un anticorps caractérisé en ce qu'il est dirigé de manière spécifique contre une protéine telle que mentionnée ci-dessus, à savoir un fragment de la nétrine 4, notamment représenté par SEQ ID NO : 2p, p variant de 1 à 63.

La présente invention concerne également une composition pharmaceutique, comprenant à titre de substance active,
- une protéine telle que définie ci-dessus, correspondant aux fragments de la nétrine 4, et de préférence l'une des séquences SEQ ID NO : 2p, p variant de 1 à 63, ou
- une séquence nucléotidique telle que définie ci-dessus, notamment l'une des séquences SEQ ID NO : 2p-1, p variant de 1 à 63, ou
- un anticorps tel que défini ci-dessus, notamment dirigé contre l'une des séquences SEQ ID NO : 2p, p variant de 1 à 63.

La présente invention concerne également l'utilisation :
- d'une protéine telle que définie ci-dessus, correspondant aux fragments de la nétrine 4, et de préférence l'une des séquences SEQ ID NO : 2p, p variant de 1 à 63, ou
- d'une séquence nucléotidique telle que définie ci-dessus, notamment l'une des séquences SEQ ID NO : 2p-1, p variant de 1 à 63, ou
- d'un anticorps tel que défini ci-dessus, notamment dirigé contre l'une des séquences SEQ ID NO : 2p, p variant de 1 à 63.
pour la préparation d'un médicament destiné au traitement de pathologies nécessitant l'inhibition de la prolifération endothéliale, notamment dans le cadre des pathologies suivantes : les cancers et les leucémies, la dégénérescence maculaire liée à l'âge, les rétinopathies diabétiques, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

La présente invention concerne l'utilisation de RGM et de nouveaux fragments de RGM

En particulier, la présente invention concerne l'utilisation :
- d'une protéine caractérisée en ce qu'elle comprend ou est constituée par :
   * la séquence SEQ ID NO : 512, SEQ ID NO : 514, SEQ ID NO : 516, SEQ ID NO : 518 ou SEQ ID NO : 520,
   * un fragment de cette protéine, sous réserve que ce fragment présente une activité anti-angiogénique, ledit fragment comprenant notamment environ 40 à environ 150 acides aminés, et étant notamment représenté par l'une des séquences SEQ ID NO : 354, SEQ ID NO : 356 ou SEQ ID NO : 358,
   toute séquence dérivée de la séquence SEQ ID NO : 512, SEQ ID NO : 514, SEQ ID NO : 516, SEQ ID NO : 518 ou SEQ ID NO : 520, ou d'un fragment défini ci-dessus, notamment par substitution, suppression ou addition d'un ou plusieurs acides aminés, sous réserve que cette séquence dérivée présente une activité anti-angiogénique, ou
   * toute séquence homologue de la séquence SEQ ID NO : 512, SEQ ID NO : 514, SEQ ID NO : 516, SEQ ID NO : 518 ou SEQ ID NO : 520, ou d'un fragment défini ci-dessus, ayant de préférence une identité d'au moins environ 50% avec la région comprise entre les acides aminés en position 180 et 290 de la séquence SEQ ID NO : 512, SEQ ID NO : 514, SEQ ID NO : 516, SEQ ID NO : 518 ou SEQ ID NO : 520, sous réserve que cette séquence homologue présente une activité anti-angiogénique,
- ou d'une séquence nucléotidique caractérisée en ce qu'elle comprend ou est constituée par une séquence nucléotidique codant :
   * soit pour la protéine telle que définie ci-dessus,
   * soit pour un fragment de la protéine telle que définie ci-dessus,
   * soit pour une séquence dérivée de la protéine telle que définie ci-dessus,
   * soit pour une séquence homologue de la protéine telle que définie ci-dessus,
   ladite séquence nucléotidique correspondant notamment à la séquence nucléotidique SEQ ID NO : 511 codant pour SEQ ID NO : 512, ou à la séquence nucléotidique SEQ ID NO : 513 codant pour SEQ ID NO : 514, ou à la séquence nucléotidique SEQ ID NO : 515 codant pour SEQ ID NO : 516, ou à la séquence nucléotidique SEQ ID NO : 517 codant pour SEQ ID NO : 518, ou à la séquence nucléotidique SEQ ID NO : 519 codant pour SEQ ID NO : 520, ou à la séquence nucléotidique SEQ ID NO : 353 codant pour SEQ ID NO : 354, ou à la séquence nucléotidique SEQ ID NO : 355 codant pour SEQ ID NO : 356, ou à la séquence nucléotidique SEQ ID NO : 357 codant pour SEQ ID NO : 358,
- ou d'un anticorps anti-idiotypique de la protéine telle que définie ci-dessus,
pour la préparation d'un médicament destiné à la prévention ou au traitement des pathologies non tumorales nécessitant l'inhibition de la prolifération endothéliale, notamment dans le cadre des pathologies suivantes : la dégénérescence maculaire liée à l'âge, les rétinopathies diabétiques, la polyarthrite rhumatoïde, les angiomes, ou dans le cadre du traitement de l'obésité ou de la néovascularisation rétinienne.

La présente invention concerne également des fragments de RGM, caractérisés en ce qu'ils comprennent ou sont constitués par l'une des séquences suivantes :
* une séquence SEQ ID NO : 354, SEQ ID NO : 356 ou SEQ ID NO : 358, ou
* toute séquence dérivée de l'une des séquences susmentionnées, notamment par substitution, suppression ou addition d'un ou plusieurs acides aminés, sous réserve que cette séquence dérivée présente une activité anti-angiogénique, ou
* toute séquence homologue de l'une des séquences susmentionnées, ayant de préférence une identité d'au moins environ 50% avec la région comprise entre les acides aminés en position 180 et 290 de SEQ ID NO : 512, sous réserve que cette séquence homologue présente une activité anti-angiogénique.

La présente invention concerne également une séquence nucléotidique codant pour l'un des fragments de RGM tels que définis ci-dessus.

Une séquence nucléotidique avantageuse selon l'invention est caractérisée en ce qu'elle comprend ou est constituée par :
- la séquence nucléotidique SEQ ID NO : 353, SEQ ID NO : 355 ou SEQ ID NO : 357,
- ou toute séquence nucléotidique dérivée, par dégénérescence du code génétique, de l'une des séquences nucléotidiques susmentionnées, et codant pour une protéine représentée par SEQ ID NO : 354, SEQ ID NO : 356 ou SEQ ID NO : 358,
- ou toute séquence nucléotidique dérivée, notamment par substitution, suppression ou addition d'un ou plusieurs nucléotides, de l'une des séquences nucléotidiques susmentionnées codant pour une protéine dérivée de SEQ ID NO : 354, SEQ ID NO : 356 ou SEQ ID NO : 358, telle que définie ci-dessus,
- ou toute séquence nucléotidique homologue de l'une des séquences nucléotidiques susmentionnées, ayant de préférence une identité d'au moins environ 50%, avec la séquence SEQ ID NO : 353, SEQ ID NO : 355 ou SEQ ID NO : 357, codant pour une protéine homologue de SEQ ID NO : 354, SEQ ID NO : 356 ou SEQ ID NO : 358, telle que définie ci-dessus,
- ou toute séquence nucléotidique complémentaire des séquences susmentionnées,
- ou toute séquence nucléotidique susceptible d'hybrider dans des conditions stringentes avec la séquence complémentaire d'une des séquences susmentionnées.

L'expression "hybrider dans des conditions stringentes" correspond notamment à un tampon d'hybridation tel que 0,5X SSC, à une température d'hybridation de 60°C, ainsi qu'à un milieu de lavage tel que 0,1 X SSC additionné de 1% de SDS et à une température de lavage de 50°C.

La présente invention concerne également un vecteur recombinant, notamment plasmide, cosmide, phage ou ADN de virus, contenant une séquence nucléotidique telle que définie ci-dessus, ledit vecteur recombinant contenant de préférence les éléments nécessaires à l'expression dans une cellule hôte des polypeptides codés par les acides nucléiques susmentionnés, insérés dans ledit vecteur.

La présente invention concerne également une cellule hôte, choisie notamment parmi les bactéries, les virus, les levures, les champignons, les plantes ou les cellules de mammifères, ladite cellule hôte étant transformée, notamment à l'aide d'un vecteur recombinant tel que défini ci-dessus.

La présente invention concerne un anticorps caractérisé en ce qu'il est dirigé de manière spécifique contre l'un des fragments de RGM susmentionnés.

La présente invention concerne également une composition pharmaceutique, comprenant à titre de substance active,
- l'un des fragments de RGM susmentionnés, ou
- une séquence nucléotidique telle que définie ci-dessus, codant pour l'un des fragments de RGM susmentionnés, ou
- un anticorps tel que défini ci-dessus, dirigé contre l'un des fragments de RGM susmentionnés.

La présente invention concerne également l'utilisation
- de l'un des fragments de RGM susmentionnés, ou
- d'une séquence nucléotidique telle que définie ci-dessus, codant pour l'un des fragments de RGM susmentionnés, ou
- d'un anticorps tel que défini ci-dessus, dirigé contre l'un des fragments de RGM susmentionnés,
pour la préparation d'un médicament destiné au traitement de pathologies nécessitant l'inhibition de la prolifération endothéliale, notamment dans le cadre des pathologies suivantes : les cancers et les leucémies, la dégénérescence maculaire liée à l'âge, les rétinopathies diabétiques, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

La présente invention concerne également l'utilisation de fragments de nétrine 1, de nétrine 3 et de ses fragments, de nétrine G1 et de ses fragments, ainsi que les nouveaux fragments correspondants

En particulier, la présente invention concerne l'utilisation :
- d'une protéine caractérisée en ce qu'elle comprend ou est constituée par :
   * la nétrine G1 représentée par la séquence SEQ ID NO : 506 ou par la séquence SEQ ID NO : 508, ou la nétrine 3 représentée par la séquence SEQ ID NO : 510,
   * un fragment de l'une de ces protéines ou de la nétrine 1 représentée par la séquence SEQ ID NO : 502 ou SEQ ID NO : 504, sous réserve que ce fragment présente une activité anti-angiogénique, ledit fragment comprenant notamment environ 40 à environ 400 acides aminés, et étant notamment représenté par l'une des séquences SEQ ID NO : 2m, m variant de 64 à 176, ou par la séquence SEQ ID NO : 372,
   * toute séquence dérivée de la séquence SEQ ID NO : 506, SEQ ID NO : 508, SEQ ID NO : 510, ou d'un fragment défini ci-dessus, notamment par substitution, suppression ou addition d'un ou plusieurs acides aminés, sous réserve que cette séquence dérivée présente une activité anti-angiogénique, ou
   * toute séquence homologue de la séquence SEQ ID NO : 506, SEQ ID NO : 508, SEQ ID NO : 510, ou d'un fragment défini ci-dessus, ayant de préférence une identité d'au moins environ 50% avec la région comprise entre les acides aminés en position 296 et 438 de la séquence SEQ ID NO : 506, sous réserve que cette séquence homologue présente une activité anti-angiogénique,
- ou d'une séquence nucléotidique caractérisée en ce qu'elle comprend ou est constituée par une séquence nucléotidique codant :
   * soit pour l'une des protéines telles que définies ci-dessus,
   * soit pour un fragment de l'une des protéines telles que définies ci-dessus,
   * soit pour une séquence dérivée de l'une des protéines telles que définies ci-dessus,
   * soit pour une séquence homologue de l'une des protéines telles que définies ci-dessus,
   ladite séquence nucléotidique correspondant notamment à la séquence nucléotidique SEQ ID NO : 505 codant pour SEQ ID NO : 506, ou à la séquence nucléotidique SEQ ID NO : 507 codant pour SEQ ID NO : 508, ou à la séquence nucléotidique SEQ ID NO : 509 codant pour SEQ ID NO : 510, ou à une séquence SEQ ID NO : 2m-1 codant pour SEQ ID NO : 2m, m variant de 64 à 176, ou à la séquence SEQ ID NO : 371 codant pour SEQ ID NO : 372,
- ou d'un anticorps anti-idiotypique de l'une des protéines telles que définies ci-dessus,
- ou d'un anticorps de l'une des protéines telles que définies ci-dessus,
- ou d'un fragment Fab d'anticorps anti-idiotypiques tels que définis ci-dessus,
pour la préparation d'un médicament destiné à la prévention ou au traitement des pathologies nécessitant l'inhibition de la prolifération endothéliale, notamment dans le cadre des pathologies suivantes : les cancers et les leucémies, la dégénérescence maculaire liée à l'âge, les rétinopathies diabétiques, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

Les séquences SEQ ID NO : 2m susmentionnées correspondent aux séquences protéiques SEQ ID NO : 128 à SEQ ID NO : 352, c'est-à-dire aux séquences protéiques suivantes : SEQ ID NO : 128, SEQ ID NO : 130, SEQ ID NO : 132, SEQ ID NO : 134, SEQ ID NO : 136, SEQ ID NO : 138, SEQ ID NO : 140, SEQ ID NO : 142, SEQ ID NO : 144, SEQ ID NO : 146, SEQ ID NO : 148, SEQ ID NO : 150, SEQ ID NO : 152, SEQ ID NO : 154, SEQ ID NO : 156, SEQ ID NO : 158, SEQ ID NO : 160, SEQ ID NO : 162, SEQ ID NO : 164, SEQ ID NO : 166, SEQ ID NO : 168, SEQ ID NO : 170, SEQ ID NO : 172, SEQ ID NO : 174, SEQ ID NO : 176, SEQ ID NO : 178, SEQ ID NO : 180, SEQ ID NO : 182, SEQ ID NO : 184, SEQ ID NO : 186, SEQ ID NO : 188, SEQ ID NO : 190, SEQ ID NO : 192, SEQ ID NO : 194, SEQ ID NO : 196, SEQ ID NO : 198, SEQ ID NO : 200, SEQ ID NO : 202, SEQ ID NO : 204, SEQ ID NO : 206, SEQ ID NO : 208, SEQ ID NO : 210, SEQ ID NO : 212, SEQ ID NO : 214, SEQ ID NO : 216, SEQ ID NO : 218, SEQ ID NO : 220, SEQ ID NO : 222, SEQ ID NO : 224, SEQ ID NO : 226, SEQ ID NO : 228, SEQ ID NO : 230, SEQ ID NO : 232, SEQ ID NO : 234, SEQ ID NO : 236, SEQ ID NO : 238, SEQ ID NO : 240, SEQ ID NO : 242, SEQ ID NO : 244, SEQ ID NO : 246, SEQ ID NO : 248, SEQ ID NO : 250, SEQ ID NO : 252, SEQ ID NO : 254, SEQ ID NO : 256, SEQ ID NO : 258, SEQ ID NO : 260, SEQ ID NO : 262, SEQ ID NO : 264, SEQ ID NO : 266, SEQ ID NO : 268, SEQ ID NO : 270, SEQ ID NO : 272, SEQ ID NO : 274, SEQ ID NO : 276, SEQ ID NO : 278, SEQ ID NO : 280, SEQ ID NO : 282, SEQ ID NO : 284, SEQ ID NO : 286, SEQ ID NO : 288, SEQ ID NO : 290, SEQ ID NO : 292, SEQ ID NO : 294, SEQ ID NO : 296, SEQ ID NO : 298, SEQ ID NO : 300, SEQ ID NO : 302, SEQ ID NO : 304, SEQ ID NO : 306, SEQ ID NO : 308, SEQ ID NO : 310, SEQ ID NO : 312, SEQ ID NO : 314, SEQ ID NO : 316, SEQ ID NO : 318, SEQ ID NO : 320, SEQ ID NO : 322, SEQ ID NO : 324, SEQ ID NO : 326, SEQ ID NO : 328, SEQ ID NO : 330, SEQ ID NO : 332, SEQ ID NO : 334, SEQ ID NO : 336, SEQ ID NO : 338, SEQ ID NO : 340, SEQ ID NO : 342, SEQ ID NO : 344, SEQ ID NO : 346, SEQ ID NO : 348, SEQ ID NO : 350, SEQ ID NO : 352.

Les séquences SEQ ID NO : 2m-1 susmentionnées codent pour les séquences protéiques susmentionnées SEQ ID NO: 2m, et correspondent aux séquences nucléotidiques suivantes : SEQ ID NO : 127 codant pour SEQ ID NO : 128, SEQ ID NO : 129 codant pour SEQ ID NO : 130, SEQ ID NO : 131 codant pour SEQ ID NO : 132, SEQ ID NO : 133 codant pour SEQ ID NO : 134, SEQ ID NO : 135 codant pour SEQ ID NO : 136, SEQ ID NO : 137 codant pour SEQ ID NO : 138, SEQ ID NO : 139 codant pour SEQ ID NO : 140, SEQ ID NO : 141 codant pour SEQ ID NO : 142, SEQ ID NO : 143 codant pour SEQ ID NO : 144, SEQ ID NO : 145 codant pour SEQ ID NO : 146, SEQ ID NO : 147 codant pour SEQ ID NO : 148, SEQ ID NO : 149 codant pour SEQ ID NO : 150, SEQ ID NO : 151 codant pour SEQ ID NO : 152, SEQ ID NO : 153 codant pour SEQ ID NO : 154, SEQ ID NO : 155 codant pour SEQ ID NO : 156, SEQ ID NO : 157 codant pour SEQ ID NO : 158, SEQ ID NO : 159 codant pour SEQ ID NO : 160, SEQ ID NO : 161 codant pour SEQ ID NO : 162, SEQ ID NO : 163 codant pour SEQ ID NO : 164, SEQ ID NO : 165 codant pour SEQ ID NO : 166, SEQ ID NO : 167 codant pour SEQ ID NO : 168, SEQ ID NO : 169 codant pour SEQ ID NO : 170, SEQ ID NO : 171 codant pour SEQ ID NO : 172, SEQ ID NO : 173 codant pour SEQ ID NO : 174, SEQ ID NO : 175 codant pour SEQ ID NO : 176, SEQ ID NO : 177 codant pour SEQ ID NO : 178, SEQ ID NO : 179 codant pour SEQ ID NO : 180, SEQ ID NO : 181 codant pour SEQ ID NO : 182, SEQ ID NO : 183 codant pour SEQ ID NO : 184, SEQ ID NO : 185 codant pour SEQ ID NO : 186, SEQ ID NO : 187 codant pour SEQ ID NO : 188, SEQ ID NO : 189 codant pour SEQ ID NO : 190, SEQ ID NO : 191 codant pour SEQ ID NO : 192, SEQ ID NO : 193 codant pour SEQ ID NO : 194, SEQ ID NO : 195 codant pour SEQ ID NO : 196, SEQ ID NO : 197 codant pour SEQ ID NO : 198, SEQ ID NO : 199 codant pour SEQ ID NO : 200, SEQ ID NO : 201 codant pour SEQ ID NO : 202, SEQ ID NO : 203 codant pour SEQ ID NO : 204, SEQ ID NO : 205 codant pour SEQ ID NO : 206, SEQ ID NO : 207 codant pour SEQ ID NO : 208, SEQ ID NO : 209 codant pour SEQ ID NO : 210, SEQ ID NO : 211 codant pour SEQ ID NO : 212, SEQ ID NO : 213 codant pour SEQ ID NO : 214, SEQ ID NO : 215 codant pour SEQ ID NO : 216, SEQ ID NO : 217 codant pour SEQ ID NO : 218, SEQ ID NO : 219 codant pour SEQ ID NO : 220, SEQ ID NO : 221 codant pour SEQ ID NO : 222, SEQ ID NO : 223 codant pour SEQ ID NO : 224, SEQ ID NO : 225 codant pour SEQ ID NO : 226, SEQ ID NO : 227 codant pour SEQ ID NO : 228, SEQ ID NO : 229 codant pour SEQ ID NO : 230, SEQ ID NO : 231 codant pour SEQ ID NO : 232, SEQ ID NO : 233 codant pour SEQ ID NO : 234, SEQ ID NO : 235 codant pour SEQ ID NO : 236, SEQ ID NO : 237 codant pour SEQ ID NO : 238, SEQ ID NO : 239 codant pour SEQ ID NO : 240, SEQ ID NO : 241 codant pour SEQ ID NO : 242, SEQ ID NO : 243 codant pour SEQ ID NO : 244, SEQ ID NO : 245 codant pour SEQ ID NO : 246, SEQ ID NO : 247 codant pour SEQ ID NO : 248, SEQ ID NO : 249 codant pour SEQ ID NO : 250, SEQ ID NO : 251 codant pour SEQ ID NO : 252, SEQ ID NO : 253 codant pour SEQ ID NO : 254, SEQ ID NO : 255 codant pour SEQ ID NO : 256, SEQ ID NO : 257 codant pour SEQ ID NO : 258, SEQ ID NO : 259 codant pour SEQ ID NO : 260, SEQ ID NO : 261 codant pour SEQ ID NO : 262, SEQ ID NO : 263 codant pour SEQ ID NO : 264, SEQ ID NO : 265 codant pour SEQ ID NO : 266, SEQ ID NO : 267 codant pour SEQ ID NO : 268, SEQ ID NO : 269 codant pour SEQ ID NO : 270, SEQ ID NO : 271 codant pour SEQ ID NO : 272, SEQ ID NO : 273 codant pour SEQ ID NO : 274, SEQ ID NO : 275 codant pour SEQ ID NO : 276, SEQ ID NO : 277 codant pour SEQ ID NO : 278, SEQ ID NO : 279 codant pour SEQ ID NO : 280, SEQ ID NO : 281 codant pour SEQ ID NO : 282, SEQ ID NO : 283 codant pour SEQ ID NO : 284, SEQ ID NO : 285 codant pour SEQ ID NO : 286, SEQ ID NO : 287 codant pour SEQ ID NO : 288, SEQ ID NO : 289 codant pour SEQ ID NO : 290, SEQ ID NO : 291 codant pour SEQ ID NO : 292, SEQ ID NO : 293 codant pour SEQ ID NO : 294, SEQ ID NO : 295 codant pour SEQ ID NO : 296, SEQ ID NO : 297 codant pour SEQ ID NO : 298, SEQ ID NO : 299 codant pour SEQ ID NO : 300, SEQ ID NO : 301 codant pour SEQ ID NO : 302, SEQ ID NO : 303 codant pour SEQ ID NO : 304, SEQ ID NO : 305 codant pour SEQ ID NO : 306, SEQ ID NO : 307 codant pour SEQ ID NO : 308, SEQ ID NO : 309 codant pour SEQ ID NO : 310, SEQ ID NO : 311 codant pour SEQ ID NO : 312, SEQ ID NO : 313 codant pour SEQ ID NO : 314, SEQ ID NO : 315 codant pour SEQ ID NO : 316, SEQ ID NO : 317 codant pour SEQ ID NO : 318, SEQ ID NO : 319 codant pour SEQ ID NO : 320, SEQ ID NO : 321 codant pour SEQ ID NO : 322, SEQ ID NO : 323 codant pour SEQ ID NO : 324, SEQ ID NO : 325 codant pour SEQ ID NO : 326, SEQ ID NO : 327 codant pour SEQ ID NO : 328, SEQ ID NO : 329 codant pour SEQ ID NO : 330, SEQ ID NO : 331 codant pour SEQ ID NO : 332, SEQ ID NO : 333 codant pour SEQ ID NO : 334, SEQ ID NO : 335 codant pour SEQ ID NO : 336, SEQ ID NO : 337 codant pour SEQ ID NO : 338, SEQ ID NO : 339 codant pour SEQ ID NO : 340, SEQ ID NO : 341 codant pour SEQ ID NO : 342, SEQ ID NO : 343 codant pour SEQ ID NO : 344, SEQ ID NO : 345 codant pour SEQ ID NO : 346, SEQ ID NO : 347 codant pour SEQ ID NO : 348, SEQ ID NO : 349 codant pour SEQ ID NO : 350, SEQ ID NO : 351 codant pour SEQ ID NO : 352.

La présente invention concerne une protéine, caractérisée en ce qu'elle comprend ou est constituée par l'une des séquences suivantes :
- une séquence SEQ ID NO : 2i, i variant de 64 à 131, ou
- toute séquence dérivée de l'une des séquences susmentionnées, notamment par substitution, suppression ou addition d'un ou plusieurs acides aminés, sous réserve que cette séquence dérivée présente une activité anti-angiogénique, ou
- toute séquence homologue de l'une des séquences susmentionnées, ayant de préférence une identité d'au moins environ 50% avec la région comprise entre les acides aminés en position 284 et 487 de SEQ ID NO : 502, sous réserve que cette séquence homologue présente une activité anti-angiogénique,
sous réserve que ladite protéine soit différente de la séquence SEQ ID NO : 502 ou SEQ ID NO : 504.

Les séquences SEQ ID NO : 2i susmentionnées sont des fragments de la nétrine 1 et correspondent aux séquences protéiques SEQ ID NO : 128 à SEQ ID NO : 262, c'est-à-dire les séquences protéiques suivantes : SEQ ID NO : 128, SEQ ID NO : 130, SEQ ID NO : 132, SEQ ID NO : 134, SEQ ID NO: 136, SEQ ID NO : 138, SEQ ID NO : 140, SEQ ID NO : 142, SEQ ID NO : 144, SEQ ID NO : 146, SEQ ID NO : 148, SEQ ID NO : 150, SEQ ID NO : 152, SEQ ID NO : 154, SEQ ID NO : 156, SEQ ID NO : 158, SEQ ID NO : 160, SEQ ID NO : 162, SEQ ID NO : 164, SEQ ID NO : 166, SEQ ID NO : 168, SEQ ID NO : 170, SEQ ID NO : 172, SEQ ID NO : 174, SEQ ID NO : 176, SEQ ID NO : 178, SEQ ID NO : 180, SEQ ID NO : 182, SEQ ID NO : 184, SEQ ID NO : 186, SEQ ID NO : 188, SEQ ID NO : 190, SEQ ID NO : 192, SEQ ID NO : 194, SEQ ID NO : 196, SEQ ID NO : 198, SEQ ID NO : 200, SEQ ID NO : 202, SEQ ID NO : 204, SEQ ID NO : 206, SEQ ID NO : 208, SEQ ID NO : 210, SEQ ID NO : 212, SEQ ID NO : 214, SEQ ID NO : 216, SEQ ID NO : 218, SEQ ID NO : 220, SEQ ID NO : 222, SEQ ID NO : 224, SEQ ID NO : 226, SEQ ID NO : 228, SEQ ID NO : 230, SEQ ID NO : 232, SEQ ID NO : 234, SEQ ID NO : 236, SEQ ID NO : 238, SEQ ID NO : 240, SEQ ID NO : 242, SEQ ID NO : 244, SEQ ID NO : 246, SEQ ID NO : 248, SEQ ID NO : 250, SEQ ID NO : 252, SEQ ID NO : 254, SEQ ID NO : 256, SEQ ID NO : 258, SEQ ID NO : 260 ou SEQ ID NO : 262.

La présente invention concerne également les fragments de la nétrine G1, caractérisés en ce qu'ils comprennent ou sont constitués par l'une des séquences suivantes :
- une séquence SEQ ID NO : 2j, j variant de 132 à 148, ou
- toute séquence dérivée de l'une des séquences susmentionnées, notamment par substitution, suppression ou addition d'un ou plusieurs acides aminés, sous réserve que cette séquence dérivée présente une activité anti-angiogénique, ou
- toute séquence homologue de l'une des séquences susmentionnées, ayant de préférence une identité d'au moins environ 50% avec la région comprise entre les acides aminés en position 296 et 438 de SEQ ID NO : 506, sous réserve que cette séquence homologue présente une activité anti-angiogénique.

Les séquences SEQ ID NO : 2j susmentionnées correspondent aux séquences protéiques SEQ ID NO : 264 à SEQ ID NO : 296, c'est-à-dire : SEQ ID NO : 264, SEQ ID NO : 266, SEQ ID NO :268, SEQ ID NO : 270, SEQ ID NO :272, SEQ ID NO : 274, SEQ ID NO : 276, SEQ ID NO : 278, SEQ ID NO : 280, SEQ ID NO : 282, SEQ ID NO : 284, SEQ ID NO : 286, SEQ ID NO : 288, SEQ ID NO : 290, SEQ ID NO : 292, SEQ ID NO : 294 ou SEQ ID NO : 296.

La présente invention concerne également les fragments de la nétrine 3, caractérisés en ce qu'ils comprennent ou sont constitués par l'une des séquences suivantes :
- une séquence SEQ ID NO : 2k, k variant de 149 à 176, ou
- toute séquence dérivée de l'une des séquences susmentionnées, notamment par substitution, suppression ou addition d'un ou plusieurs acides aminés, sous réserve que cette séquence dérivée présente une activité anti-angiogénique, ou
- toute séquence homologue de l'une des séquences susmentionnées, ayant de préférence une identité d'au moins environ 50% avec la région comprise entre les acides aminés en position 254 et 433 de SEQ ID NO : 510, sous réserve que cette séquence homologue présente une activité anti-angiogénique.

Les séquences SEQ ID NO : 2k susmentionnées correspondent aux séquences protéiques SEQ ID NO : 298 à 352, c'est-à-dire aux séquence protéiques suivantes : SEQ ID NO : 298, SEQ ID NO : 300, SEQ ID NO : 302, SEQ ID NO : 304, SEQ ID NO : 306, SEQ ID NO : 308, SEQ ID NO : 310, SEQ ID NO : 312, SEQ ID NO : 314, SEQ ID NO : 316, SEQ ID NO : 318, SEQ ID NO : 320, SEQ ID NO : 322, SEQ ID NO : 324, SEQ ID NO : 326, SEQ ID NO : 328, SEQ ID NO : 330, SEQ ID NO : 332, SEQ ID NO : 334, SEQ ID NO : 336, SEQ ID NO : 338, SEQ ID NO : 340, SEQ ID NO : 342, SEQ ID NO : 344, SEQ ID NO : 346, SEQ ID NO : 348, SEQ ID NO : 350 ou SEQ ID NO : 352.

La présente invention concerne également une séquence nucléotidique codant pour une protéine telle que définie ci-dessus, à savoir une séquence nucléotidique codant pour les fragments de la nétrine 1.

Une séquence nucléotidique avantageuse selon l'invention est caractérisée en ce qu'elle comprend ou est constituée par :
- la séquence nucléotidique SEQ ID NO : 2i-1, i variant de 64 à 131,
- ou toute séquence nucléotidique dérivée, par dégénérescence du code génétique, de l'une des séquences nucléotidiques susmentionnées, et codant pour une protéine représentée par l'une des séquences SEQ ID NO : 2i, i variant de 64 à 131,
- ou toute séquence nucléotidique dérivée, notamment par substitution, suppression ou addition d'un ou plusieurs nucléotides, de l'une des séquences nucléotidiques susmentionnées codant pour une protéine dérivée de l'une des séquences SEQ ID NO : 2i, i variant de 64 à 131, telle que définie ci-dessus,
- ou toute séquence nucléotidique homologue de l'une des séquences nucléotidiques susmentionnées, ayant de préférence une identité d'au moins environ 50%, avec l'une des séquences SEQ ID NO : 2i-1 codant pour une protéine homologue de SEQ ID NO : 2i, i variant de 64 à 131, telle que définie ci-dessus,
- ou toute séquence nucléotidique complémentaire des séquences susmentionnées,
- ou toute séquence nucléotidique susceptible d'hybrider dans des conditions stringentes avec la séquence complémentaire d'une des séquences susmentionnées sous réserve que ladite séquence nucléotidique soit différente de la séquence SEQ ID NO : 501 ou SEQ ID NO : 503.

Les séquences SEQ ID NO : 2i-1 susmentionnées codent pour les fragments de nétrine 1 susmentionnés, représentés par SEQ ID NO 2i, et correspondent aux séquences nucléotidiques suivantes : SEQ ID NO : 127 codant pour SEQ ID NO : 128, SEQ ID NO : 129 codant pour SEQ ID NO : 130, SEQ ID NO : 131 codant pour SEQ ID NO : 132, SEQ ID NO : 133 codant pour SEQ ID NO : 134, SEQ ID NO : 135 codant pour SEQ ID NO : 136, SEQ ID NO : 137 codant pour SEQ ID NO : 138, SEQ ID NO : 139 codant pour SEQ ID NO : 140, SEQ ID NO : 141 codant pour SEQ ID NO : 142, SEQ ID NO : 143 codant pour SEQ ID NO : 144, SEQ ID NO : 145 codant pour SEQ ID NO : 146, SEQ ID NO : 147 codant pour SEQ ID NO : 148, SEQ ID NO : 149 codant pour SEQ ID NO : 150, SEQ ID NO : 151 codant pour SEQ ID NO : 152, SEQ ID NO : 153 codant pour SEQ ID NO : 154, SEQ ID NO : 155 codant pour SEQ ID NO : 156, SEQ ID NO : 157 codant pour SEQ ID NO : 158, SEQ ID NO : 159 codant pour SEQ ID NO : 160, SEQ ID NO : 161 codant pour SEQ ID NO : 162, SEQ ID NO : 163 codant pour SEQ ID NO : 164, SEQ ID NO : 165 codant pour SEQ ID NO : 166, SEQ ID NO : 167 codant pour SEQ ID NO : 168, SEQ ID NO : 169 codant pour SEQ ID NO : 170, SEQ ID NO : 171 codant pour SEQ ID NO : 172, SEQ ID NO : 173 codant pour SEQ ID NO : 174, SEQ ID NO : 175 codant pour SEQ ID NO : 176, SEQ ID NO : 177 codant pour SEQ ID NO : 178, SEQ ID NO : 179 codant pour SEQ ID NO : 180, SEQ ID NO : 181 codant pour SEQ ID NO : 182, SEQ ID NO : 183 codant pour SEQ ID NO : 184, SEQ ID NO : 185 codant pour SEQ ID NO : 186, SEQ ID NO : 187 codant pour SEQ ID NO : 188, SEQ ID NO : 189 codant pour SEQ ID NO : 190, SEQ ID NO : 191 codant pour SEQ ID NO : 192, SEQ ID NO : 193 codant pour SEQ ID NO : 194, SEQ ID NO : 195 codant pour SEQ ID NO : 196, SEQ ID NO : 197 codant pour SEQ ID NO : 198, SEQ ID NO : 199 codant pour SEQ ID NO : 200, SEQ ID NO : 201 codant pour SEQ ID NO : 202, SEQ ID NO : 203 codant pour SEQ ID NO : 204, SEQ ID NO : 205 codant pour SEQ ID NO : 206, SEQ ID NO : 207 codant pour SEQ ID NO : 208, SEQ ID NO : 209 codant pour SEQ ID NO : 210, SEQ ID NO : 211 codant pour SEQ ID NO : 212, SEQ ID NO : 213 codant pour SEQ ID NO : 214, SEQ ID NO : 215 codant pour SEQ ID NO : 216, SEQ ID NO : 217 codant pour SEQ ID NO : 218, SEQ ID NO : 219 codant pour SEQ ID NO : 220, SEQ ID NO : 221 codant pour SEQ ID NO : 222, SEQ ID NO : 223 codant pour SEQ ID NO : 224, SEQ ID NO : 225 codant pour SEQ ID NO : 226, SEQ ID NO : 227 codant pour SEQ ID NO : 228, SEQ ID NO : 229 codant pour SEQ ID NO : 230, SEQ ID NO : 231 codant pour SEQ ID NO : 232, SEQ ID NO : 233 codant pour SEQ ID NO : 234, SEQ ID NO : 235 codant pour SEQ ID NO : 236, SEQ ID NO : 237 codant pour SEQ ID NO : 238, SEQ ID NO : 239 codant pour SEQ ID NO : 240, SEQ ID NO : 241 codant pour SEQ ID NO : 242, SEQ ID NO : 243 codant pour SEQ ID NO : 244, SEQ ID NO : 245 codant pour SEQ ID NO : 246, SEQ ID NO : 247 codant pour SEQ ID NO : 248, SEQ ID NO : 249 codant pour SEQ ID NO : 250, SEQ ID NO : 251 codant pour SEQ ID NO : 252, SEQ ID NO : 253 codant pour SEQ ID NO : 254, SEQ ID NO : 255 codant pour SEQ ID NO : 256, SEQ ID NO : 257 codant pour SEQ ID NO : 258, SEQ ID NO : 259 codant pour SEQ ID NO : 260 ou SEQ ID NO : 261 codant pour SEQ ID NO : 262.

La présente invention concerne également une composition pharmaceutique, comprenant à titre de substance active,
- une protéine correspondant aux fragments de nétrine 1, tels que définis ci-dessus, ou
- une séquence nucléotidique codant pour lesdits fragments de nétrine 1, tels que définis ci-dessus, ou
- un anticorps, caractérisé en ce qu'il est dirigé de manière spécifique contre une protéine telle que définie ci-dessus , ou
- un anticorps anti-idiotypique d'une protéine telle que définie ci-dessus, ou
- un fragment Fab d'anticorps anti-idiotypiques d'une protéine telle que définie ci-dessus.

La présente invention concerne l'utilisation :
- d'une protéine correspondant aux fragments de nétrine 1, tels que définis ci-dessus, ou
- d'une séquence nucléotidique codant pour lesdits fragments de nétrine 1, tels que définis ci-dessus, ou
- d'un anticorps anti-idiotypique d'une protéine telle que définie ci-dessus,
pour la préparation d'un médicament destiné à la prévention ou au traitement des pathologies nécessitant l'inhibition de la prolifération et/ou de la migration endothéliale, notamment dans le cadre des pathologies suivantes : les cancers et les leucémies, la dégénérescence maculaire liée à l'âge, la néovascularisation choroïdienne compliquant la myopie, la néovascularisation de la cornée en particulier le rejet de greffe, les glaucomes, les rétinopathies diabétiques ou du prématuré, les rétinopathies diabétiques, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

La présente invention concerne également l'utilisation :
- d'un anticorps, caractérisé en ce qu'il est dirigé de manière spécifique contre une protéine telle que définie ci-dessus, ou
- d'un fragment Fab d'anticorps anti-idiotypiques d'une protéine telle que définie ci-dessus,
pour la préparation d'un médicament destiné à la prévention ou au traitement des pathologies nécessitant la stimulation de la prolifération et/ou de la migration endothéliale, notamment dans le cadre des pathologies suivantes : pathologies ischémiques telles que l'artérite des membres inférieurs, l'infarctus du myocarde, les accidents vasculaires cérébraux, la sclérodermie ou la maladie de Raynaud.

La présente invention concerne également une séquence nucléotidique codant pour une protéine telle que définie ci-dessus, à savoir une séquence nucléotidique codant pour les fragments de la nétrine G1.

Une séquence nucléotidique avantageuse selon l'invention est caractérisée en ce qu'elle comprend ou est constituée par :
- la séquence nucléotidique SEQ ID NO : 2j-1, j variant de 132 à 148,
- ou toute séquence nucléotidique dérivée, par dégénérescence du code génétique, de l'une des séquences nucléotidiques susmentionnées, et codant pour une protéine représentée par l'une des séquences SEQ ID NO : 2j, j variant de 132 à 148,
- ou toute séquence nucléotidique dérivée, notamment par substitution, suppression ou addition d'un ou plusieurs nucléotides, de l'une des séquences nucléotidiques susmentionnées codant pour une protéine dérivée de l'une des séquences SEQ ID NO : 2j, j variant de 132 à 148, telle que définie ci-dessus,
- ou toute séquence nucléotidique homologue de l'une des séquences nucléotidiques susmentionnées, ayant de préférence une identité d'au moins environ 50%, avec l'une des séquences SEQ ID NO : 2j-1 codant pour une protéine homologue de l'une des séquences SEQ ID NO : 2j, j variant de 132 à 148, telle que définie ci-dessus,
- ou toute séquence nucléotidique complémentaire des séquences susmentionnées,
- ou toute séquence nucléotidique susceptible d'hybrider dans des conditions stringentes avec la séquence complémentaire d'une des séquences susmentionnées.

Les séquences SEQ ID NO : 2j-1 susmentionnées codent pour les fragments de nétrine G1 susmentionnés, représentés par SEQ ID NO : 2j, et correspondent aux séquences nucléotidiques suivantes : SEQ ID NO : 263 codant pour SEQ ID NO : 264, SEQ ID NO : 265 codant pour SEQ ID NO : 266, SEQ ID NO : 267 codant pour SEQ ID NO : 268, SEQ ID NO : 269 codant pour SEQ ID NO : 270, SEQ ID NO : 271 codant pour SEQ ID NO : 272, SEQ ID NO : 273 codant pour SEQ ID NO : 274, SEQ ID NO : 275 codant pour SEQ ID NO : 276, SEQ ID NO : 277 codant pour SEQ ID NO : 278, SEQ ID NO : 279 codant pour SEQ ID NO : 280, SEQ ID NO : 281 codant pour SEQ ID NO : 282, SEQ ID NO : 283 codant pour SEQ ID NO : 284, SEQ ID NO : 285 codant pour SEQ ID NO : 286, SEQ ID NO : 287 codant pour SEQ ID NO : 288, SEQ ID NO : 289 codant pour SEQ ID NO : 290, SEQ ID NO : 291 codant pour SEQ ID NO : 292, SEQ ID NO : 293 codant pour SEQ ID NO : 294 ou SEQ ID NO : 295 codant pour SEQ ID NO : 296.

La présente invention concerne également une séquence nucléotidique codant pour une protéine telle que définie ci-dessus, à savoir une séquence nucléotidique codant pour les fragments de la nétrine 3.

Une séquence nucléotidique avantageuse selon l'invention est caractérisée en ce qu'elle comprend ou est constituée par :
- la séquence nucléotidique SEQ ID NO : 2k-1, k variant de 149 à 176,
- ou toute séquence nucléotidique dérivée, par dégénérescence du code génétique, de l'une des séquences nucléotidiques susmentionnées, et codant pour une protéine représentée par l'une des séquences SEQ ID NO : 2k, k variant de 149 à 176,
- ou toute séquence nucléotidique dérivée, notamment par substitution, suppression ou addition d'un ou plusieurs nucléotides, de l'une des séquences nucléotidiques susmentionnées codant pour une protéine dérivée de l'une des séquences SEQ ID NO : 2k, k variant de 149 à 176, telle que définie ci-dessus,
- ou toute séquence nucléotidique homologue de l'une des séquences nucléotidiques susmentionnées, ayant de préférence une identité d'au moins environ 50%, avec l'une des séquences SEQ ID NO : 2k-1 codant pour une protéine homologue de l'une des séquences SEQ ID NO : 2k, k variant de 149 à 176, telle que définie ci-dessus,
- ou toute séquence nucléotidique complémentaire des séquences susmentionnées,
- ou toute séquence nucléotidique susceptible d'hybrider dans des conditions stringentes avec la séquence complémentaire d'une des séquences susmentionnées.

Les séquences SEQ ID NO : 2k-1 susmentionnées codent pour les fragments de nétrine 3 susmentionnés, représentés par SEQ ID NO 2k, et correspondent aux séquences nucléotidiques suivantes : SEQ ID NO : 297 codant pour SEQ ID NO : 298, SEQ ID NO : 299 codant pour SEQ ID NO : 300, SEQ ID NO : 301 codant pour SEQ ID NO : 302, SEQ ID NO : 303 codant pour SEQ ID NO : 304, SEQ ID NO : 305 codant pour SEQ ID NO : 306, SEQ ID NO : 307 codant pour SEQ ID NO : 308, SEQ ID NO : 309 codant pour SEQ ID NO : 310, SEQ ID NO : 311 codant pour SEQ ID NO : 312, SEQ ID NO : 313 codant pour SEQ ID NO : 314, SEQ ID NO : 315 codant pour SEQ ID NO : 316, SEQ ID NO : 317 codant pour SEQ ID NO : 318, SEQ ID NO : 319 codant pour SEQ ID NO : 320, SEQ ID NO : 321 codant pour SEQ ID NO : 322, SEQ ID NO : 323 codant pour SEQ ID NO : 324, SEQ ID NO : 325 codant pour SEQ ID NO : 326, SEQ ID NO : 327 codant pour SEQ ID NO : 328, SEQ ID NO : 329 codant pour SEQ ID NO : 330, SEQ ID NO : 331 codant pour SEQ ID NO : 332, SEQ ID NO : 333 codant pour SEQ ID NO : 334, SEQ ID NO : 335 codant pour SEQ ID NO : 336, SEQ ID NO : 337 codant pour SEQ ID NO : 338, SEQ ID NO : 339 codant pour SEQ ID NO : 340, SEQ ID NO : 341 codant pour SEQ ID NO : 342, SEQ ID NO : 343 codant pour SEQ ID NO : 344, SEQ ID NO : 345 codant pour SEQ ID NO : 346, SEQ ID NO : 347 codant pour SEQ ID NO : 348, SEQ ID NO : 349 codant pour SEQ ID NO : 350 ou SEQ ID NO : 351 codant pour SEQ ID NO : 352.

La présente invention concerne également un vecteur recombinant, notamment plasmide, cosmide, phage ou ADN de virus, contenant une séquence nucléotidique telle que définie ci-dessus, à savoir une séquence nucléotidique codant pour l'un des fragments de nétrine 1, de nétrine 3, ou de nétrine G, ledit vecteur recombinant étant notamment caractérisé en ce qu'il contient les éléments nécessaires à l'expression dans une cellule hôte des polypeptides codés par les acides nucléiques susmentionnés, insérés dans ledit vecteur.

La présente invention concerne également une cellule hôte, choisie notamment parmi les bactéries, les virus, les levures, les champignons, les plantes ou les cellules de mammifères, ladite cellule hôte étant transformée, notamment à l'aide d'un vecteur recombinant tel que défini ci-dessus.

La présente invention concerne également un anticorps caractérisé en ce qu'il est dirigé de manière spécifique contre une protéine telle que définie ci-dessus, notamment dirigé contre un fragment de la nétrine 1, de la nétrine 3, ou de la nétrine G ou tels que définis ci-dessus.

La présente invention concerne également une composition pharmaceutique, comprenant à titre de substance active,
- l'un des fragments de la nétrine 1, de la nétrine 3, ou de la nétrine G susmentionnés, ou
- une séquence nucléotidique telle que définie ci-dessus, codant pour l'un des fragments de la nétrine 1, de la nétrine 3, ou de la nétrine G susmentionnés, ou
- un anticorps tel que défini ci-dessus, dirigé contre l'un des fragments de la nétrine 1, de la nétrine 3, de la nétrine G susmentionnés, ou
- un anticorps anti-idiotypique de la nétrine 1, de la nétrine 3, ou de la nétrine G susmentionnés, ou
- un fragment Fab d'anticorps anti-idiotypique tels que définis ci-dessus.

La présente invention concerne également l'utilisation :
- d'une protéine caractérisée en ce qu'elle comprend ou est constituée par l'une des séquences suivantes :
   - une séquence SEQ ID NO : 2i, i variant de 64 à 131, ou
   - toute séquence dérivée de l'une des séquences susmentionnées, notamment par substitution, suppression ou addition d'un ou plusieurs acides aminés, sous réserve que cette séquence dérivée présente une activité anti-angiogénique, ou
   - toute séquence homologue de l'une des séquences susmentionnées, ayant de préférence une identité d'au moins environ 50% avec la région comprise entre les acides aminés en position 284 et 487 de SEQ ID NO : 502, sous réserve que cette séquence homologue présente une activité anti-angiogénique,
   sous réserve que ladite protéine soit différente de la séquence SEQ ID NO : 502 ou SEQ ID NO : 504
- ou d'une séquence nucléotidique codant pur l'une des protéines susmentionnées,
- ou d'un anticorps caractérisé en ce qu'il est dirigé de manière spécifique contre une protéine telle que définie ci-dessus,
- ou d'un anticorps anti-idiotypique d'une protéine telle que définie ci-dessus,
- ou d'un fragment Fab d'anticorps anti-idiotypique tels que définis ci-dessus,
pour la préparation d'un médicament destiné à la prévention ou au traitement des pathologies nécessitant l'inhibition de la prolifération endothéliale, notamment dans le cadre des pathologies suivantes : les cancers et les leucémies, la néovascularisation choroïdienne compliquant la myopie, la néovascularisation de la cornée en particulier le rejet de greffe, les glaucomes, les rétinopathies diabétiques ou du prématuré, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

La présente invention concerne les nouvelles utilisations des nétrines.

En particulier, la présente invention concerne l'utilisation :
- d'une protéine caractérisée en ce qu'elle comprend ou est constituée par :
   * la nétrine G1 représentée par la séquence SEQ ID NO : 506 ou par la séquence SEQ ID NO : 508, la nétrine 3 représentée par la séquence SEQ ID NO : 510 ou la nétrine 4 représentée par la séquence SEQ ID NO : 498 ou par la séquence SEQ ID NO : 500,
   * un fragment de l'une de ces protéines ou de la nétrine 1 représentée par la séquence SEQ ID NO : 502 ou SEQ ID NO : 504, sous réserve que ce fragment présente une activité d'activation des péricytes, ledit fragment comprenant notamment environ 40 à environ 400 acides aminés, et étant notamment représenté par l'une des séquences SEQ ID NO : 2m, m variant de 1 à 176, ou par la séquence SEQ ID NO : 372,
   * toute séquence dérivée de la séquence SEQ ID NO : 506, SEQ ID NO : 508, SEQ ID NO : 510, SEQ ID NO : 498, SEQ ID NO : 500 ou d'un fragment défini ci-dessus, notamment par substitution, suppression ou addition d'un ou plusieurs acides aminés, sous réserve que cette séquence dérivée présente une activité d'activation des péricytes, ou
   * toute séquence homologue de la séquence SEQ ID NO : 506, SEQ ID NO : 508, SEQ ID NO : 510, SEQ ID NO : 498, SEQ ID NO : 500 ou d'un fragment défini ci-dessus, ayant de préférence une identité d'au moins environ 50% avec la région comprise entre les acides aminés en position 296 et 438 de la séquence SEQ ID NO : 506, sous réserve que cette séquence homologue présente une activité d'activation des péricytes,
- ou d'une séquence nucléotidique caractérisée en ce qu'elle comprend ou est constituée par une séquence nucléotidique codant :
   * soit pour l'une des protéines telles que définies ci-dessus,
   * soit pour un fragment de l'une des protéines telles que définies ci-dessus,
   * soit pour une séquence dérivée de l'une des protéines telles que définies ci-dessus,
   * soit pour une séquence homologue de l'une des protéines telles que définies ci-dessus,
   ladite séquence nucléotidique correspondant notamment à la séquence nucléotidique SEQ ID NO : 505 codant pour SEQ ID NO : 506, ou à la séquence nucléotidique SEQ ID NO : 507 codant pour SEQ ID NO : 508, ou à la séquence nucléotidique SEQ ID NO : 509 codant pour SEQ ID NO : 510, ou à la séquence nucléotidique SEQ ID NO : 497 codant pour SEQ ID NO : 498, ou à la séquence nucléotidique SEQ ID NO 499 codant pour SEQ ID NO : 500, ou à une séquence SEQ ID NO : 2m-1 codant pour SEQ ID NO : 2m, m variant de 1 à 176, ou à la séquence SEQ ID NO : 371 codant pour SEQ ID NO : 372,
- ou d'un anticorps anti-idiotypique de l'une des protéines telles que définies ci-dessus,
- ou d'un anticorps de l'une des protéines telles que définies ci-dessus,
- ou d'un fragment Fab d'anticorps anti-idiotypiques tels que définis ci-dessus,
pour la préparation d'un médicament destiné à la prévention ou au traitement des pathologies non tumorales liées à, ou causées par, une raréfaction des péricytes ou des cellules musculaires lisses.

La présente invention concerne également l'utilisation telle que définie ci-dessus de la protéine nétrine 4, représentée par la séquence SEQ ID NO : 498 ou SEQ ID NO : 500, pour la préparation d'un médicament susceptible d'être administré à raison d'environ 0,1 à environ 20 mg/kg, notamment par voie intraveineuse, par voie sous-cutanée, par voie systémique, par injection intra-vitréenne, par voie locale au moyen d'infiltrations ou par l'intermédiaire d'un collyre, éventuellement associé à une électroperméation.

La présente invention concerne également l'utilisation telle que définie ci-dessus, caractérisée en ce que l'activité d'inhibition de la formation des plaques d'athérome est mesurée selon le test de prolifération ou de migration susmentionnés, et en ce que cette activité d'inhibition correspond à un pourcentage d'inhibition compris de 20% à 100% des plaques d'athérome obtenues en l'absence de la protéine, ou de la séquence nucléotidique ou de l'anticorps anti-idiotypique tels que définis ci-dessus.

L'activité anti-athéromateuse est mesurée par le test tel que décrit dans Arnal et al. (2003).

Selon un mode de réalisation avantageux, la présente invention concerne l'utilisation telle que définie ci-dessus d'anticorps anti-idiotypiques d'une nétrine, notamment de la protéine nétrine 4 mutée ou non, représentée par la séquence SEQ ID NO : 522 ou SEQ ID NO : 524, et par la séquence SEQ ID NO : 498 ou SEQ ID NO : 500, reconnaissant le récepteur UNC5H4 à la surface des péricytes ou cellules musculaires lisses, pour la préparation d'un médicament destiné à la prévention ou au traitement des pathologies suivantes :
- la dégénérescence maculaire liée à l'âge, la néovascularisation choroidienne compliquant la myopie
- les rétinopathies diabétiques ou du prématuré,
- le glaucome néovasculaire (de la cornée, de la rétine...)
- les néovascularisations cornéennes en particulier le rejet de greffe
- la polyarthrite rhumatoïde,
- le psoriasis, en particulier la polyarthrite psoriasique,
- les angiomes,
- l'athérosclérose,
- l'obésité,
- les malformations intestinales,
- la maladie de Crohn,
- les démences vasculaires, sous-corticales vasculaires dont Cadasil est un exemple,
- la maladie d'Alzheimer,
- les pathologies dégénératives osseuses et les fractures, et
- les anévrysmes et les dissections vasculaires.

La présente invention concerne également l'utilisation du récepteur UNC5H4 à la surface des péricytes ou cellules musculaires lisses, ou de péricytes ou cellules musculaires lisses porteuses de ces récepteurs, pour la mise en oeuvre d'un procédé de criblage de composés activateurs de la prolifération ou de la migration des péricytes ou des cellules musculaires lisses.

La présente invention concerne un procédé de criblage de composés activateurs de la prolifération ou de la migration des péricytes ou des cellules musculaires lisses, caractérisé en ce qu'il comprend les étapes suivantes :
- la mesure de l'inhibition de la liaison de la nétrine 4 à son récepteur UNC5H4, ou de l'inhibition de l'activité mitogénique, chimiotactique, anti-apoptotique ou de différenciation des nétrines ou de tout autre facteur de type nétrine sur les cellules musculaires lisses ou péricytes, et
- la mesure de la perte de l'inhibition de l'activité mitogénique ou chimiotactique ou anti-apoptotique ou de différenciation des nétrines ou de tout autre facteur de type nétrine sur les cellules musculaires lisses ou péricytes, par adjonction d'un anticorps neutralisant le récepteur UNCSH4.

Les tests utilisés dans le cadre de ce procédé de criblage sont les tests tels que décrits ci-dessus de prolifération ou de migration.

La présente invention concerne un procédé de criblage de composés modulateurs de la liaison entre le récepteur UNC5H4 et la nétrine 4 mutée ou non, représentée par la séquence SEQ ID NO : 522 ou SEQ ID NO : 524, ou par la séquence SEQ ID NO : 498 ou SEQ ID NO : 500, caractérisé en ce qu'il comprend les étapes suivantes :
- la mise en présence dudit composé modulateur avec le récepteur UNC5H4 et la nétrine 4, et
- la mesure du taux de liaison spécifique entre le récepteur UNC5H4 et la nétrine 4, une différence dudit taux en présence dudit composé par rapport au taux normal en l'absence dudit composé étant une indication de la propriété dudit composé à moduler la liaison entre le récepteur UNC5H4 et la nétrine 4.

Le test de criblage est effectué de la manière suivante :
Des immunoglobulines de chèvre dirigées contre les domaines Fc des IgG humaines sont incubées sur des plaques de microtitration (0,1-20 µg/ml dans du tampon carbonate 50 mM pH 9,6). Après saturation des sites non spécifiques par une solution de sérum albumine diluée à 5 mg/ml dans le même tampon, les protéines contenant les domaines extracellulaires du récepteur UNC5H4 fusionné à une séquence Fc d'IgG humaine sont immobilisées sur les plaques de microtitration (incubation à une concentration comprise entre 1 et 1000 ng/ml). Les composés sont ajoutés dilués en série dans du tampon PBS contenant 0,05% de Tween 20 et 10-500 pg de nétrine 1, 3, 4 ou G soit marquée par tout procédé convenable (iode radioactif, biotine, fluorochrome). Après rinçages, la quantité de nétrine liée au récepteur est révélée soit par adjonction d'une concentration appropriée d'anticorps anti-nétrine couplé à la peroxydase, soit par mesure de radioactivité, soit par adjonction d'avidine couplée à la peroxydase, soit par mesure de fluorescence. La peroxydase est ensuite révélée par une réaction colorimétrique. Dans tous les cas, la quantité du composé à tester est mesurée par sa capacité à inhiber la quantité de nétrine fixée.

| **Protéines** | Séquence protéique | Séquence nucléotidique |
|---|---|---|
| **Nétrine 4** (avec peptide signal)*(1-628)* | SEQ ID NO : 498 | SEQ ID NO : 497 |
| **Nétrine 4** (sans peptide signal)*(20-628)* | SEQ ID NO : 500 | SEQ ID NO : 499 |
| **Nétrine 1** (avec peptide signal)*(1-604)* | SEQ ID NO : 502 | SEQ ID NO : 501 |
| **Nétrine 1** (sans peptide signal)*(25-604)* | SEQ ID NO : 504 | SEQ ID NO : 503 |
| **Nétrine G1** (avec peptide signal)*(1-438)* | SEQ ID NO : 506 | SEQ ID NO : 505 |
| **Nétrine G1** (sans peptide signal)*(29-438)* | SEQ ID NO : 508 | SEQ ID NO : 507 |
| **Nétrine 3** *(1-580)* | SEQ ID NO : 510 | SEQ ID NO : 509 |
| **RGMA** *(1-450)* | SEQ ID NO : 512 | SEQ ID NO : 511 |
| **RGMB** *(1-437)* | SEQ ID NO : 514 | SEQ ID NO : 513 |
| **RGMC** *(1-426)* | SEQ ID NO : 516 | SEQ ID NO : 515 |
| **RGMB sans peptide signal** *(46-437)* | SEQ ID NO : 518 | SEQ ID NO : 517 |
| **RGMC sans peptide signal** *(36-426)* | SEQ ID NO : 520 | SEQ ID NO : 519 |
| **Nétrine 4 mutée** (avec peptide signal)*(1-628)* | SEQ ID NO : 522 | SEQ ID NO : 521 |
| **Nétrine 4 mutée** (sans peptide signal)*(20-628)* | SEQ ID NO : 524 | SEQ ID NO : 523 |

| **Fragments de nétrine 4** | Séquence protéique | Séquence nucléotidique |
|---|---|---|
| **1** *(1-260)* | SEQ ID NO : 360 | SEQ ID NO : 359 |
| **2** *(261-515)* | SEQ ID NO : 362 | SEQ ID NO : 361 |
| **3***(516-628)* | SEQ ID NO : 364 | SEQ ID NO : 363 |
| **1**+**2***(1-515)* | SEQ ID NO : 366 | SEQ ID NO : 365 |
| **1a+2** *(32-515)* | SEQ ID NO : 368 | SEQ ID NO : 367 |
| **1a+2+3** *(32-628)* | SEQ ID NO : 370 | SEQ ID NO : 369 |
| **2a** *(261-320)* | SEQ ID NO : 2 | SEQ ID NO : 1 |
| **2b** *(332-387)* | SEQ ID NO : 4 | SEQ ID NO : 3 |
| **2c** *(394-445)* | SEQ ID NO : 6 | SEQ ID NO : 5 |
| **1a** *(32-260)* | SEQ ID NO : 8 | SEQ ID NO : 7 |
| **1+3** *(1-260 + 516-628)* | SEQ ID NO : 10 | SEQ ID NO : 9 |
| **2+3** *(261-628)* | SEQ ID NO : 12 | SEQ ID NO : 11 |
| **1+2a** *(1-260 + 261-320)* | SEQ ID NO : 14 | SEQ ID NO : 13 |
| **1+2b** *(1-260 + 332-387)* | SEQ ID NO : 16 | SEQ ID NO : 15 |
| **1+2c** *(1-260 + 394-445)* | SEQ ID NO : 18 | SEQ ID NO : 17 |
| **1a+2a** *(32-260 + 261-320)* | SEQ ID NO : 20 | SEQ ID NO : 19 |
| **1a+2b** *(32-260 + 332-387)* | SEQ ID NO : 22 | SEQ ID NO : 21 |
| **1a+2c** *(32-260 + 394-445)* | SEQ ID NO : 24 | SEQ ID NO : 23 |
| **2a+2b** *(261-320 + 332-387)* | SEQ ID NO : 26 | SEQ ID NO : 25 |
| **2b+2c** *(332-387 + 394-445)* | SEQ ID NO : 28 | SEQ ID NO : 27 |
| **2a+2c** *(261-320 + 394-445)* | SEQ ID NO : 30 | SEQ ID NO : 29 |
| **2a+2b+2c** *(261-320 +332-387 + 394-445)* | SEQ ID NO : 32 | SEQ ID NO : 31 |
| **1+2a+2b** *(1-260 + 261-320 + 332-387)* | SEQ ID NO : 34 | SEQ ID NO : 33 |
| **1+2b+2c** *(1-260 + 332-387 + 394-445)* | SEQ ID NO : 36 | SEQ ID NO : 35 |
| **1+2a+2c** *(1-260 + 261-320 + 394-445)* | SEQ ID NO : 38 | SEQ ID NO : 37 |
| **1a+2a+2b** *(32-260 + 261-320 + 332-387)* | SEQ ID NO : 40 | SEQ ID NO : 39 |
| **1a+2b+2c** *(32-260* + *332-387* + *394-445)* | SEQ ID NO : 42 | SEQ ID NO : 41 |
| **1a+2a+2c** *(32-260* + *261-320* + *394-445)* | SEQ ID NO : 44 | SEQ ID NO : 43 |
| **1+2a+2b+2c** *(1-260* + *261-320* + *332-387* + *394-445)* | SEQ ID NO : 46 | SEQ ID NO : 45 |
| **1a+2a+2b+2c** *(32-260* + *261-320* + *332-387* + *394-445)* | SEQ ID NO : 48 | SEQ ID NO : 47 |
| **2a+3** *(261-320* + *516-628)* | SEQ ID NO : 50 | SEQ ID NO : 49 |
| **2b+3** *(332-387* + *516-628)* | SEQ ID NO : 52 | SEQ ID NO : 51 |
| **2c+3** *(394-445* + *516-628)* | SEQ ID NO : 54 | SEQ ID NO : 53 |
| **1a+3** *(32-260* + *516-628)* | SEQ ID NO : 56 | SEQ ID NO : 55 |
| **1a+2a+3** *(32-260* + *261-320* + *516-628)* | SEQ ID NO : 58 | SEQ ID NO : 57 |
| **1a+2b+3** *(32-260* + *332-387* + *516-628)* | SEQ ID NO : 60 | SEQ ID NO : 59 |
| **1a+2c+3** *(32-260* + *394-445* + *516-628)* | SEQ ID NO : 62 | SEQ ID NO : 61 |
| **1+2a+3** *(1-260* + *261-320* + *516-628)* | SEQ ID NO : 64 | SEQ ID NO : 63 |
| **1+2b+3** *(1-260* + *332-387* + *516-628)* | SEQ ID NO : 66 | SEQ ID NO : 65 |
| **1+2c+3** *(1-260* + *394-445* + *516-628)* | SEQ ID NO : 68 | SEQ ID NO : 67 |
| **2a+2b+3** *(261-320* + *332-387* + *516-628)* | SEQ ID NO : 70 | SEQ ID NO : 69 |
| **2a+2c+3** *(261-320* + *394-445* + *516-628)* | SEQ ID NO : 72 | SEQ ID NO : 71 |
| **2b+2c+3** *(332-387* + *394-445* + *516-628)* | SEQ ID NO : 74 | SEQ ID NO : 73 |
| **2a+2b+2c+3** *(261-320* + *332-387* + *394-445* + *516-628)* | SEQ ID NO : 76 | SEQ ID NO : 75 |
| **1+2a+2b+3** *(1-260* + *261-320* + *332-387* + *516-628)* | SEQ ID NO : 78 | SEQ ID NO : 77 |
| **1+2b+2c+3** *(1-260* + *332-387* + *394-445* + *516-628)* | SEQ ID NO : 80 | SEQ ID NO : 79 |
| **1+2a+2c+3** *(1-260* + *261-320* + *394-445* + *516-628)* | SEQ ID NO : 82 | SEQ ID NO : 81 |
| **1a+2a+2b+3** *(32-260* + *261-320* + *332-387* + *516-628)* | SEQ ID NO : 84 | SEQ ID NO : 83 |
| **1a+2b+2c+3** *(32-260 + 332-387 + 394-445 + 516-628)* | SEQ ID NO : 86 | SEQ ID NO : 85 |
| **1a+2a+2c+3** *(32-260 + 261-320 + 394-445 + 516-628)* | SEQ ID NO : 88 | SEQ ID NO : 87 |
| **1+2a+2b+2c+3** *(1-260 + 261-320 + 332-387 + 394-445 + 516-628)* | SEQ ID NO : 90 | SEQ ID NO : 89 |
| **1a+2a+2b+2c+3** *(32-260 + 261-320 + 332-387 + 394-445 + 516-628)* | SEQ ID NO : 92 | SEQ ID NO : 91 |
| **1 sans peptide signal** *(20-260)* | SEQ ID NO : 94 | SEQ ID NO : 93 |
| **1+2 sans peptide signal** *(20-516)* | SEQ ID NO : 96 | SEQ ID NO : 95 |
| **1+3 sans peptide signal** *(20-260 + 516-628)* | SEQ ID NO : 98 | SEQ ID NO : 97 |
| **1+2a sans peptide signal** *(20-260 + 261-320)* | SEQ ID NO : 100 | SEQ ID NO : 99 |
| **1+2b sans peptide signal** *(20-260 + 332-387)* | SEQ ID NO : 102 | SEQ ID NO : 101 |
| **1+2c sans peptide signal** *(20-260* + *394-445)* | SEQ ID NO : 104 | SEQ ID NO : 103 |
| **1+2a+2b sans peptide signal** *(20-260* + *261-320* + *332-387)* | SEQ ID NO : 106 | SEQ ID NO : 105 |
| **1+2b+2c sans peptide signal** *(20-260* + *332-387* + *394-445)* | SEQ ID NO : 108 | SEQ ID NO : 107 |
| **1+2a+2c sans peptide signal** *(20-260* + *261-320* + *394-445)* | SEQ ID NO : 110 | SEQ ID NO : 109 |
| **1+2a+2b+2c sans peptide signal** *(20-260* + *261-320 +332-387* + *394-445)* | SEQ ID NO : 112 | SEQ ID NO : 111 |
| **1+2a+3 sans peptide signal** *(20-260* + *261-320* + *516-628)* | SEQ ID NO : 114 | SEQ ID NO : 113 |
| **1+2b+3 sans peptide signal** *(20-260* + *332-387* + *516-628)* | SEQ ID NO : 116 | SEQ ID NO : 115 |
| **1+2c+3 sans peptide signal** *(20-260* + *394-445* + *516-628)* | SEQ ID N0 118 | SEQ ID N0 117 |
| **1+2a+2b+3 sans peptide signal** *(20-260* + *261-320* + *332-387* + *516-628)* | SEQ ID NO : 120 | SEQ ID NO : 119 |
| **1+2b+2c+3 sans peptide signal** *(20-260* + *332-387* + *394-445* + *516-628)* | SEQ ID NO : 122 | SEQ ID NO : 121 |
| **1+2a+2c+3 sans peptide signal** *(20-260* + *261-320* + *394-445* + *516-628)* | SEQ ID NO : 124 | SEQ ID NO : 123 |
| **1+2a+2b+2c+3 sans peptide signal** *(20-260* + *261-320* + *332-387* + *394-445* + *516-628)* | SEQ ID NO : 126 | SEQ ID NO : 125 |

| **Fragments de nétrine 1** | Séquence protéique | Séquence nucléotidique |
|---|---|---|
| **1** *(1-283)* | SEQ ID NO : 128 | SEQ ID NO : 127 |
| **2** *(284-487)* | SEQ ID NO : 130 | SEQ ID NO : 129 |
| **3** *(488-604)* | SEQ ID NO : 132 | SEQ ID NO : 131 |
| **1+2** *(1-487)* | SEQ ID NO : 372 | SEQ ID NO : 371 |
| **2a** *(284-331)* | SEQ ID NO : 134 | SEQ ID NO : 133 |
| **2b** *(341-396)* | SEQ ID NO : 136 | SEQ ID NO : 135 |
| **2c** *(403-450)* | SEQ ID NO : 138 | SEQ ID NO : 137 |
| **1a** *(49-283)* | SEQ ID NO : 140 | SEQ ID NO : 139 |
| **1+3** *(1-283* + *488-604)* | SEQ ID NO : 142 | SEQ ID NO : 141 |
| **2+3** *(284-604)* | SEQ ID NO : 144 | SEQ ID NO : 143 |
| **1a+2** *(49-487)* | SEQ ID NO : 146 | SEQ ID NO : 145 |
| **1a+2+3** *(49-604)* | SEQ ID NO : 148 | SEQ ID NO : 147 |
| **1+2a** *(1-283* + *284-331)* | SEQ ID NO : 150 | SEQ ID NO : 149 |
| **1+2b** *(1-283* + *341-396)* | SEQ ID NO : 152 | SEQ ID NO : 151 |
| **1+2c** *(1-283* + *403-450)* | SEQ ID NO : 154 | SEQ ID NO : 153 |
| **1a+2a** *(49-283* + *284-331)* | SEQ ID NO : 156 | SEQ ID NO : 155 |
| **1a+2b** *(49-283* + *341-396)* | SEQ ID NO : 158 | SEQ ID NO : 157 |
| **1a+2c** *(49-283* + *403-450)* | SEQ ID NO : 160 | SEQ ID NO : 159 |
| **2a+2b** *(284-331* + *341-396)* | SEQ ID NO : 162 | SEQ ID NO : 161 |
| **2b+2c** *(341-396* + *403-450)* | SEQ ID NO : 164 | SEQ ID NO : 163 |
| **2a+2c** *(284-331* + *403-450)* | SEQ ID NO : 166 | SEQ ID NO : 165 |
| **2a+2b+2c** *(284-331 + 341-396* + *403-450)* | SEQ ID NO : 168 | SEQ ID NO : 167 |
| **1+2a+2b** *(1-283* + *284-331* + *341-396)* | SEQ ID NO : 170 | SEQ ID NO : 169 |
| **1+2b+2c** *(1-283* + *341-396 403-450)* | SEQ ID NO : 172 | SEQ ID NO : 171 |
| **1+2a+2c** *(1-283* + *284-331* + *403-450)* | SEQ ID NO : 174 | SEQ ID NO : 173 |
| **1a+2a+2b** *(49-283* + *284-331* + *341-396)* | SEQ ID NO : 176 | SEQ ID NO : 175 |
| **1a+2b+2c** *(49-283* + *341-396* + *403-450)* | SEQ ID NO : 178 | SEQ ID NO : 177 |
| **1a+2a+2c** *(49-283* + *284-331* + *403-450)* | SEQ ID NO : 180 | SEQ ID NO : 179 |
| **1+2a+2b+2c** *(1-283 + 284-331 + 341-396 + 403-450)* | SEQ ID NO : 182 | SEQ ID NO : 181 |
| **1a+2a+2b+2c** *(49-283 + 284-331 + 341-396 + 403-450)* | SEQ ID NO : 184 | SEQ ID NO : 183 |
| **2a+3** *(284-331 + 488-604)* | SEQ ID NO : 186 | SEQ ID NO : 185 |
| **2b+3** *(341-396 + 488-604)* | SEQ ID NO : 188 | SEQ ID NO : 187 |
| **2c+3** *(403-450 + 488-604)* | SEQ ID NO : 190 | SEQ ID NO : 189 |
| **1a+3** *(49-283 + 488-604)* | SEQ ID NO : 192 | SEQ ID NO : 191 |
| **1a+2a+3** *(49-283* + *284-331* + *488-604)* | SEQ ID NO : 194 | SEQ ID NO : 193 |
| **1a+2b+3** *(49-283* + *341-396* + *488-604)* | SEQ ID NO : 196 | SEQ ID NO : 195 |
| **1a+2c+3** *(49-283* + *403-450* + *488-604)* | SEQ ID NO : 198 | SEQ ID NO : 197 |
| **1+2a+3** *(1-283* + *284-331* + *488-604)* | SEQ ID NO : 200 | SEQ ID NO : 199 |
| **1+2b+3** *(1-283* + *341-396* + *488-604)* | SEQ ID NO : 202 | SEQ ID NO : 201 |
| **1+2c+3** *(1-283* + *403-450* + *488-604)* | SEQ ID NO : 204 | SEQ ID NO : 203 |
| **2a+2b+3** *(284-331* + *341-396* + *488-604)* | SEQ ID NO : 206 | SEQ ID NO : 205 |
| **2a+2c+3** *(284-331* + *403-450* + *488-604)* | SEQ ID NO : 208 | SEQ ID NO : 207 |
| **2b+2c+3** *(341-396* + *403-450* + *488-604)* | SEQ ID NO : 210 | SEQ ID NO : 209 |
| **2a+2b+2c+3** *(284-331 + 341-396 + 403-450 + 488-604)* | SEQ ID NO : 212 | SEQ ID NO : 211 |
| **1+2a+2b+3** *(1-283 + 284-331 + 341-396 + 488-604)* | SEQ ID NO : 214 | SEQ ID NO : 213 |
| **1+2b+2c+3** *(1-283 + 341-396 + 403-450 + 488-604)* | SEQ ID NO : 216 | SEQ ID NO : 215 |
| **1+2a+2c+3** *(1-283 + 284-331 + 403-450 + 488-604)* | SEQ ID NO : 218 | SEQ ID NO : 217 |
| **1a+2a+2b+3** *(49-283 + 284-331 + 341-396 + 488-604)* | SEQ ID NO : 220 | SEQ ID NO : 219 |
| **1a+2b+2c+3** *(49-283 + 341-396 + 403-450 + 488-604)* | SEQ ID NO : 222 | SEQ ID NO : 221 |
| **1a+2a+2c+3** *(49-283 + 284-331 + 403-450 + 488-604)* | SEQ ID NO : 224 | SEQ ID NO : 223 |
| **1+2a+2b+2c+3** *(1-283 + 284-331 + 341-396 + 403-450 + 488-604)* | SEQ ID NO : 226 | SEQ ID NO : 225 |
| **1a+2a+2b+2c+3** *(49-283 + 284-331 + 341-396 + 403-450 + 488-604)* | SEQ ID NO : 228 | SEQ ID NO : 227 |
| **1 sans peptide signal** *(25-283)* | SEQ ID NO : 230 | SEQ ID NO : 229 |
| **1+2 sans peptide signal** *(25-487)* | SEQ ID NO : 232 | SEQ ID NO : 231 |
| **1+3 sans peptide signal** *(25-283 + 488-604)* | SEQ ID NO : 234 | SEQ ID NO : 233 |
| **1+2a sans peptide signal** *(25-283 + 284-331)* | SEQ ID NO : 236 | SEQ ID NO : 235 |
| **1+2b sans peptide signal** *(25-283 + 341-396)* | SEQ ID NO : 238 | SEQ ID NO : 237 |
| **1+2c sans peptide signal** *(25-283 + 403-450)* | SEQ ID NO : 240 | SEQ ID NO : 239 |
| **1+2a+2b sans peptide signal** *(25-283 + 284-331 + 341-396)* | SEQ ID NO : 242 | SEQ ID NO : 241 |
| **1+2b+2c sans peptide signal** *(25-283 + 341-396 + 403-450)* | SEQ ID NO : 244 | SEQ ID NO : 243 |
| **1+2a+2c sans peptide signal** *(25-283 + 284-331 + 403-450)* | SEQ ID NO : 246 | SEQ ID NO : 245 |
| **1+2a+2b+2c sans peptide signal** *(25-283 + 284-331 + 341-396 + 403-450)* | SEQ ID NO : 248 | SEQ ID NO : 247 |
| **1+2a+3 sans peptide signal** *(25-283 + 284-331 + 488-604)* | SEQ ID NO : 250 | SEQ ID NO : 249 |
| **1+2b+3 sans peptide signal** *(25-283 + 341-396 + 488-604)* | SEQ ID NO : 252 | SEQ ID NO : 251 |
| **1+2c+3 sans peptide signal** *(25-283 + 403-450 + 488-604)* | SEQ ID NO : 254 | SEQ ID NO : 253 |
| **1+2a+2b+3 sans peptide signal** *(25-283 + 284-331 + 341-396 + 488-604)* | SEQ ID NO : 256 | SEQ ID NO : 255 |
| **1+2b+2c+3 sans peptide signal** *(25-283 + 341-396 + 403-450 + 488-604)* | SEQ ID NO : 258 | SEQ ID NO : 257 |
| **1+2a+2c+3 sans peptide signal** *(25-283 + 284-331 + 403-450 + 488-604)* | SEQ ID NO : 260 | SEQ ID NO : 259 |
| **1+2a+2b+2c+3 sans peptide signal** *(25-283 + 284-331 + 341-396 + 403-450 + 488-604)* | SEQ ID NO : 262 | SEQ ID NO : 261 |

| **Fragments de nétrine G1** | Séquence protéique | Séquence nucléotidique |
|---|---|---|
| **1** *(1-295)* | SEQ ID NO : 264 | SEQ ID NO : 263 |
| **2** *(296-438)* | SEQ ID NO : 266 | SEQ ID NO : 265 |
| **1a** *(71-295)* | SEQ ID NO : 268 | SEQ ID NO : 267 |
| **2a** *(296-342)* | SEQ ID NO : 270 | SEQ ID NO : 269 |
| **2b** *(373-409)* | SEQ ID NO : 272 | SEQ ID NO : 271 |
| **1+2a** *(1-295 + 296-342)* | SEQ ID NO : 274 | SEQ ID NO : 273 |
| **1+2b** *(1-295 + 373-409)* | SEQ ID NO : 276 | SEQ ID NO : 275 |
| **1+2a+2b** *(1-295 + 296-342 + 373-409)* | SEQ ID NO : 278 | SEQ ID NO : 277 |
| 1**a+2** *(71-295 + 296-438)* | SEQ ID NO : 280 | SEQ ID NO : 279 |
| **1a+2a** *(71-295 + 296-342)* | SEQ ID NO : 282 | SEQ ID NO : 281 |
| **1a+2b** *(71-295 + 373-409)* | SEQ ID NO : 284 | SEQ ID NO : 283 |
| **2a+2b** *(296-342 + 373-409)* | SEQ ID NO : 286 | SEQ ID NO : 285 |
| **1a+2a+2b** *(71-295 + 296-342 + 373-409)* | SEQ ID NO : 288 | SEQ ID NO : 287 |
| **1 sans peptide signal** (29-295) | SEQ ID NO : 290 | SEQ ID NO : 289 |
| **1+2a sans peptide signal** *(29-295 + 296-342)* | SEQ ID NO : 292 | SEQ ID NO : 291 |
| **1+2b sans peptide signal** *(29-295 + 373-409)* | SEQ ID NO : 294 | SEQ ID NO : 293 |
| **1+2a+2b sans peptide signal** *(29-295 + 296-342 + 373-409)* | SEQ ID NO : 296 | SEQ ID NO : 295 |

| **Fragments de nétrine 3** | Séquence protéique | Séquence nucléotidique |
|---|---|---|
| **1** *(1-253)* | SEQ ID NO : 298 | SEQ ID NO : 297 |
| **1a** *(34-253)* | SEQ ID NO : 300 | SEQ ID NO : 299 |
| **2** *(254-433)* | SEQ ID NO : 302 | SEQ ID NO : 301 |
| **2a** *(254-299)* | SEQ ID NO : 304 | SEQ ID NO : 303 |
| **2b** *(373-422)* | SEQ ID NO : 306 | SEQ ID NO : 305 |
| **3** *(433-580)* | SEQ ID NO : 308 | SEQ ID NO : 307 |
| **1+2** *(1-422)* | SEQ ID NO : 310 0 | SEQ ID NO : 309 |
| **2+3** *(254-433)* | SEQ ID NO : 312 | SEQ ID NO : 311 1 |
| **1+3** *(1-253 + 433-580)* | SEQ ID NO : 314 | SEQ ID NO : 313 3 |
| **1+2a** *(1-299)* | SEQ ID NO : 316 | SEQ ID NO : 315 |
| **1+2b** *(1-253 + 373-422)* | SEQ ID NO : 318 | SEQ ID NO : 317 7 |
| **1+2a+2b** *(1-253 + 254-299 + 373-422)* | SEQ ID NO : 320 | SEQ ID NO : 319 |
| **2a+3** *(254-299 + 433-580)* | SEQ ID NO : 322 | SEQ ID NO : 321 |
| **2b+3** *(373-422 + 433-580)* | SEQ ID NO : 324 | SEQ ID NO : 323 |
| **2a+2b** *(254-299 + 373-422)* | SEQ ID NO : 326 | SEQ ID NO : 325 |
| **2a+2b+3** *(254-299 + 373-422 + 433-580)* | SEQ ID NO : 328 | SEQ ID NO : 327 |
| **1+2a+3** *(1-253 + 254-299 + 433-580)* | SEQ ID NO : 330 | SEQ ID NO : 329 |
| **1+2b+3** *(1-253 + 373-422 + 433-580)* | SEQ ID NO : 332 | SEQ ID NO : 331 1 |
| **1+2a+2b+3** *(1-253 + 254-299 + 373-422 + 433-580)* | SEQ ID NO : 334 | SEQ ID NO : 333 |
| **1a+2** *(34-253 + 254-433)* | SEQ ID NO : 336 | SEQ ID NO : 335 |
| **1a+3** *(34-253 + 433-580)* | SEQ ID NO : 338 | SEQ ID NO : 337 |
| **1a+2a** *(34-253 + 254-299)* | SEQ ID NO : 340 | SEQ ID NO : 339 |
| **1+2b** *(34-253 + 373-422)* | SEQ ID NO : 342 | SEQ ID NO : 341 |
| **1a+2+3** *34-253 + 254-433 + 433-580)* | SEQ ID NO : 344 | SEQ ID NO : 343 |
| **1a+2a+2b** *(34-253 + 254-299 + 373-422)* | SEQ ID NO : 346 | SEQ ID NO : 345 |
| **1a+2a+3** *(34-253 + 254-299 + 433-580)* | SEQ ID NO : 348 | SEQ ID NO : 347 |
| **1a+2b+3** *(34-253 + 373-422 + 433-580)* | SEQ ID NO : 350 | SEQ ID NO : 349 |
| **1a+2a+2b+3** *(34-253 + 254-299 + 373-422 + 433-580)* | SEQ ID NO : 352 | SEQ ID NO : 351 |

| **Fragments de RGM** | Séquence protéique | Séquence nucléotidique |
|---|---|---|
| **RGMA** *(180-290)* | SEQ ID NO : 354 | SEQ ID NO : 353 |
| **RGMB** *(180-290)* | SEQ ID NO : 356 | SEQ ID NO : 355 |
| **RGMC** *(180-290)* | SEQ ID NO : 358 | SEQ ID NO : 357 |

| **Fragments de nétrine 4 mutée** | Séquence protéique | Séquence nucléotidique |
|---|---|---|
| **1** *(1-260)* | SEQ ID NO : 374 | SEQ ID NO : 373 |
| **2** *(261-515)* | SEQ ID NO : 376 | SEQ ID NO : 375 |
| **1+2** *(1-515)* | SEQ ID NO : 378 | SEQ ID NO : 377 |
| **1a+2** *(32-515)* | SEQ ID NO : 380 | SEQ ID NO : 379 |
| **1a+2+3** *(32-628)* | SEQ ID NO : 382 | SEQ ID NO : 381 1 |
| **2b** *(332-387)* | SEQ ID NO : 384 | SEQ ID NO : 383 |
| **1a** *(32-260)* | SEQ ID NO : 386 | SEQ ID NO : 385 |
| **1+3** *(1-260* + *516-628)* | SEQ ID NO : 388 | SEQ ID NO : 387 |
| **2+3** *(261-628)* | SEQ ID NO : 390 | SEQ ID NO : 389 |
| **1+2a** *(1-260* + *261-320)* | SEQ ID NO : 392 | SEQ ID NO : 391 |
| **1+2b** *(1-260* + *332-387)* | SEQ ID NO : 394 | SEQ ID NO : 393 |
| **1+2c** *(1-260* + *394-445)* | SEQ ID NO : 396 | SEQ ID NO : 395 |
| **1a+2a** *(32-260* + *261-320)* | SEQ ID NO : 398 | SEQ ID NO : 397 |
| **1a+2b** *(32-260* + *332-387)* | SEQ ID NO : 400 | SEQ ID NO : 399 |
| **1a+2c** *(32-260* + *394-445)* | SEQ ID NO : 402 | SEQ ID NO : 401 |
| **2a+2b** *(261-320* + *332-387)* | SEQ ID NO : 404 | SEQ ID NO : 403 |
| **2b+2c** *(332-387 + 394-445)* | SEQ ID NO : 406 | SEQ ID NO : 405 |
| **2a+2b+2c** *(261-320* + *332-387 + 394-445)* | SEQ ID NO : 408 | SEQ ID NO : 407 |
| **1+2a+2b** *(1-260* + *261-320* + *332-387)* | SEQ ID NO : 410 | SEQ ID NO : 409 |
| **1+2b+2c** *(1-260* + *332-387 + 394-445)* | SEQ ID NO : 412 | SEQ ID NO : 411 |
| **1+2a+2c** *(1-260* + *261-320* + *394-445)* | SEQ ID NO : 414 | SEQ ID NO : 413 |
| **1a+2a+2b** *(32-260* + *261-320* + *332-387)* | SEQ ID NO : 416 | SEQ ID NO : 415 |
| **1a+2b+2c** *(32-260* + *332-387 + 394-445)* | SEQ ID NO : 418 | SEQ ID NO : 417 |
| **1a+2a+2c** *(32-260* + *261-320* + *394-445)* | SEQ ID NO : 420 | SEQ ID NO : 419 |
| **1+2a+2b+2c** *(1-260 + 261-320 +332-387 + 394-445)* | SEQ ID NO : 422 | SEQ ID NO : 421 |
| **1a+2a+2b+2c** *(32-260* + *261-320* + *332-387* + *394-445)* | SEQ ID NO : 424 | SEQ ID NO : 423 |
| **2b+3** *(332-387* + *516-628)* | SEQ ID NO : 426 | SEQ ID NO : 425 |
| **1a+3** *(32-260* + *516-628)* | SEQ ID NO : 428 | SEQ ID NO : 427 |
| **1a+2a+3** *(32-260* + *261-320* + *516-628)* | SEQ ID NO : 430 | SEQ ID NO : 429 |
| **1a+2b+3** *(32-260* + *332-387* + *516-628)* | SEQ ID NO : 432 | SEQ ID NO : 431 |
| **1a+2c+3** *(32-260* + *394-445* + *516-628)* | SEQ ID NO : 434 | SEQ ID NO : 433 |
| **1+2a+3** *(1-260* + *261-320* + *516-628)* | SEQ ID NO : 436 | SEQ ID NO : 435 |
| **1+2b+3** *(1-260* + *332-387* + *516-628)* | SEQ ID NO : 438 | SEQ ID NO : 437 |
| **1+2c+3** *(1-260* + 394-445 + *516-628)* | SEQ ID NO : 440 | SEQ ID NO : 439 |
| **2a+2b+3** *(261-320* + *332-387* + *516-628)* | SEQ ID NO : 442 | SEQ ID NO : 441 |
| **2b+2c+3** *(332-387* + *394-445* + *516-628)* | SEQ ID NO : 444 | SEQ ID NO : 443 |
| **2a+2b+2c+3** *(261-320* + *332-387* + *394-445* + *516-628)* | SEQ ID NO : 446 | SEQ ID NO : 445 |
| **1+2a+2b+3** *(1-260* + *261-320* + *332-387* + *516-628)* | SEQ ID NO : 448 | SEQ ID NO : 447 |
| **1+2b+2c+3** *(1-260 + 332-387 + 394-445 + 516-628)* | SEQ ID NO : 450 | SEQ ID NO : 449 |
| **1+2a+2c+3** *(1-260* + *261-320* + *394-445* + *516-628)* | SEQ ID NO : 452 | SEQ ID NO : 451 1 |
| **1a+2a+2b+3** *(32-260* + *261-320* + *332-387* + *516-628)* | SEQ ID NO : 454 | SEQ ID NO : 453 |
| **1a+2b+2c+3** *(32-260* + *332-387* + *394-445* + *516-628)* | SEQ ID NO : 456 | SEQ ID NO : 455 |
| **1a+2a+2c+3** *(32-260* + *261-320* + *394-445* + *516-628)* | SEQ ID NO : 458 | SEQ ID NO : 457 |
| **1+2a+2b+2c+3** *(1-260* + *261-320* + *332-387* + *394-445* + *516-628)* | SEQ ID NO : 460 | SEQ ID NO : 459 |
| **1a+2a+2b+2c+3** *(32-260* + *261-320* + *332-387* + *394-445* + *516-628)* | SEQ ID NO : 462 | SEQ ID NO : 461 |
| **1 sans peptide signal** *(20-260)* | SEQ ID NO : 464 | SEQ ID NO : 463 |
| **1+2 sans peptide signal** *(20-516)* | SEQ ID NO : 466 | SEQ ID NO : 465 |
| **1+3 sans peptide signal** *(20-260* + *516-628)* | SEQ ID NO : 468 | SEQ ID NO : 467 |
| **1+2a sans peptide signal** *(20-260* + *261-320)* | SEQ ID NO : 470 | SEQ ID NO : 469 |
| **1+2b sans peptide signal** *(20-260 + 332-387)* | SEQ ID NO : 472 | SEQ ID NO : 471 |
| **1+2c sans peptide signal** *(20-260 + 394-445)* | SEQ ID NO : 474 | SEQ ID NO : 473 |
| **1+2a+2b sans peptide signal** *(20-260 + 261-320 + 332-387)* | SEQ ID NO : 476 | SEQ ID NO : 475 |
| **1+2b+2c sans peptide signal** *(20-260 + 332-387 + 394-445)* | SEQ ID NO : 478 | SEQ ID NO : 477 |
| **1+2a+2c sans peptide signal** *(20-260 + 261-320 + 394-445)* | SEQ ID NO : 480 | SEQ ID NO : 479 |
| **1+2a+2b+2c sans peptide signal** *(20-260 + 261-320 +332-387 + 394-445)* | SEQ ID NO : 482 | SEQ ID NO : 481 1 |
| **1+2a+3 sans peptide signal** *(20-260 + 261-320 + 516-628)* | SEQ ID NO : 484 | SEQ ID NO : 483 |
| **1+2b+3 sans peptide signal** *(20-260 + 332-387 + 516-628)* | SEQ ID NO : 486 | SEQ ID NO : 485 |
| **1+2c+3 sans peptide signal** *(20-260 + 394-445 + 516-628)* | SEQ ID NO : 488 | SEQ ID NO : 487 |
| **1+2a+2b+3 sans peptide signal** *(20-260 + 261-320 + 332-387 + 516-628)* | SEQ ID NO : 490 | SEQ ID NO : 489 |
| **1+2b+2c+3 sans peptide signal** *(20-260 + 332-387 + 394-445 + 516-628)* | SEQ ID NO : 492 | SEQ ID NO : 491 |
| **1+2a+2c+3 sans peptide signal** *(20-260 + 261-320 + 394-445 + 516-628)* | SEQ ID NO : 494 | SEQ ID NO : 493 |
| **1+2a+2b+2c+3 sans peptide signal** *(20-260 + 261-320 + 332-387 + 394-445 + 516-628)* | SEQ ID NO : 496 | SEQ ID NO : 495 |

### PARTIE EXPÉRIMENTALE

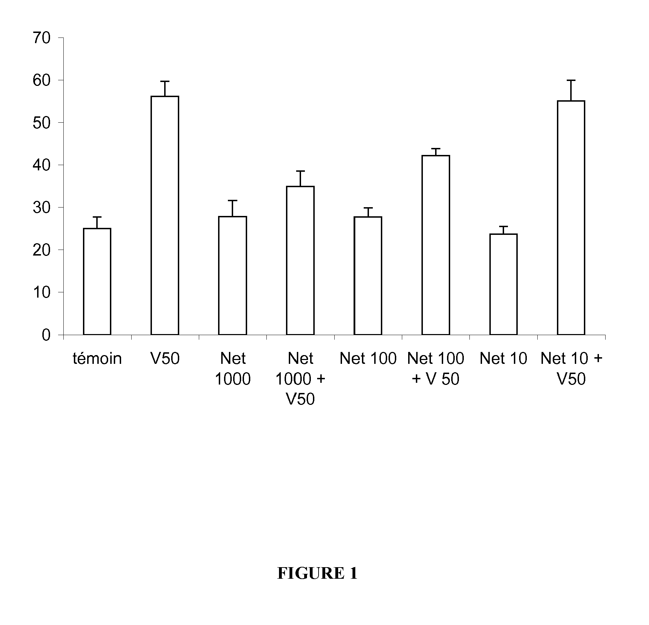

La Figure 1 correspond au test de migration des cellules HUAEC. Les cellules sont comptées dans 8 champs et la moyenne et l'écart-type sont représentés sur l'axe des ordonnées. L'axe des abscisses correspond à la concentration de la protéine nétrine 4 en ng/ml en présence (V 50) ou absence de 50 ng/ml de VEGF (facteur de croissance endothéliale vasculaire).

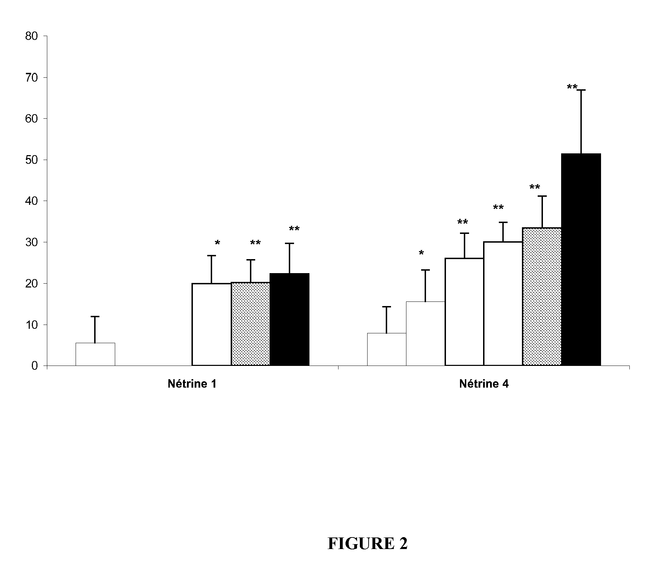

La Figure 2 correspond au test de migration des cellules musculaires lisses d'aorte. Les cellules sont comptées dans 8 champs et la moyenne et l'écart-type sont représentés sur l'axe des ordonnées. L'axe des abscisses correspond à la concentration des protéines nétrine 1 et nétrine 4 en ng/ml. Les colonnes blanches correspondent à une concentration de 0 ng/ml ; les colonnes avec les hachures verticales correspondent à une concentration de 6 ng/ml ; les colonnes avec les hachures horizontales correspondent à une concentration de 18 ng/ml ; les colonnes avec les hachures obliques correspondent à une concentration de 55 ng/ml ; les colonnes grises correspondent à une concentration de 160 ng/ml et les colonne noires correspondent à une concentration de 500 ng/ml.

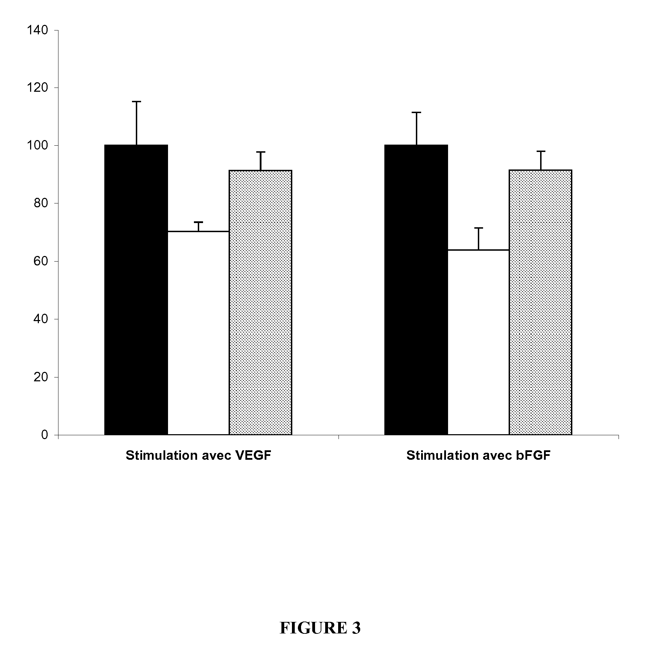

La Figure 3 correspond au test de prolifération des cellules HUAEC. L'axe des abscisses correspond à la concentration de la protéine nétrine 4 (500 ng/ml) en présence de 2 ng/ml de VEGF ou FGF2 comme indiqué sur la figure et l'axe des ordonnées au pourcentage de prolifération. Les colonnes noires correspondent au témoin ; les colonnes blanches correspondent au pourcentage de prolifération en présence de 500 ng/ml de nétrine 4 et les colonnes grises correspondent au pourcentage de prolifération en présence de 500 ng/ml de nétrine 4 et de 5 µg/ml d'IgG dirigées contre le récepteur néogénine.

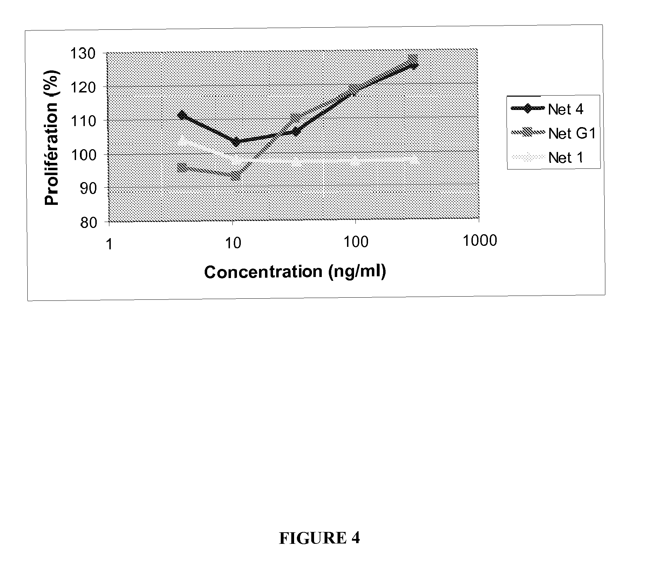

La Figure 4 correspond à l'analyse de l'effet de la nétrine 4 (NET-4), de la nétrine 1 (NET-1) et de la nétrine G1 (NET-G1) sur la prolifération des VSM. La courbe représentée par des losanges correspond à la nétrine 4. La courbe représentée par des carrés correspond à la nétrine G1. La courbe représentée par des triangles correspond à la nétrine 1.

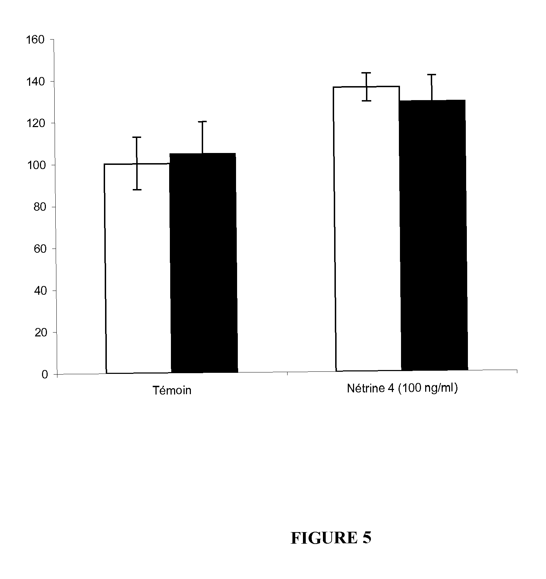

La Figure 5 représente l'effet d'un anticorps anti-néogénine sur la prolifération des VSM. L'axe des ordonnées représente la densité optique à 595 nm. Les colonnes blanches correspondent à la prolifération sans anticorps anti-néogénine et les colonnes noires correspondent à la prolifération en présence de 5 µg/ml d'anticorps anti-néogénine.

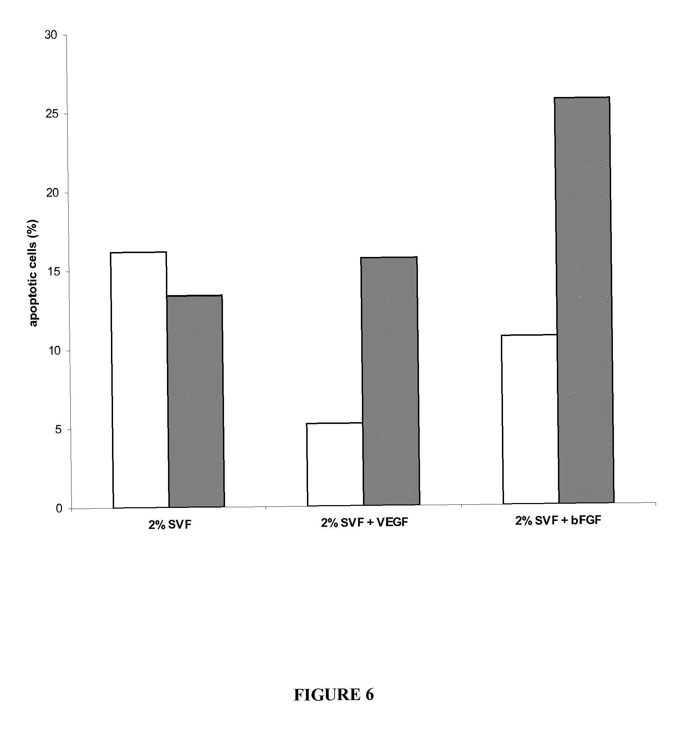

La Figure 6 correspond au test d'apoptose des cellules HUAEC. L'axe des abscisses correspond à la concentration de la protéine nétrine 4 (500 ng/ml) en présence de 50 ng/ml de VEGF ou FGF2 comme indiqué sur la figure et l'axe des ordonnées au pourcentage de cellules apoptotiques. Les colonnes blanches correspondent au témoin sans nétrine 4 ; les colonnes grises correspondent au pourcentage d'apoptose en présence de 500 ng/ml de nétrine 4.

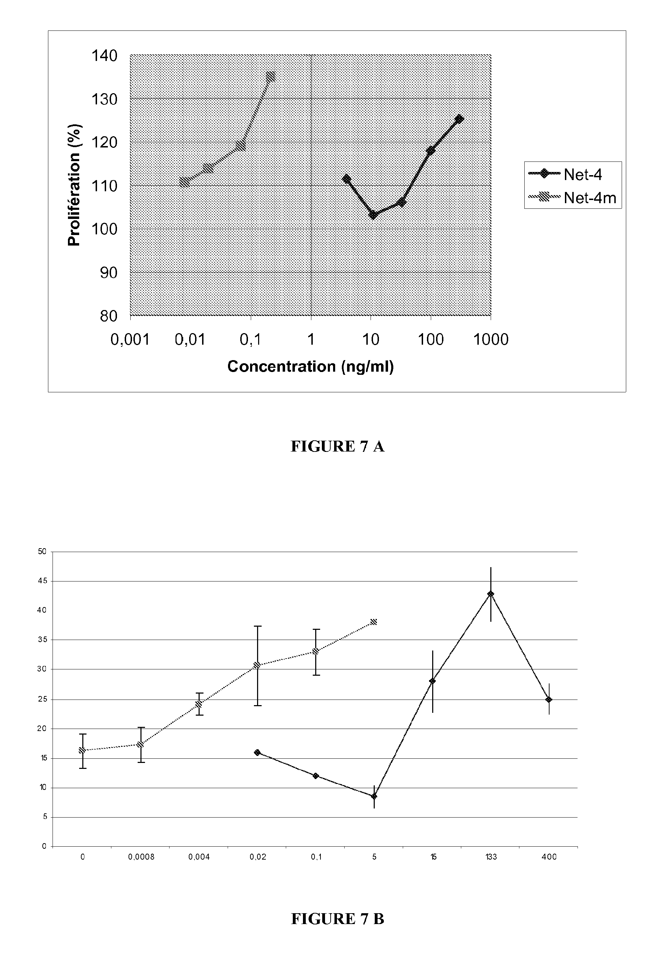

La Figure 7A correspond au test de prolifération des cellules musculaires lisses d'aorte. L'axe des abscisses correspond à la concentration en ng/ml de la protéine nétrine 4 ou de la protéine nétrine 4 mutée et l'axe des ordonnées au pourcentage de prolifération. La courbe de gauche représentée par des carrés correspond à la nétrine 4 mutée. La courbe de droite représentée par des losanges correspond à la nétrine 4.
La Figure 7B correspond au test de migration des cellules musculaires lisses d'aorte. Les cellules sont comptées dans 8 champs et la moyenne et l'écart-type sont représentés sur l'axe des ordonnées. L'axe des abscisses correspond à la concentration des protéines nétrine 4 et nétrine 4 mutée en ng/ml. La courbe représentée par des carrés correspond à la nétrine 4 mutée. La courbe représentée par des losanges correspond à la nétrine 4.

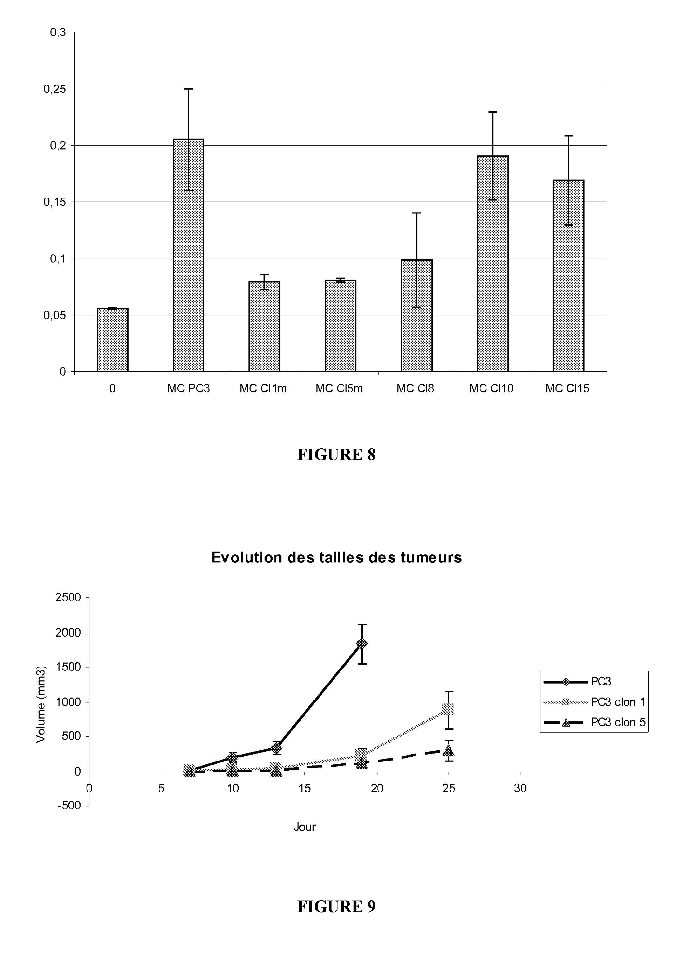

La Figure 8 correspond à un test de prolifération de cellules HUAEC afin de déterminer l'effet des surnageants des cellules PC3 transfectées avec la nétrine 4 mutée (clone 1 et clone 5) ou non mutée (clone 8, 10 et 15). L'axe des ordonnées représente le pourcentage de prolifération. La colonne 1 correspond au témoin (DMEM seul) ; la colonne 2 correspond aux cellules PC3 non transfectées ; la colonne 3 correspond au clone 1 ; la colonne 4 correspond au clone 5 ; la colonne 5 correspond au clone 8 ; la colonne 6 correspond au clone 10 ; la colonne 7 correspond au clone 15.
La Figure 9 correspond à une analyse de la progression tumorale. L'axe des abscisses correspond au temps exprimé en jours, j = 0 étant le jour où la greffe tumorale est pratiquée. L'axe des ordonnées correspond au volume des tumeurs (en mm³). La courbe comportant les losanges correspond aux cellules PC3 non transfectées. La courbe comportant les carrés correspond aux cellules PC3 transfectées avec le clone 1. La courbe comportant les triangles correspond aux cellules PC3 transfectées avec le clone 5.

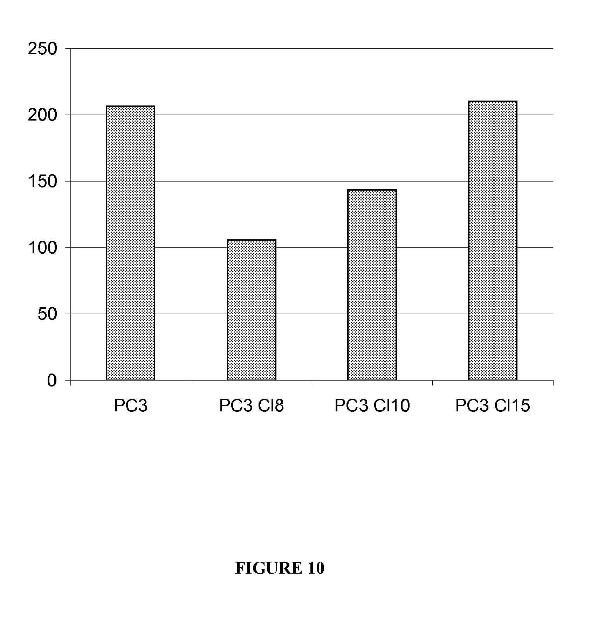

La Figure 10 correspond à une analyse de la progression tumorale en présence d'AVASTIN. L'axe des ordonnées correspond au rapport du volume tumoral mesuré après le traitement sur le volume tumoral mesuré avant le traitement. La colonne 1 correspond aux cellules PC3 non transfectées ; la colonne 2 correspond au clone 8 de PC3 ; la colonne 3 correspond au clone 10 de PC3 ; la colonne 4 correspond au clone 15 5 de PC3.

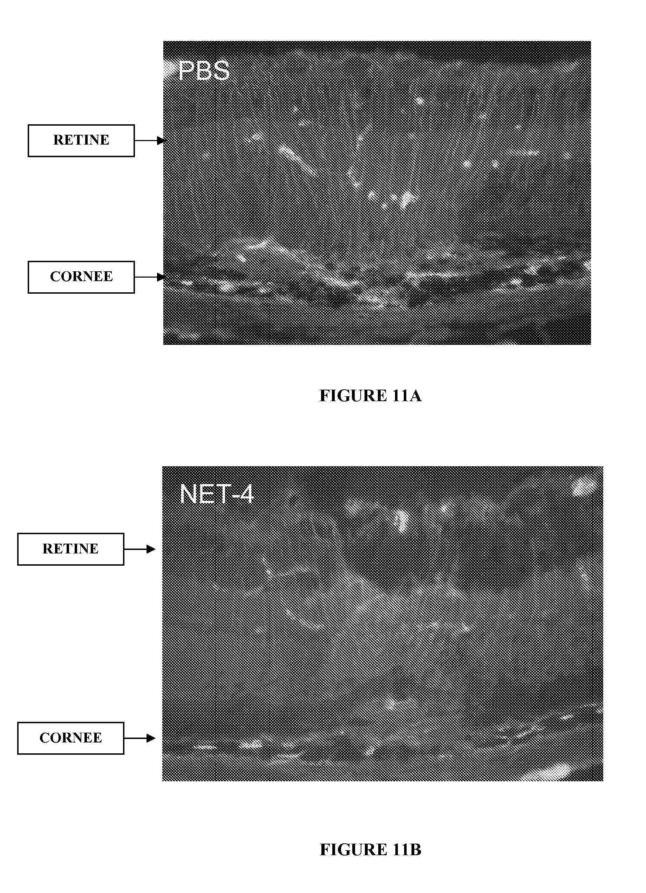

Les Figures 11A et 11B correspondent à une analyse de l'effet de la nétrine 4 sur la vascularisation choroïdienne chez la souris. Dans la figure 11A, les souris ont reçu une injection de 1 µl de PBS. Dans la figure 11B, les souris ont reçu une injection de1 µl de PBS contenant 1µg de nétrine 4. Les cellules endothéliales sont révélées par immunomarquage et apparaissent en blanc sur les figures 11A et 11B.

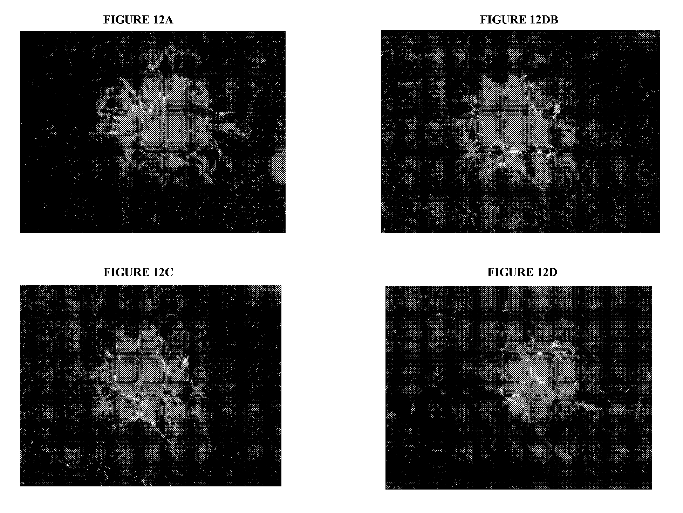

Les Figures 12A, 12B, 12C correspondent à la perfusion d'un dextran fluorescent qui visualise les néovaisseaux choroïdiens autour d'un impact laser en gris au centre. Les rats ont reçu deux ou trois impacts laser à J (jours)=0, puis à J=7, J=10 une injection sous rétinienne du véhicule seul (PBS) (12 A) ou contenant de la nétrine 1 (12 B) ou 2 µg de nétrine 4 ( 12 C) ou 100 pg de nétrine 4 mutée (12 D). A J=14, les rats sont sacrifiés et perfusés par la lectine fluorescente qui permet de visualiser les néovaisseaux choroïdiens apparaissant en blancs sur les figures et entourant un impact laser en gris.

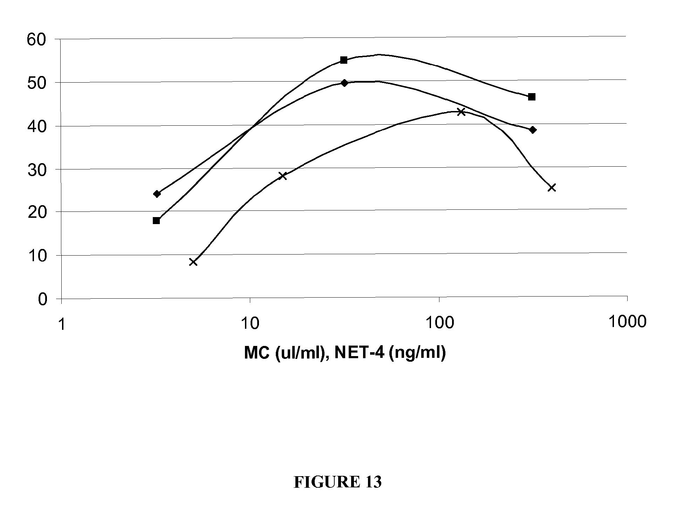

La Figure 13 correspond au test de migration des cellules musculaires lisses d'aorte en présence de mutants de délétion de la nétrine 4 mutée. Les cellules sont comptées dans 8 champs et la moyenne et l'écart-type sont représentés sur l'axe des ordonnées. L'axe des abscisses correspond à la concentration de la nétrine 4 en ng/ml.

La courbe avec les croix correspond à la nétrine 4 non mutée. La courbe avec les losanges correspond au milieu conditionné des cellules transfectées avec une séquence complète de nétrine 4 mutée. La courbe avec les carrés correspond au milieu conditionné des cellules transfectées avec la séquence SEQ ID NO : 528.

### PARTIE EXPÉRIMENTALE

### Matériels :

Les molécules nétrine 1 (SEQ ID NO : 502), nétrine 4 (SEQ ID NO : 498), nétrine G1 (SEQ ID NO : 506), nétrine 3 (SEQ ID NO : 510) et nétrine 4 mutée (SEQ ID NO : 522) sont des protéines recombinantes. Les molécules nétrine 1, nétrine 4 et nétrine G1 sont disponibles dans le commerce auprès de R&D.

L'isoforme de 165 acides aminés du VEGF est produite par infection de cellules d'insecte SF9 par un baculovirus recombinant contenant l'ADNc correspondant (Plouët et al., 1997).

Des cellules endothéliales artérielles ombilicales humaines (HUAEC) ont été isolées à partir d'artères ombilicales perfusées avec de la collagènase (Sigma) pour digérer la membrane basale. Les cellules HUAEC ont été maintenues dans du milieu EBM (Clonetics) additionné de 15% de sérum de veau foetal (SVF) inactivé par la chaleur, 100 µg/ml de pénicilline et 100 µg/ml de streptomycine à 37°C dans 10% de CO₂. Les cultures souches ont reçu 2 ng/ml de VEGF chaque jour pair.

Des cellules musculaires lisses d'aorte ont été maintenues dans du milieu DMEM additionné de 15% de sérum de veau foetal (SVF) inactivé par la chaleur, 100 µg/ml de pénicilline et 100 µg/ml de streptomycine à 37°C dans 10% de CO₂. Les cultures souches ont reçu 2 ng/ml de FGF chaque jour pair.

### IDENTIFICATION D'UNE NÉTRINE 4 MUTÉE

### Clonage de la nétrine 4 mutée

Les ARN totaux des cellules de l'artère de cordon ombilical humain (HUAEC) ont été extraits à l'aide du TriPure (Roche). Ensuite les ARN ont été transcrits en utilisant le kit pour RT-PCR (AMV) de Roche selon les indications du fabricant.

Les amorces (5')-TT CTA GAC ATG GGG AGC TGC GCG CGG-(3') (sens) et (5')-C ATT AAC GTC GAA CTG ACA GGT ATC-(3') (sens contraire) ont servi à l'amplification de la séquence 1-1039 de la nétrine 4, tandis que les amorces (5')-AG CAC TGT GCC CCG TTA TAC AAT GA-(3') (sens) et (5')-GGG GAT CCA CTT GCA CTC TCT TTT TAA AAT ATC C-(3') (sens contraire) ont été utilisées pour amplifier la séquence 914-1884 de la nétrine 4.

Les conditions adoptées pour l'amplification ont été : 35 cycles avec dénaturation à 94°C pendant 1 minute ; hybridation à 55°C pendant 1 minute ; et extension à 72°C pendant 1 minute.

Les produits obtenus ont été mélangés et utilisés pour effectuer une nouvelle PCR avec les amorces (5')-TT CTA GAC ATG GGG AGC TGC GCG CGG-(3') (sens) et (5')-CGG GAT CCA CTT GCA CTC TCT TTT TAA AAT ATC C-(3') (sens contraire) dans les mêmes conditions décrites précédemment, avec un nombre de cycles de 25 au lieu de 35. Ce produit PCR contenant toute la séquence de la nétrine 4 a été cloné dans le vecteur intermédiaire pCR2.1 (Invitrogen). Après digestion par Xba I et Bam HI, la séquence de la nétrine 4 a été extraite de ce vecteur et insérée dans le vecteur pcDNA3.1 (-)/His myc C digéré par les mêmes enzymes de restriction. Ce dernier vecteur contenant la séquence complète de la nétrine 4 mutée a été utilisé pour transfecter des cellules. Une manipulation identique a conduit à l'obtention d'un vecteur d'expression de la nétrine 4 sauvage en utilisant la séquence de la nétrine 4 sauvage.

La nétrine 4 mutée a été produite par transfection de cellules CHO pgsA 745 par le vecteur contenant la séquence de la nétrine 4 sauvage selon un protocole déjà décrit (Plouët, 1997). La protéine a été purifiée par chromatographie d'affinité pour l'héparine sépharose et éluée par un gradient discontinu de NaCl (0, 3; 1,0 et 2,0 M NaCl. NET 4m est éluée par NaCI 2M, et a un degré de pureté > 90%.

L'activité biologique de la nétrine 4 mutée (NET 4m) a été comparée à celle de la nétrine 4 sauvage (NET 4) selon le test de prolifération des cellules musculaires lisses. La Figure 7A montre que la NET 4 m déclenche une activité mitogénique à une concentration 1000 fois inférieure à celle de NET 4. De même dans un test de migration, NET-4 m est 1000 fois plus active que NET-4 (Voir Figure 7B).

### Construction de mutants de délétion pour la nétrine 4 mutée

Le vecteur pcDNA3.1 (-)/His myc C contenant la séquence complète de la nétrine 4 mutée (628 AA) a été digéré par l'enzyme de restriction BamH1, traité avec le fragment klenow de la polymérase 1, puis digéré avec l'enzyme de restriction PshA1. Le fragment linéarisé correspondant au vecteur pcDNA3.1 (-)/His myc C contenant la séquence nétrine 4 mutée délétée du domaine cter (478-628) est ensuite isolé après migration sur gel d'agarose et purifié sur colonne Qiagen. Après ligation, on obtient donc un vecteur d'expression pcDNA3.1 (-)/His myc C contenant la séquence de la nétrine 4 mutée délétée du domaine cter (1-477). le vecteur pcDNA3.1 (-)/His myc C contenant la séquence complète de la nétrine 4 mutée (628 aa) a été digéré par l'enzyme de restriction Xcml. Cette enzyme qui coupe la séquence interne de la nétrine 4 en deux sites (aa288/aa488) permet de déléter la protéine de son domaine central (domaine V à motifs EGF). Après purification du fragment et ligation, on obtient donc un vecteur d'expression pcDNA3.1 (-)/His myc C contenant la séquence de la nétrine 4 mutée délétée du domaine central (288/488). Cependant, puisque la ligation des deux sites Xcml conduit à l'apparition d'un codon stop (aa 313), ce vecteur code pour une protéine nétrine 4 mutée tronquée de 312 acides aminés comportant la séquence du domaine laminine (1-288) et une séquence protéique de 24 acides aminés.

### Tests de migration des cellules endothéliales vasculaires d'artères ombilicales humaines (HUAEC)

Des cellules endothéliales vasculaires d'artères ombilicales humaines (HUAEC) sont inoculées dans des puits de 4 cm² à haute densité (50 000 cellules/puits). Quand la monocouche est confluente, la prolifération est arrêtée par l'incubation, pendant une nuit, en présence de M199 (Life Sciences) contenant 2% de SVF. Une blessure est alors pratiquée dans la monocouche à l'aide d'un grattoir mousse, permettant de délimiter une surface libre de toute cellule. Les monocouches sont ensuite lavées 3 fois par du M199 pour enlever les cellules non adhérentes. Une photographie est alors prise pour délimiter la surface avant toute migration cellulaire. Les puits sont ensuite incubés en M199 et 2% de SVF seul ou en présence de 50 ng/ml de VEGF en présence de concentrations variables de nétrine 4 (NET-4) ou nétrine 1 (NET-1) ou nétrine G1 (NET-G1). Après 24 heures, les puits sont lavés 3 fois et colorés au May-Crrunwald-Giemsa et photographiés et les cellules comptées dans 8 champs par condition. Les photographies prises avant et après l'expérience sont alors superposées pour permettre le comptage des cellules ayant migré. Les résultats sont exprimés en nombre de cellules par champs.

Les résultats de ces tests pour la nétrine 4 sont indiqués dans la Figure 1.

D'après la Figure 1, on constate que l'addition de NET-4 en l'absence de VEGF n'a pas d'effet sur la migration basale des cellules HUAEC. En revanche, NET-4 inhibe l'activité du VEGF et 50% de l'effet maximal est obtenu avec une concentration de 200 ng/ml de NET-4.

### Tests de migration des cellules musculaires lisses (VSM)

Des cellules musculaires lisses (VSM) sont inoculées dans des puits de 4 cm² à haute densité (50 000 cellules/puits). Quand la monocouche est confluente, la prolifération est arrêtée par l'incubation, pendant une nuit, en présence de M199 (Life Sciences) contenant 2% de SVF. Une blessure est alors pratiquée dans la monocouche à l'aide d'un grattoir mousse, permettant de délimiter une surface libre de toute cellule. Les monocouches sont ensuite lavées 3 fois dans du DMEM pour enlever les cellules non adhérentes. Une photographie est alors prise pour délimiter la surface avant toute migration cellulaire. Les puits sont ensuite incubés en milieu DMEM sans SVF en présence de concentrations variables de nétrine 4 (NET-4) ou nétrine 1 (NET-1). Après 24 h les puits sont lavés 3 fois et colorés au May-Crrunwald-Giemsa et photographiés. Les photographies prises avant et après l'expérience sont alors superposées pour permettre le comptage des cellules ayant migré.

Les résultats de ces tests sont indiqués dans la Figure 2.

D'après la Figure 2, on constate que la nétrine 1 et la nétrine 4 ont un effet positif sur la migration des VSM. En effet, la nétrine 1 et la nétrine 4 stimulent la migration des cellules musculaires lisses et 50% de l'effet maximal est obtenu avec une concentration de 20 ng/ml de NET-4.

### Tests de prolifération des cellules endothéliales vasculaires d'artères ombilicales humaines (HUAEC)

Des plaques de culture à 96 puits ont été ensemencées avec 2 000 cellules HUAEC par puits dans du EBM additionné de 10% de SVF. Les cellules ont été stimulées ou non avec 2 ng/ml de VEGF₁₆₅ et différentes concentrations de nétrine 1 ou nétrine 4. Dans certains puits, 5 mg/ml d'anticorps anti-néogénine ont été ajoutés. Au bout de 5 jours, les puits ont été rincés doucement avec du DMEM et les cellules ont été fixées dans 1% de glutaldéhyde pendant 20 minutes à température ambiante. Les cellules fixées ont été quantifiées par incorporation de cristal violet (Kueng et al., 1989) : les cellules ont été incubées dans 0,1 % de cristal violet (Sigma) pendant 20 minutes à température ambiante, le colorant non incorporé a été éliminé en lavant complètement les puits avec de grandes quantités d'eau et le colorant violet cristallisé incorporé a ensuite été solubilisé par 100 µl de 10% d'acide acétique par puits. Les lectures de densité optique ont été effectuées à 595 nm. Des résultats similaires ont été obtenus dans trois expériences séparées (voir Figure 1). Les valeurs indiquées sont des densités optiques moyennes de 6 puits ± SD.

Les protéines nétrine 1 ou nétrine 4 utilisées seules n'ont pas d'effet significatif sur la prolifération basale (due au sérum seul). En revanche, comme indiqué sur la Figure 3, la protéine nétrine 4 inhibe la prolifération induite par le VEGF sur un mode dépendant de la dose. 50% de l'effet maximal est obtenu avec une concentration de 200 ng/ml. L'addition d'anticorps anti-néogénine inverse complètement l'activité inhibitrice de la prolifération induite par la nétrine 1 ou la nétrine 4.

### Tests de prolifération des cellules musculaires lisses (VSM)

Des plaques de culture à 96 puits ont été ensemencées avec 2 000 cellules VSM par puits dans du milieu DMEM additionné de 10% de SVF. Après 6 h les cellules sont transférées dans du milieu DMEM contenant 2% SVF puis stimulées ou non avec différentes concentrations de nétrine 1, nétrine G1 ou nétrine 4. Au bout de 5 jours, les puits les puits ont été rincés avec du DMEM et les cellules ont été fixées dans 1 % de glutaldéhyde, colorées au cristal violet et solubilisées par de l'acide acétique. Les lectures de densité optique ont été effectuées à 595 nm. Des résultats similaires ont été obtenus dans trois expériences séparées. Les valeurs indiquées sont des densités optiques moyennes de 6 puits ± SD.

La Figure 4 montre que les protéines, nétrine G1 et nétrine 4 utilisées seules ont un effet significatif sur la prolifération sur un mode dépendant de la dose. 50% de l'effet maximal est obtenu avec une concentration de 20 ng/ml. En revanche nétrine 1 exerce un effet non significatif sur la prolifération.

Des plaques de culture à 96 puits ont été ensemencées avec 2 000 cellules VSM par puits dans du milieu DMEM additionné de 10% de SVF. Après 6 h les cellules sont transférées dans du milieu DMEM contenant 2% SVF puis stimulées ou non avec différentes concentrations de nétrine 1, nétrine G1 ou nétrine 4. Dans certains puits 5 µg/ml d'anticorps anti-néogénine ont été ajoutés. Au bout de 5 jours, les puits ont été rincés doucement avec du DMEM et les cellules ont été fixées dans 1 % de glutaldéhyde pendant 20 minutes à température ambiante. Les cellules fixées ont été quantifiées par incorporation de cristal violet (Kueng et al., 1989) : les cellules ont été incubées dans 0,1% de cristal violet (Sigma) pendant 20 minutes à température ambiante, le colorant non incorporé a été éliminé en lavant complètement les puits avec de grandes quantités d'eau et le colorant violet cristallisé incorporé a ensuite été solubilisé par 100 µl de 10% d'acide acétique par puits. Les lectures de densité optique ont été effectuées à 595 nm. Des résultats similaires ont été obtenus dans trois expériences séparées (voir Figure 4). Les valeurs indiquées sont des densités optiques moyennes de 6 puits ± SD.

La Figure 5 montre que l'addition d'anticorps anti-néogénine ne reverse pas l'activité stimulante de la prolifération induite par la nétrine 4.

### Tests d'adhésion cellulaire

Des plaques ELISA à 96 puits (Nunc) ont été recouvertes de protéine VEGF₁₆₅ selon le protocole décrit dans l'article de Hutchings et al. (2003), diluée dans du tampon carbonate 0,05 M à pH 9,6 pendant la nuit à 4°C. Les sites de liaison non spécifiques ont été bloqués pendant 1 heure à 37°C avec 5 mg/ml de BSA (sérum albumine bovine) dans du tampon carbonate et lavés deux fois avec du DMEM avant les expériences. Les cellules ont été trypsinisées, lavées et remises en suspension dans 5 ml de DMEM avec 10% de SVF dans un tube de plastique non traité et incubées pendant 1 heure à 37°C avec 10% de CO₂. Les cellules ont ensuite été concentrées par centrifugation et remises en suspension dans un mélange DMEM + 0,2% BSA sans sérum et la suspension cellulaire a été traitée pendant 20 minutes (37°C, 10% de CO₂) avec la protéine nétrine 4 utilisée pour moduler l'adhésion. 40 000 cellules par puits ont été distribuées dans les puits dans un volume de 100 µl de DMEM + 0,2% de BSA. Les cellules ont été laissées adhérer à 37°C sous 10% de CO₂ pendant le temps voulu. Les puits ont été lavés doucement trois fois avec du DMEM pour retirer les cellules non adhérentes et les cellules adhérentes ont été fixées avec 1 % de glutaraldéhyde pendant 20 minutes à température ambiante. Les cellules fixées ont été quantifiées par incorporation de cristal violet (Kueng et al., 1989) : les cellules ont été incubées avec 0,1% de violet cristallisé (Sigma) dilué dans 0,2 M de tampon borate à pH 9,5 pendant 20 minutes à température ambiante, le colorant non incorporé a été éliminé en lavant complètement les puits avec de grandes quantités d'eau et le colorant cristal violet incorporé a ensuite été solubilisé par 100 µl de 10% d'acide acétique par puits.

### Test d'apoptose

50 000 cellules HUAEC sont déposées sur des puits de culture préalablement gélatinisées dans du milieu EBM contenant 10% de SVF et 2 ng/ml de VEGF. 2 jours après les cellules sont incubées une nuit dans du milieu EBM contenant 2% de SVF et 200 ng/ml de nétrine 4 en présence ou absence FGF2 ou VEGF. Les cellules sont rincées 24 h après et incubées avec du iodure de propidium, fixées et examinées au microscope à fluorescence. Les cellules présentant des noyaux fragmentés sont comptées en fluorescence et les cellules totales sont comptées sous éclairage direct. L'opération est effectué dans au moins 8 champs représentant un minimum de 300 cellules. Le pourcentage de cellules présentant un noyau fragmenté est alors calculé.

La nétrine 4 utilisée seule n'a pas d'effet significatif sur l'apoptose. En revanche, la protéine nétrine 4 inhibe totalement l'effet anti-apoptotique exercé par le VEGF ou le FGF-2 (voir Figure 6).

### Production d'anticorps anti-idiotypiques

Dans un premier temps on prépare un anticorps neutralisant de nétrine 1, 3, G1 ou 4 ou de l'un de ses fragments susmentionnés (SEQ ID NO : 2 à 352) en injectant à un animal, notamment une souris, ladite nétrine ou ledit fragment mélangée avec de l'adjuvant complet de Freund (1 volume par volume de nétrine ou de fragment de nétrine). On choisira une quantité de nétrine ou de fragment de nétrine comprise entre 10 et 500 µg/kg de poids corporel pour immuniser l'animal. La même opération est effectuée à 15 et 30 jours d'intervalle, excepté que l'adjuvant complet est remplacé par de l'adjuvant incomplet. Au jour 40 une saignée est pratiquée, le sérum est séparé et les immunoglobulines sont purifiées par toute méthode de fractionnement habituelle, notamment précipitation au sulfate d'ammonium, chromatographie d'affinité pour la protéine A ou G. On mesure l'activité neutralisante des immunoglobulines par n'importe quel test décrit (par exemple, dans le cas de la nétrine 4 ou de l'un de ses fragments, liaison de la nétrine 4 marquée au domaine extracellulaire de l'un quelconque de ses récepteurs, prolifération, migration, adhésion cellulaire). Ainsi, un lot d'immunoglobulines sera dit neutralisant quand il aura la capacité d'inhiber l'interaction de la nétrine 1, 3, 4 ou G1 avec soit le domaine extracellulaire de dcc, de néogénine, de UNC5H1, de UNC5H2, de UNC5H3 ou de UNCSH4.

Dans un deuxième temps, on prépare des anticorps anti-idiotypiques de la nétrine 1, 3, G1 ou 4, ou de l'un de leurs fragments en injectant à des souris par voie sous-cutanée 1-100 µg de la préparation des immunoglobulines neutralisant l'activité de ladite nétrine ou dudit fragment précédemment décrites en association avec 100 µl d'adjuvant, notamment de l'adjuvant complet de Freund (Sigma). L'injection est répétée 15, 30 et 45 jours après. Cinquante-cinq jours après la première injection, on injecte à des souris 10 µg du même anticorps par voie intrapéritonéale. Cinquante-huit jours après la première injection, les souris sont sacrifiées et leurs rates sont prélevées et dilacérées dans du milieu ISCOVE pour libérer les splénocytes. Les splénocytes sont fusionnés avec des cellules de myélome de souris, notamment des cellules AG8X 63 (Kearney et al., 1979), et incubés à raison de 100 000 cellules/puits. La fusion s'effectue par ajout de 20 fois 50 µl de polyéthylène glycol (PEG) à 30 secondes d'intervalle. Quatre ml de milieu ISCOVE préchauffé à 37°C sont alors ajoutés goutte à goutte sur la suspension cellulaire, puis après une période d'incubation de 4 minutes à 37°C, 4 ml sont ajoutés. La suspension est centrifugée puis le culot cellulaire est alors repris dans 100 ml de milieu ISCOVE complémenté avec 20% de sérum de veau foetal et du HAT IX (50X : Hypoxanthine 5 mM, Aminoptérine 20 µM et Thymidine 0,8 mM) et distribuées à raison de 100 µl par puits sur les macrophages. Après 5 jours, 100 µl de milieu HAT sont ajoutés, et entre 8 et 14 jours le milieu conditionné de chaque hybridome est prélevé pour mesurer par ELISA les anticorps dirigés contre les anticorps ayant servi d'agent immunogène, c'est à dire les anticorps anti-nétrine 1, 3, G1 ou 4. On mesure alors l'activité des anticorps anti-idiotypiques par un test ELISA :
Les fragments Fab des immunoglobulines anti-nétrine 1, 3, G1 ou 4, préparés par toute technique conventionnelle, notamment une digestion à la papaïne, sont immobilisés sur des plaques de microtitration (0,1-20 µg/ml dans du tampon carbonate 50 mM pH 9,6). Après saturation des sites non spécifiques par une solution de sérum albumine diluée à 5 mg/ml dans le même tampon, les surnageants de cultures d'hybridomes sont ajoutés dilués pour moitié dans du tampon PBS (solution tampon phosphate) contenant 0,05% de Tween 20. Après rinçages, les anticorps anti-idiotypiques sont révélés par adjonction d'une concentration appropriée d'anticorps anti-Fc de souris couplé à la peroxydase. La quantité d'anticorps anti-idiotypique fixé est alors mesurée par révélation de la peroxydase et est proportionnelle à l'intensité de la réaction colorimétrique.
Les hybridomes sélectionnés par leur capacité à sécréter des anticorps dirigés contre des anticorps anti-nétrine 1, 3, G1 ou 4 sont alors clonés, c'est-à-dire que les cellules sont ensemencées en condition de dilution limite (5 cellules/ml) sous un volume de 0,1 ml par puits. Le milieu est changé après 10 jours. Après 15 jours, certains puits contiennent des foyers de cellules qui se sont multipliées à partir de la cellule ensemencée au départ, donc toutes ces cellules sont identiques et sont issues du même clone. Quand la surface occupée par les cellules représente au moins la moitié de la surface totale du puits, le milieu est prélevé et analysé.

On met en oeuvre alors un second ELISA : des immunoglobulines de chèvre dirigées contre les domaines Fc des IgG humaines sont incubées sur des plaques de microtitration (0,1-20 µg/ml dans du tampon carbonate 50 mM pH 9,6). Après saturation des sites non spécifiques par une solution de sérum albumine diluée à 5 mg/ml dans le même tampon, les protéines contenant les domaines extracellulaires des récepteurs des nétrines fusionnés à une séquence Fc d'IgG humaine sont immobilisés sur les plaques de microtitration (incubation à une concentration comprise entre 1 et 100 µg/ml). Les surnageants de cultures d'hybridomes sont ajoutés dilués pour moitié dans du tampon PBS contenant 0,05% de Tween 20. Après rinçages, les anticorps anti-idiotypiques sont révélés par adjonction d'une concentration appropriée d'anticorps anti-Fc de souris couplé à la peroxydase. La quantité d'anticorps anti-idiotypique fixé est alors mesurée par révélation de la peroxydase et est proportionnelle à l'intensité de la réaction colorimétrique.

Une fois les clones identifiés, leur nature monoclonale est affirmée par l'opération classique consistant à ensemencer une plaque de 96 puits avec des cellules issues du même clone diluées en conditions limites comme précédemment. Les clones sécréteurs doivent donc tous sécréter un anticorps de même spécificité pour que l'on déclare cet anticorps monoclonal. Un troisième clonage est alors effectué exactement dans les mêmes conditions pour s'assurer que les clones sont bien monoclonaux.

Les anticorps anti-idiotypiques monoclonaux sont criblés par une batterie de tests, notamment par un test ELISA sur domaines extracellulaires des récepteurs connus des nétrines (dcc, néogénine, UNC5-A, UNC5-B, UNC5-C, UNC5-D), d'inhibition de la prolifération ou de la migration des cellules HUAEC ou des tests de mesure *in vivo* d'une activité anti-angiogénique.

Les anticorps anti-idiotypiques sont donc des mimes de domaines de nétrines. Le but est de fabriquer une "image interne" d'un domaine de nétrine et donc de pouvoir obtenir un anticorps liant 1 récepteur de nétrine sans lier tous les récepteurs. Une fois la spécificité attestée, on détermine la fonction agoniste de cet anticorps donné en mesurant son activité sur des cellules c'est à dire que l'on inhibe les fonctions des HUAEC sans inhiber ou stimuler les fonctions des nétrines sur les VSM et/ou on stimule les VSM sans affecter les HUAEC.

L'intérêt général de ces anticorps est de pouvoir mimer une fonction des nétrines sur une cible cellulaire sans affecter d'autres cibles. Ainsi, il serait intéressant de stimuler les fonctions des péricytes sans induire l'apoptose des cellules endothéliales ou inhiber leur migration, leur prolifération, leur différenciation dans certaines pathologies :
- la dégénérescence maculaire liée à l'âge,
- les rétinopathies diabétiques, à un stade précoce où la raréfaction des péricytes précède la néovascularisation,
- le glaucome néovasculaire (de la cornée, de la rétine...),
- la polyarthrite rhumatoïde,
- le psoriasis, en particulier la polyarthrite psoriasique,
- les angiomes,
- l'athérosclérose,
- l'obésité,
- les cancers.

Inversement, il serait intéressant d'inhiber la prolifération des cellules endothéliales sans affecter les péricytes pour les pathologies ci-dessus.

### Angiogenèse in vitro

Quatre queues de rat ont été dépiautées et disséquées pour récupérer les faisceaux blancs qui sont constitués en majeure partie de collagène de type I. Le collagène est extrait de ces fibres dans 50 ml d'acide acétique 0,5 M froid et agités sur une nuit. Le liquide est ensuite centrifugé à 5000g pendant 40 minutes et le surnageant est récupéré. L'extraction est refaite une fois avec 20 ml d'acide acétique, les surnageants sont mélangés et puis dialysés contre 1 1 d'acide acétique 0,2 M. La concentration en collagène est ajustée à 3 mg/ml par pesée. La préparation des gels pour l'angiogenèse *in vitro* s'effectue sur de la glace pour conserver la solution de collagène sous forme liquide. Un ml de collagène (5 mg/ml) est mélangé à 0,5 ml de DMEM 10X (contenant une concentration 10X en antibiotiques et en glutamine), 0,9 ml de H₂O stérile et 0,1 ml de bicarbonate de sodium 1M. Une fois le pH ajusté à 7,4, il est ajouté un volume égal de matrigel (Becton Dickinson). Le gel est coulé dans des puits de culture (2 mm d'épaisseur) et incubé à 37°C pour se solidifier. Les cellules sont rajoutées après 15 minutes (100 000 cellules/cm²) sur la surface du gel. Après 2 heures, les différents facteurs solubles sont ajoutés et les cellules sont observées et photographiées après 24 heures.

### Comparaison de l'activité de NET-4 et NET-4m sur la prolifération (Figure 7 A) et la migration (Figure 7 B) des VSM

Des plaques de culture à 96 puits ont été ensemencées avec 2 000 cellules VSM par puits dans du milieu DMEM additionné de 10% de SVF. Après 6 h les cellules sont transférées dans du milieu DMEM contenant 2% SVF puis stimulées ou non avec différentes concentrations de nétrine 4 (NET-4) ou nétrine 4 mutée (NET-4m). Au bout de 5 jours, les puits ont été rincés avec du DMEM et les cellules ont été fixées dans 1% de glutaldéhyde, colorées au cristal violet et solubilisées par de l'acide acétique. Les lectures de densité optique ont été effectuées à 595 nm. Des résultats similaires ont été obtenus dans trois expériences séparées. Les valeurs indiquées sont des densités optiques moyennes de 6 puits ± SD.

### Test de prolifération des VSM (figure 7 A)

La nétrine 4 mutée a été produite par transfection de cellules CHO pgsA 745 par le vecteur contenant la séquence de la nétrine 4 sauvage selon un protocole déjà décrit (Plouët, 1997). La protéine a été purifiée par chromatographie d'affinité pour l'héparine sépharose et éluée par un gradient discontinu de NaCl (0, 3; 1,0 et 2,0 M NaCl. NET 4M est éluée par NaCl 2M, et a un degré de pureté > 90%.

L'activité biologique de NET 4 m a été comparée à celle de la NET 4 sauvage selon le test de prolifération des cellules musculaires lisses. La Figure 7A montre que la moitié de la stimulation maximale est obtenue avec une concentration de 120 ng/ml de nétrine 4 non mutée (NET-4) et 0.1 ng/ml de nétrine 4 mutée (NET-4m). Ceci signifie que l'activité mitogénique de la nétrine 4 mutée est 1000 fois plus active que la nétrine 4 non mutée.

### Test de migration des VSM (figure 7 B)

La monocouche confluente de VSM est incubée, pendant une nuit, en présence de DMEM. Une blessure est alors pratiquée dans la monocouche à l'aide d'un grattoir mousse, puis les puits sont lavés 3 fois par du DMEM puis incubés en présence de concentrations variables de nétrine 4 (NET-4) ou nétrine 4 mutée (NET-4m). Après 24 heures, les puits sont lavés 3 fois et colorés au May-Grunwald-Giemsa et photographiés et les cellules comptées dans 8 champs par condition. Les résultats sont exprimés en nombre de cellules par champs.

Il apparaît que la moitié de la stimulation maximale est obtenue avec une concentration de 12 ng/ml de nétrine 4 non mutée (NET-4) et 0,004 ng/ml de nétrine 4 mutée (NET-4m). Ceci signifie que l'activité chimiotactique de la nétrine 4 mutée est 3000 fois plus active que la nétrine 4 non mutée.

### Transfection de cellules cancéreuses PC3

Les cellules de cancer de la prostate sont cultivées en milieu DMEM additionnée d'antibiotiques et de 10% de sérum de veau foetal. Le protocole de transfection a été établi comme suit :
- J1 : inoculation de cellules à basse densité (10000 cellules/cm²) dans une boîte de 10 cm de diamètre
- J2 : transfection par pcDNA3-NET4 ou pcDNA-3-NET-4m
   5 µg de plasmide sont mélangés avec 5 µl de lipofectine et 100 µl de DMEM (sans antibiotiques) pendant une demi-heure à température ambiante et agités doucement. Le mélange est ensuite dilué à 5 ml dans du DMEM et déposé goutte à goutte dans la boite contenant les cellules PC3. Après 6 heures d'incubation dans une étuve à 37°C, le milieu est aspiré et remplacé par 10 ml de milieu frais.
- J3 : rinçage de la boite et incubation pendant 24 heures avec du milieu DMEM contenant 10% de sérum de veau foetal et les antibiotiques
- J4 : trypsinisation des cellules, incubation dans 4 boîtes de 10 cm de diamètre dans du milieu DMEM complet additionné de 500 µg/ml de généticine (Sigma)
- J17 : prélèvement de clones de cellules (100-400 cellules/clone) à l'aide d'une micropipette et transfert dans des puits de 2 cm²
- J24 : trypsinisation et incubation des clones cellulaires dans des boîtes de 12 puits (120000 cellules/puits)
- J27 : rinçage des puits et inoculation dans du milieu DMEM sans sérum
- J30 : collecte des milieux conditionnés et analyse de la quantification de nétrine 4 dans chaque milieu

Après avoir vérifié que les clones transfectés par la nétrine 4 (NET-4) ou la nétrine 4 mutée (NET-4m) ont un temps de doublement équivalent (26-30 heures), le contenu de nétrine 4 de chaque milieu a été mesuré comme décrit précédemment dans le paragraphe concernant le test de prolifération. 4 µl de milieu conditionné ont été ajoutés à 100 µl de milieu de culture. Les résultats sont exprimés en pourcentage de prolifération par rapport au contrôle (puits contenant 4 µl de milieu DMEM).

Il apparaît sur la Figure 8 que le milieu de cellules non transfectées ainsi que les clones 10 et 15 de NET 4 induisent une prolifération équivalente des cellules HUAEC, de l'ordre de 300% par rapport au contrôle.

En revanche le milieu conditionné des clones 1, 5 de NET-4 m ainsi que le clone 8 de NET-4 stimulent la prolifération des cellules HUAEC de seulement 200%, ce qui correspond à environ 50% de la prolifération induite par le milieu conditionné des cellules PC3.

Donc la nétrine 4 (NET-4) ou la nétrine 4 mutée (NET-4 m) inhibent la prolifération des HUAEC sans modifier la prolifération des cellules cancéreuses PC3.

### Analyse de la tumorigénicité des clones de PC3 transfectées

Les cellules PC3 non transfectées et les cellules PC3 transfectées par NET-4 m (clones 1 et 5) ont été injectées à des souris nude dans le flanc (1 million de cellules par injection).

La longueur (L) et la largeur (1) de chaque tumeur a été mesurée au pied à coulisse et le volume exprimé par la formule 0,52x Lx 1²

Il apparaît sur la Figure 9 que les clones 1 et 5 donnent naissance à des tumeurs beaucoup plus petites que les tumeurs obtenues avec des cellules PC3. La réduction est supérieure à 80 %.

Donc la nétrine 4 mutée (NET-4 m) exerce une activité antitumorale par son activité anti-angiogénique.

### Effet synergique de NET 4 sur l'inhibition du VEGF.

Il est actuellement bien admis que le VEGF est un acteur majeur de l'angiogenèse pathologique et que son inhibition est une voie thérapeutique majeure. Un anticorps anti-VEGF est commercialisé sous le nom d'AVASTIN. Sachant que la nétrine 4 (NET-4) agit par un mécanisme d'action différent du VEGF, nous avons mesuré l'effet synergique de la nétrine 4 (NET 4) avec un anticorps anti-VEGF commercialisé sous le nom d'AVASTIN.

Des souris ont reçu une greffe de PC3 non transfectées ou de PC3 transfectées par NET-4 clone 8 présentant une réduction marginale du volume tumoral ou clone 10 ou 15 qui donnent naissance à des tumeurs de volume équivalent à celui des cellules PC3 non transfectées (non montré). Une fois que les tumeurs ont présenté un volume supérieur à 600 mm3, les souris ont reçu une injection péritonéale d'AVASTIN (50 µg tous les 3 jours), dose correspondant aux recommandations thérapeutiques en pathologie humaine (5mg/Kg/semaine) et le volume tumoral a été mesuré comme décrit précédemment.

Il apparaît sur la Figure 10 que l'AVASTIN n'a aucun effet sur les tumeurs PC3 non transfectées ou PC3 clone 10 ou 15 qui ne sécrètent pas d'activité NET-4 suffisante pour inhiber la progression tumorale. Après 4 jours l'accroissement du volume tumoral par rapport au volume au début du traitement est de 200, 150 et 200 % respectivement. En revanche le volume des tumeurs clone 8, qui sécrète une activité NET-4 importante, reste stable : donc la nétrine 4 permet de restaurer une sensibilité aux traitements anti-VEGF dans des tumeurs de taille importante

### Angiogenèse choroïdienne chez la souris

12 Souris C57BL/6J âgées de 9 semaines ont reçu une photocoagulation laser de la rétine selon le protocole suivant.

Les souris sont anesthésiées grâce à une injection intrapéritonéale de pentobarbital sodium (Centravet) (200µl par souris d'une solution diluée à 0,6 mg/100µl). Après endormissement, les souris subissent une dilatation pupillaire au tropicamide 1 %. Une lame de verre ronde de 12mm de diamètre fait office de lentille de contact pour magnifier le fond d'oeil. Puis un impact de laser (50µm, 400 mW, 0,05s) est réalisé sur chaque rétine à environ 2 diamètres papillaires du nerf optique en nasal.

Après 7 jours, les souris subissent une anesthésie locale par instillation oculaire de tétracaïne et une antisepsie par instillation de bétadine 5%. A l'aide du microinjecteur-microcapillaire des injections intravitréenne de 1µl de solution de PBS ou de nétrine 4 à différentes concentrations sont réalisées. 3 souris sont injectées avec du PBS, 3 souris sont injectées avec de la nétrine 4 (0,1 mg/ml), 2 souris sont injectées avec de la nétrine 4 (0,3 mg/ml), 2 souris sont injectées avec de la nétrine 4 (1 mg/ml), 2 souris contrôles ne subissent aucune injection.

Après 10 jours, les souris sont sacrifiées, énuclées par montage des deux yeux en OCT puis les rétines sont coupées au microtome et révélées par les anticorps anti-CD3 marquant les cellules endothéliales qui apparaissent en blanc.

Sur les Figures 11A et 11B, il apparaît que l'injection de nétrine 4 à 0,1 et 0,3 µg n'a pas d'effet. En revanche à la dose de 1µg/oeil il y a une nette réduction de la vascularisation choroïdienne et de la réaction inflammatoire gliale.

En particulier sur la Figure 11A, il apparaît à l'intérieur de la rétine une augmentation ponctiforme de la vascularisation et une efflorescence de toute la vascularisation choroïdienne en blanc.

Sur la Figure 11B on remarque une diminution de la néovascularisation rétinienne mais surtout une réduction de l'épaisseur de la zone néovascularisée et du nombre de vaisseaux.

### Angiogenèse choroïdienne chez le rat

Les rats sont anesthésiés grâce à une injection intrapéritonéale de pentobarbital sodium (Centravet) (1 ml par rat d'une solution diluée à 0,6 mg/100µl). Après endormissement, les rats subissent une dilatation pupillaire au tropicamide 1%. Une lame de verre ronde de 12mm de diamètre fait office de lentille de contact pour magnifier le fond d'oeil. Puis trois impacts de laser (50µm, 400 mW, 0,05s) sont réalisés sur chaque rétine à environ 2 diamètres papillaires du nerf optique en nasal.

Après 7 et 10 jours les rats ont reçu (4 par groupe, soit 6-8 yeux analysés par condition, donc 12-18 impacts par condition) une injection sous rétinienne (sous un volume de 5 µl) contenant :
- PBS
- NET-4 (2µg)
- NET-1 (2 µg)
- NET-4 m et purifiée (100 pg)

Après 14 jours les rats ont été anesthésiés, perfusés par une solution de dextran fluorescent (en blanc) qui permet de visualiser la perfusion des vaisseaux. Les rétines ont ensuite été montées "à plat et photographiés.

Dans les yeux traités par le véhicule seul on voit une zone centrale marquée en gris au niveau de l'impact correspondant à une prolifération endothéliale et une couronne de vaisseaux marqués en blanc. Ces vaisseaux étant visualisés par le dextran fluorescent, on peut affirmer qu'ils sont fonctionnels. Il apparaît que les injections de NET-4 et de NET-1 ont un effet marginal à 2 µg/ rat. En revanche, l'injection de 100 pg de NET-4 m inhibe presque totalement la perfusion et donc la néovascularisation choroïdienne.

La quantification a été effectuée en mesurant les pixels par µ² et réduit la perfusion de 50%.

Les résultats sur les Figures 12A à 12D, montrent que la nétrine 1 et la nétrine 4 exercent une réduction marginale de la néovascularisation par rapport au contrôle alors qu'une dose 2000 fois plus faible de nétrine 4 mutée exerce une réduction significativement importante : p inférieur à 0,02.

### Effet de NET-4 m (MC4) et du fragment de séquence SEO ID NO : 528 sur la migration des VSM (MC5).

Des cellules pgsA-745 CHO ont été transfectées avec les vecteurs d'expression PCDNA-3 contenant la séquence complète de nétrine 4 mutée (MC4) ou la séquence SEQ ID NO : 528 (MC5). Après 16 h les cellules sont incubées avec du milieu DMEM et les milieux conditionnés collectés après 48 h. L'activité de migration sur les cellules VSM est mesurée comme décrit précédemment (voir Figure 13).

Sur la Figure 13, il apparaît que MC4 et MC5 ont la même activité spécifique (équivalent à µg/ml de nétrine 4 de référence). Donc la délétion de 340 acides aminés située en position C terminale n'affecte pas la fonction de la nétrine 4 mutée.

### RÉFÉRENCES

- Celerier J, Cruz A, Lamande N, Gasc JM, Corvol P (2002) Angiotensinogen and its cleaved derivatives inhibit angiogenesis. Hypertension. 39(2):224-8,
- Cooper HM, Gad JM, Keeling SL (1999) The Deleted in Colorectal Cancer netrin guidance system: a molecular strategy for neuronal navigation. Clin Exp Pharmacol Physiol. 26(9):749-51 ;
- Corset V, Nguyen-Ba-Charvet KT, Forcet C, Moyse E, Chedotal A, Mehlen P (2000) Netrin-1-mediated axon outgrowth and cAMP production requires interaction with adenosine A2b receptor. Nature. 407: 747-50 ;
- Hutchings H, Ortéga N, Plouët J (2003) Extracellular matrix bound vascular endothelial growth factor promotes endothelial cell adhesion, migration and survival through integrin ligation. FASEB J. 17: 1520-1522 ;
- Inoki I, Shiomi T, Hashimoto G, Enomoto H, Nakamura H, Makino K, Ikeda E, Takata S, Kobayashi K, Okada Y (2002) Connective tissue growth factor binds vascular endothelial growth factor (VEGF) and inhibits VEGF-induced angiogenesis. FASEB J. 16(2):219-21,
- Jain RK, Schlenger K, Hockel M, Yuan F (1997) Quantitative angiogenesis assays: progress and problems. Nat Med. 3(11):1203-8,
- Kearney JF, Radbruch A, Liesegang B, Rajewsky K (1979) A new mouse myeloma cell line that has lost immunoglobulin expression but permits the construction of antibody-secreting hybrid cell lines. J. Immunol. 123, 1548-50 ;
- Kueng W, Silber E, Eppenberger U (1989) Quantification of cell cultures on 96-well plates. Anal. Biochem, 182(1): 16-9 ;
- Livesey FJ (1999) Netrins and netrin receptors. Cell Mol Life Sci, 56(1-2): 62-8 ;
- Lu X, le Noble F, Yuan L, Jiang Q, de Lafarge B, Sugiyama D, Breant C, Claes F, De Smet F, Thomas JL, Autiero M, Carmeliet P, Tessier-Lavigne M, Eichmann A (2004) The netrin receptor UNC5B mediates guidance events controlling morphogenesis ofthe vascular system. Nature. 432: 179-186 ;
- Matsunaga E, Tauszig-Delamasure S, Monnier PP, Mueller BK, Strittmatter SM, Mehlen P, Chedotal A (2004) RGM and its receptor neogenin regulate neuronal survival. Nat Cell Biol. 8, 749-755 ;
- Mehlen P, Mazelin L (2003) The dependence receptors DCC and UNC5H as a link between neuronal guidance and survival. Biol Cell. 95(7): 425-36 ;
- Nakashiba T, Ikeda T, Nishimura S, Tashiro K, Honjo T, Culotti JG, Itohara S (2000) Netrin-G1: a novel glycosyl phosphatidylinositol-linked mammalian netrin that is functionally divergent from classical netrins. J Neurosci. 20(17): 6540-50 ;
- Papanikolaou, G, Ludwig, EH, MacDonald, ML, Franchini, PL, Dube, M-P, Andres, L, MacFarlane, J, Sakellaropoulos, N, Politou, M, Nemeth, E, Thompson, J, Risler, J, Zaborowska, C, Babakaiff, R, Radomski, CC, Christakis, JL, Brissot, P, Lockitch, G, Ganz, T, Hayden, MR, Samuels, ME et Goldberg, P (2004) Mutations in HFE2 cause iron overload in chromosome 1q linked juvenile hemochromatosis. Nat. Genet. 77-82 36, 77-82 ;
- Plouët J, Moro F, Coldeboeuf N, Bertagnolli S, Clamens S, Bayard F (1997) Extracellular cleavage of the vascular endothelial growth factor 189 aa form by urokinae is required for its mitogenic activity. J. Biol. Chem., 272, 13390-13396 ;
- Yurchenco PD, Wadsworth WG (2004) Assembly and tissue functions of early embryonic laminins and netrins. Curr Opin Cell Biol. 16(5):572-9.
- Arnal JF, Gourdy P, Garmy-Susini B, Delmas E, Bayard F (2003) Usefulness of experimental models to understand the vascular effects of estrogens. Med Sci (Paris). 19(12): 1226-32 ;
- Brooks PC, Clark RA, Cheresh DA (1994) Requirement of vascular integrin alpha v beta 3 for angiogenesis. Science, 264, 569-71 ;
- O'Reilly et al. (1997) Cell 88:277-285 ;
- Ortéga N, Hutchings H, Plouët J (1999) Signal relays in the VEGF system. Front. Biosc., 4, D141-D152 ;

## Revendications

1. Utilisation :
- d'un fragment de la nétrine 1 représentée par la séquence SEQ ID NO : 502 ou SEQ ID NO : 504, ledit fragment étant constitué par l'une des séquences, SEQ ID NO : 144 ou SEQ ID NO : 2i, i variant de 64 à 71 et de 73 à 131, ou
- d'une séquence nucléotidique étant l'une des séquences SEQ ID NO : 143 ou SEQ ID NO : 2i-1, i variant de 64 à 71 et de 73 à 131,
pour la préparation d'un médicament destiné au traitement des pathologies nécessitant l'inhibition de la prolifération et/ou de la migration endothéliale.

2. Utilisation selon la revendication 1, où lesdites pathologies nécessitant l'inhibition de la prolifération endothéliale sont choisies parmi les pathologies suivantes : les cancers et les leucémies, la dégénérescence maculaire liée à l'âge, les rétinopathies diabétiques, la polyarthrite rhumatoïde, la polyarthrite psoriasique, les angiomes, les angiosarcomes, la maladie de Castelman et le sarcome de Kaposi, ou dans le cadre du traitement de l'obésité ou de la néovascularisation rétinienne.

3. Utilisation selon la revendication 1 ou 2, pour la préparation d'un médicament susceptible d'être administré à raison d'environ 0,1 à environ 20 mg/kg, notamment par voie intraveineuse, par voie sous-cutanée, par voie systémique, par injection intra-vitréenne, par voie locale au moyen d'infiltrations ou par l'intermédiaire d'un collyre, éventuellement associé à une électroperméation.

4. Fragment de la protéine nétrine 1 représenté par la séquence SEQ ID NO SEQ ID NO : 502 ou SEQ ID NO : 504, ledit fragment étant **caractérisé en ce qu'**il est constitué par l'une des séquences suivantes :
* la séquence SEQ ID NO : 144 ou SEQ ID NO : 2i, i variant de 64 à 71 et de 73 à 131, ou
* toute séquence dérivée de l'une des séquences susmentionnées, notamment par substitution, suppression ou addition d'un ou plusieurs acides aminés, sous réserve que cette séquence dérivée présente une activité anti-angiogénique, ou
* ou toute séquence nucléotidique homologue de l'une des séquences nucléotidiques susmentionnées, ayant de préférence une identité d'au moins environ 50%, avec l'une des séquences SEQ ID NO : 2i-1 codant pour une protéine homologue de SEQ ID NO : 2i, i variant de 64 à 131, sous réserve que cette séquence homologue présente une activité anti-angiogénique.

5. Fragment de la protéine nétrine 1 selon la revendication 4, ledit fragment étant **caractérisé en ce qu'**il est constitué par l'une des séquences SEQ ID NO : 2i, i variant de 64 à 131, notamment la séquence SEQ ID NO : 144.

6. Séquence nucléotidique codant pour un fragment tel que défini dans l'une quelconque des revendications 4 à 5.

7. Séquence nucléotidique selon la revendication 6, **caractérisée en ce qu'**elle comprend ou est constituée par :
- l'une des séquences nucléotidiques SEQ ID NO : 2i-1 codant pour SEQ ID NO : 2i, i variant de 64 à 131,
- ou toute séquence nucléotidique dérivée, par dégénérescence du code génétique, de l'une des séquences nucléotidiques susmentionnées, et codant pour une protéine représentée par SEQ ID NO : 2i, i variant de 64 à 131,
- ou toute séquence nucléotidique dérivée, notamment par substitution, suppression ou addition d'un ou plusieurs nucléotides, de l'une des séquences nucléotidiques susmentionnées codant pour une protéine dérivée de l'une des séquences SEQ ID NO : 2i, i variant de 64 à 131, telle que définie ci-dessus,
- ou toute séquence nucléotidique homologue de l'une des séquences nucléotidiques susmentionnées, ayant de préférence une identité d'au moins environ 50%, avec l'une des séquences SEQ ID NO :2i-1 codant pour une protéine homologue de SEQ ID NO : 2i, i variant de 64 à 131,
- ou toute séquence nucléotidique complémentaire des séquences susmentionnées,
- ou toute séquence nucléotidique susceptible d'hybrider dans des conditions stringentes avec la séquence complémentaire d'une des séquences susmentionnées.

8. Vecteur recombinant, notamment plasmide, cosmide, phage ou ADN de virus, contenant une séquence nucléotidique telle que définie dans l'une quelconque des revendications 6 ou 7, notamment SEQ ID NO : 2i-1, i variant de 64 à 131, notamment la séquence SEQ ID NO : 143.

9. Vecteur recombinant selon la revendication 8, contenant les éléments nécessaires à l'expression dans une cellule hôte des polypeptides codés par lesdits acides nucléiques, notamment SEQ ID NO : 2i-1, i variant de 64 à 131, insérés dans ledit vecteur.

10. Cellule hôte, choisie notamment parmi les bactéries, les virus, les levures, les champignons, les plantes ou les cellules de mammifères, ladite cellule hôte étant transformée, notamment à l'aide d'un vecteur recombinant telle que défini dans la revendication 8 ou 9.

11. Composition pharmaceutique, comprenant à titre de substance active,
- un fragment de la nétrine 1 tel que défini dans l'une quelconque des revendications 4 à 5, de préférence l'une des séquences SEQ ID NO : 144 ou SEQ ID NO : 2i, i variant de 64 à 71 et de 73 à 131, ou
- une séquence nucléotidique telle que définie dans la revendication 6 ou 7, notamment l'une des séquences SEQ ID NO : 143 ou SEQ ID NO : 2i-l, i variant de 64 à 71 et de 73 à 131.

12. Produit de combinaison comprenant:
- un fragment de la nétrine 1 tel que défini dans l'une quelconque des revendications 4 à 5, de préférence l'une des séquences SEQ ID NO : 144 ou SEQ ID NO : 2i, i variant de 64 à 71 et de 73 à 131,
en association avec un agent anti-angiogénique AVASTIN (bevacizumab), MACUGEN (pegaptanib) et LUCENTIS (ranibizumab), ou tout autre agent anti-VEGF pour une utilisation simultanée, séparée ou étalée dans le temps destiné au traitement ou à la prévention des traitement des pathologies tumorales ou non tumorales telles que définies dans les revendications 1 à 2.

13. Utilisation :
- d'un fragment de la nétrine 1, étant l'une des séquences SEQ ID NO : 144 ou SEQ ID NO : 2i, i variant de 64 à 71 et 73 à 131, ou
- d'une séquence nucléotidique étant l'une des séquences SEQ ID NO : 143 ou SEQ ID NO : 2i-1, i variant de 64 à 71 et 73 à 131,
en association avec un agent anti-angiogénique notamment choisi parmi *: AVASTIN* (bevacizumab), MACUGEN (pegaptanib) et LUCENTIS (ranibizumab), ou tout autre agent anti-VEGF pour la préparation d'un médicament destiné à la prévention ou au traitement des pathologies tumorales ou non tumorales telles que définies dans les revendications 1 à 2.
